# EUROPEAN PATENT APPLICATION

(11) **EP 4 468 082 A1**
(43) Date of publication of application: **27.11.2024**
(21) Application number: 24174275.8
(22) Date of filing: 06.05.2024
(51) Int. Cl.: G03F 7/09

(54) **MATERIAL FOR FORMING ORGANIC FILM, PATTERNING PROCESS, AND ORGANIC FILM COMPOUND**

(30) Priority: 10.05.2023 JP 2023077725
(71) Applicant: SHIN-ETSU CHEMICAL CO., LTD., Tokyo 1000005 (JP)
(72) Inventor: KORI, Daisuke, Niigata (JP); YAMAMOTO, Yasuyuki, Niigata (JP)
(74) Representative: Sonnenhauser, Thomas Martin

(57) **Abstract**

The present invention is a material for forming an organic film, containing: an organic film compound represented by the following general formula (1); and an organic solvent. This provides: an organic film compound which allows the formation of an organic film having not only excellent heat resistance and properties of filling and planarizing a pattern formed on a substrate, but also favorable film-formability and adhesiveness to a substrate; a material for forming an organic film containing the compound; and a patterning process using the material.

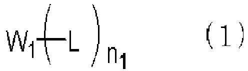

## Description

### TECHNICAL FIELD

The present invention relates to: a material for forming an organic film, a patterning process, and an organic film compound.

### BACKGROUND ART

As LSI advances toward high integration and high processing speed, miniaturization of pattern size is progressing rapidly. Along with the miniaturization, lithography technology has achieved a fine patterning by shortening the wavelength of a light source and selecting an appropriate resist composition accordingly. The composition mainly used is a positive photoresist composition for monolayer. The monolayer positive photoresist composition not only allows a resist resin to have a skeleton having etching resistance against dry etching with chlorine- or fluorine-based gas plasma, but also provides a switching mechanism that makes an exposed part soluble, thereby dissolving the exposed part to form a pattern and processing a substrate to be processed by dry etching while using the remaining resist pattern as an etching mask.

However, there arises a problem that when the pattern becomes finer, that is, the pattern width is reduced without changing the thickness of the photoresist film to be used, resolution performance of the photoresist film is lowered. In addition, pattern development of the photoresist film with a developer excessively increases a so-called aspect ratio of the pattern, resulting in pattern collapse. Therefore, the photoresist film has been thinned along with the miniaturization of the pattern.

On the other hand, a substrate to be processed has been generally processed by dry etching while using a pattern-formed photoresist film as an etching mask. In practice, however, there is no dry etching method capable of providing an absolute etching selectivity between the photoresist film and the substrate to be processed. There has been a problem that the resist film is thus damaged and collapses during processing of the substrate, and the resist pattern cannot be precisely transferred to the substrate to be processed. Accordingly, higher dry etching resistance has been required in a resist composition along with the miniaturization of the pattern. However, on the other hand, resins used for photoresist compositions have been required to have low absorbance at the wavelength to be used for the exposure in order to achieve a higher resolution. Accordingly, as the exposure light shifts from i-beam to KrF and to ArF to have a shorter wavelength, the resin also shifts to novolak resins, polyhydroxystyrene, and resins having an aliphatic polycyclic skeleton. This shift actually accelerates an etching rate under the dry etching conditions during substrate processing, and recent photoresist compositions having high resolution tend to have low etching resistance.

As a result, a substrate to be processed has to be dry-etched with a thinner photoresist film having lower etching resistance. It is important to provide a material for this process and the process itself.

A multilayer resist method is one solution for these problems. This method is as follows: a resist underlayer film having a different etching selectivity from a photoresist film (i.e., a resist upper layer film) is placed between the resist upper layer film and a substrate to be processed; a pattern is formed in the resist upper layer film; then, the pattern is transferred to the resist underlayer film by dry etching while using the resist upper layer film pattern as a dry etching mask; and the pattern is further transferred to the substrate to be processed by dry etching while using the resist underlayer film as a dry etching mask.

One of the multilayer resist methods is a 3-layer resist method, which can be performed with a typical resist composition used in the monolayer resist method. For example, this 3-layer resist method includes the following steps: an organic film containing a novolak resin or the like is formed as a resist underlayer film on a substrate to be processed; a silicon-containing film is formed thereon as a resist middle layer film; and a usual organic photoresist film is formed thereon as a resist upper layer film. Since the organic resist upper layer film exhibits a favorable etching selectivity ratio relative to the silicon-containing resist middle layer film when dry etching is performed with fluorine-based gas plasma, the resist upper layer film pattern can be transferred to the silicon-containing resist middle layer film by dry etching with fluorine-based gas plasma. According to this process, even when using a resist composition which is difficult to form a pattern in so that the pattern has a sufficient film thickness for directly processing the substrate to be processed or a resist composition which does not have sufficient dry etching resistance for processing the substrate, a pattern can be transferred to a silicon-containing resist middle layer film. In addition, by subsequently transferring the pattern by dry etching using an oxygen- or hydrogen-based gas plasma, it is possible to obtain a pattern of an organic film (resist underlayer film) containing a novolak resin or the like having a sufficient dry etching resistance for processing the substrate. As such a resist underlayer film, many are already known, such as those disclosed in Patent Document 1, for example.

Meanwhile, in recent years, consideration of the production of semiconductor devices having a new structure such as a multigate structure has become active. In response, demands are rising for a resist underlayer film having better planarizing property and filling property than before. For example, when an underlying substrate to be processed has a fine pattern structure such as a hole, a trench, or a fin, it is necessary to have filling (gap-filling) property for filling in the pattern with a film without any voids by using a resist underlayer film. In addition, when the underlying substrate to be processed has a step(s), or when a pattern-dense region and a pattern-free region exist on the same wafer, it is necessary to planarize the film surface with the resist underlayer film. By planarizing the surface of the resist underlayer film, fluctuation in the film thickness of a resist middle layer film or a resist upper layer film formed thereon is controlled, whereby a focus margin in lithography or a margin in the subsequent processing step of the substrate to be processed can be suppressed from decreasing.

Moreover, the organic film material excellent in filling and planarizing properties is not limited to the underlayer film for a multilayer resist, and is also widely usable as a planarizing material for manufacturing a semiconductor device, e.g., for planarizing a substrate prior to patterning by nanoimprinting. Furthermore, for global planarizing in the semiconductor device manufacturing process, a CMP process is now generally used. However, the CMP process is costly, so that this material is also expected to be used for the global planarizing method, instead of CMP.

For forming a flat film for planarizing an uneven semiconductor substrate, a resist underlayer film material containing a polymer obtained by a reaction between an aromatic compound and a compound having a carbon-oxygen double bond such as a carbonyl group is proposed (Patent Document 2). However, planarizing performance etc. of the material in wide trench portions in the substrate are insufficient to meet the demands in cutting-edge devices, and a resist underlayer film material excellent in flatness on a wider range of substrate structures has come to be required.

In addition, the structures of substrates to be processed are becoming complex as described above. Furthermore, for the surface of a substrate to be processed, the following materials are also being examined: novel materials having high electron mobility using strained silicon, gallium arsenic, and so forth; ultrathin polysilicon controlled in units of angstrom; and the like. Thus, films are expected to be formed on surfaces of substrates to be processed having various shapes and qualities. Therefore, in order to ensure a process margin, not only excellent filling and planarizing properties, but also being able to form a film without dependence on the quality or shape of the substrate to be processed is an important feature.

### CITATION LIST

### PATENT LITERATURE

Patent Document 1: JP 2004-205685 A
Patent Document 2: WO 2019/225615 A1

### SUMMARY OF INVENTION

### TECHNICAL PROBLEM

The present invention has been made in view of the above circumstances. An object of the present invention is to provide: an organic film compound which allows the formation of an organic film having not only excellent heat resistance and properties of filling and planarizing a pattern formed on a substrate, but also favorable film-formability and adhesiveness to a substrate; a material for forming an organic film containing the compound; and a patterning process using the material.

### SOLUTION TO PROBLEM

To achieve the object, the present invention provides a material for forming an organic film, comprising: an organic film compound represented by the following general formula (1); and an organic solvent, 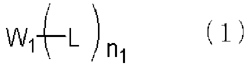 wherein W₁ represents an organic group having a valency of "n₁" and containing an aromatic ring, each L represents a substituent on the aromatic ring and is L₁, L₂, or both, represented by the following general formulae (2), and when a total number of the substituents L on the aromatic ring is "n₁", a number of L₁ is "x", and a number of L₂ is "y", "n₁", "x", and "y" satisfy relationships x + y = n₁, 2 ≤ n₁ ≤ 8, 0 ≤ y ≤ 8, and 0 ≤ x ≤ 8, 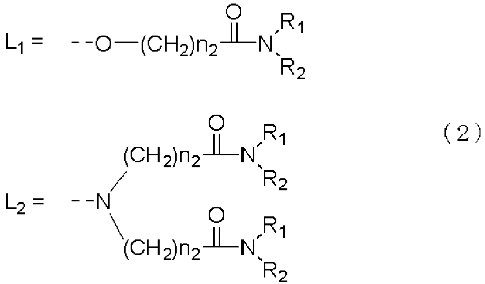 wherein "n₂" represents an integer of 1 to 3, R₁ represents one of the following formulae (3), and R₂ represents one of the following general formulae (4), 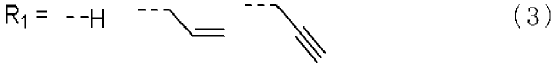 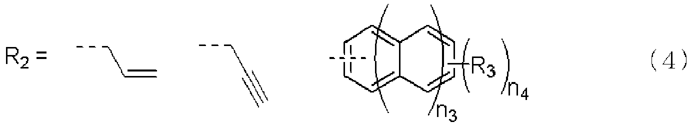 wherein R₃ represents one of the following formulae (5), "n₃" represents 0 or 1, and "n₄" represents 1 or 2.

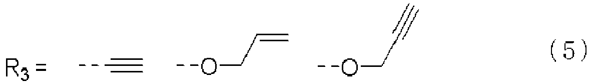

Such a material for forming an organic film is capable of forming an organic film that is not only excellent in heat resistance and filling and planarizing properties of a pattern formed in a substrate, but also has favorable film-formability and adhesiveness to a substrate.

Furthermore, in the present invention, the L₁ and L₂ constituting the substituents L on the aromatic ring of the organic film compound represented by the general formula (1) are preferably represented by the following general formulae (6), 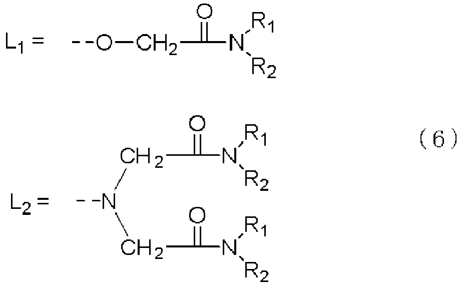 wherein R₁ and R₂ are as defined above.

By introducing such structures, it is possible to achieve both of the conflicting properties, thermal flowability and heat resistance.

Furthermore, in the present invention, the R₁ and R₂ in the L₁ and L₂ constituting the substituents L on the aromatic ring of the organic film compound represented by the general formula (1) are preferably any of combinations shown by the following general formulae (7), 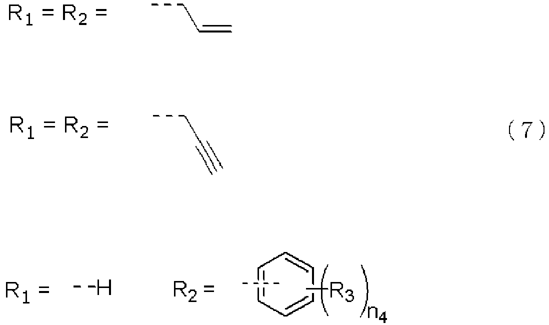 wherein R₃ and "n₄" are as defined above.

When the R₁ and R₂ are constituted by such combinations, it is possible to enhance thermosetting property and form an organic film having good heat resistance.

The total number "n₁" of the substituents L on the aromatic ring of the organic film compound represented by the general formula (1) preferably satisfies 3 ≤ n₁ ≤ 6.

When the number of thermosetting groups, which are substituents, is adjusted within such a range, the thermosetting groups are not excessive, and therefore, rapid thermosetting does not occur and thermal flowability does not become degraded, and therefore, planarizing property does not become degraded; and there is no risk of insufficient thermosetting groups causing insufficient solvent resistance, heat resistance, etc.

Furthermore, in the present invention, the organic film compound preferably satisfies 1.00 ≤ Mw/Mn ≤ 1.15, where Mw is a weight-average molecular weight and Mn is a number-average molecular weight measured by gel permeation chromatography in terms of polystyrene.

When the Mw/Mn is as described, it is possible to form an organic film excellent in filling property and flatness.

Furthermore, in the present invention, the organic solvent is preferably a mixture of one or more kinds of organic solvent having a boiling point of lower than 180°C and one or more kinds of organic solvent having a boiling point of 180°C or higher.

When the organic solvent is the above mixture, the organic film is provided with thermal flowability by adding the high-boiling-point solvent to the organic film compound, so that the material for forming an organic film is provided with both high filling and planarizing properties.

Furthermore, in the present invention, the material for forming an organic film preferably further comprises at least one of a surfactant and a plasticizer.

The material for forming an organic film containing such components has better coating property and filling and planarizing properties.

In addition, the present invention provides a patterning process for forming a pattern in a body to be processed, comprising:
forming an organic film by using the above-described material for forming an organic film on a body to be processed;
forming a silicon-containing resist middle layer film by using a silicon-containing resist middle layer film material on the organic film;
forming a resist upper layer film by using a photoresist composition on the silicon-containing resist middle layer film;
forming a circuit pattern in the resist upper layer film;
transferring the pattern to the silicon-containing resist middle layer film by etching while using the resist upper layer film having the formed pattern as a mask;
transferring the pattern to the organic film by etching while using the silicon-containing resist middle layer film having the transferred pattern as a mask; and
further forming the pattern in the body to be processed by etching the body to be processed while using the organic film having the transferred pattern as a mask.

A fine pattern can be formed in the body to be processed with high precision by the patterning process according to the 3-layer resist process.

In addition, the present invention provides a patterning process for forming a pattern in a body to be processed, comprising:
forming an organic film by using the above-described material for forming an organic film on a body to be processed;
forming a silicon-containing resist middle layer film by using a silicon-containing resist middle layer film material on the organic film;
forming an organic antireflective coating on the silicon-containing resist middle layer film;
forming a resist upper layer film by using a photoresist composition on the organic antireflective coating, so that a 4-layered film structure is constructed;
forming a circuit pattern in the resist upper layer film;
transferring the pattern to the organic antireflective coating and the silicon-containing resist middle layer film by etching while using the resist upper layer film having the formed pattern as a mask;
transferring the pattern to the organic film by etching while using the silicon-containing resist middle layer film having the transferred pattern as a mask; and
further forming the pattern in the body to be processed by etching the body to be processed while using the organic film having the transferred pattern as a mask.

A fine pattern can be formed in the body to be processed with even higher precision by the patterning process according to the 4-layer resist process.

In addition, the present invention provides a patterning process for forming a pattern in a body to be processed, comprising:
forming an organic film by using the above-described material for forming an organic film on a body to be processed;
forming an inorganic hard mask middle layer film selected from a silicon oxide film, a silicon nitride film, and a silicon oxynitride film on the organic film;
forming a resist upper layer film by using a photoresist composition on the inorganic hard mask middle layer film;
forming a circuit pattern in the resist upper layer film;
transferring the pattern to the inorganic hard mask middle layer film by etching while using the resist upper layer film having the formed pattern as a mask;
transferring the pattern to the organic film by etching while using the inorganic hard mask middle layer film having the transferred pattern as a mask; and
further forming the pattern in the body to be processed by etching the body to be processed while using the organic film having the transferred pattern as a mask.

A fine pattern can be formed on the body to be processed with high precision by the patterning process according to the 3-layer resist process.

In addition, the present invention provides a patterning process for forming a pattern in a body to be processed, comprising:
forming an organic film by using the above-described material for forming an organic film on a body to be processed;
forming an inorganic hard mask middle layer film selected from a silicon oxide film, a silicon nitride film, and a silicon oxynitride film on the organic film;
forming an organic antireflective coating on the inorganic hard mask middle layer film;
forming a resist upper layer film by using a photoresist composition on the organic antireflective coating, so that a 4-layered film structure is constructed;
forming a circuit pattern in the resist upper layer film;
transferring the pattern to the organic antireflective coating and the inorganic hard mask middle layer film by etching while using the resist upper layer film having the formed pattern as a mask;
transferring the pattern to the organic film by etching while using the inorganic hard mask middle layer film having the transferred pattern as a mask; and
further forming the pattern in the body to be processed by etching the body to be processed while using the organic film having the transferred pattern as a mask.

A fine pattern can be formed on the body to be processed with higher precision by the patterning process according to this 4-layer resist process.

In this case, the inorganic hard mask middle layer film is preferably formed by a CVD method or an ALD method.

When the inorganic hard mask middle layer film is formed by a CVD method or an ALD method, a fine pattern can be formed in the body to be processed with higher precision.

In this event, the pattern in the resist upper layer film is preferably formed by a lithography using light with a wavelength of 10 nm or more to 300 nm or less, a direct drawing with electron beam, nanoimprinting, or a combination thereof.

When the circuit pattern is formed in the resist upper layer film by such methods, a fine pattern can be formed in the body to be processed with higher precision.

In this event, alkali development or development with an organic solvent is preferably performed as a development method in the patterning process.

When alkali development or development with an organic solvent is employed as the development method, a fine pattern can be formed in the body to be processed with higher precision.

In this event, the body to be processed is preferably a semiconductor device substrate or the semiconductor device substrate coated with any of a metal film, a metal carbide film, a metal oxide film, a metal nitride film, a metal oxycarbide film, and a metal oxynitride film.

In the present invention, those given above can be used, for example, as the body to be processed.

In this event, the metal is preferably silicon, titanium, tungsten, hafnium, zirconium, chromium, germanium, cobalt, copper, silver, gold, aluminum, indium, gallium, arsenic, palladium, iron, tantalum, iridium, manganese, molybdenum, ruthenium, or an alloy thereof.

These metals can be used as the metal.

The present invention also provides an organic film compound represented by the following general formula (1), 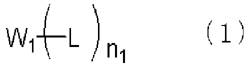 wherein W₁ represents an organic group having a valency of "n₁" and containing an aromatic ring, each L represents a substituent on the aromatic ring and is L₁, L₂, or both, represented by the following general formulae (2), and when a total number of the substituents L on the aromatic ring is "n₁", a number of L₁ is "x", and a number of L₂ is "y", "n₁", "x", and "y" satisfy relationships x + y = n₁, 2 ≤ n₁ ≤ 8, 0 ≤ y ≤ 8, and 0 ≤ x ≤ 8, 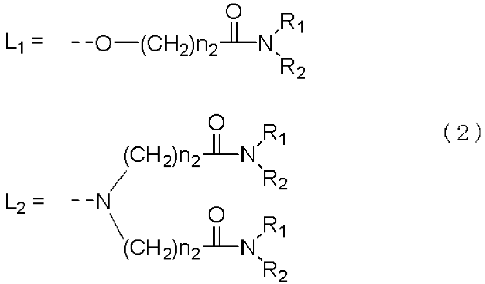 wherein "n₂" represents an integer of 1 to 3, R₁ represents one of the following formulae (3), and R₂ represents one of the following general formulae (4), 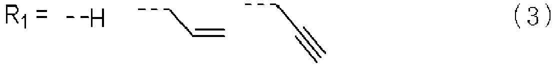 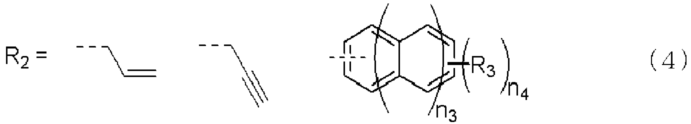 wherein R₃ represents one of the following formulae (5), "n₃" represents 0 or 1, and "n₄" represents 1 or 2.

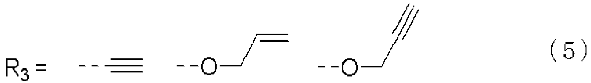

Such an organic film compound can be contained in a material for forming an organic film capable of forming an organic film that is not only excellent in heat resistance and filling and planarizing properties of a pattern formed in a substrate, but also has favorable film-formability and adhesiveness to a substrate.

### ADVANTAGEOUS EFFECTS OF INVENTION

As described above, the inventive material for forming an organic film has various properties, such as heat resistance and filling and planarizing properties, and is a material useful for forming an organic film that can be formed with no dependence on the substrate to be processed. Therefore, the material is extremely useful as a material for forming an organic film in a multilayer resist process, such as a 2-layer resist process, a 3-layer resist process using a silicon-containing resist underlayer film, or a 4-layer resist process using a silicon-containing resist middle layer film and an organic antireflective coating, or as a planarizing material for manufacturing a semiconductor device, for example. Furthermore, according to the inventive patterning process, a fine pattern can be formed in a substrate to be processed with high precision in a multilayer resist process.

The inventive organic film compound, represented by the general formula (1), has excellent heat resistance and thermosetting property, and therefore, the compound can be a material for an organic film on its own. In addition, since the compound has excellent thermal flowability, the organic film has excellent properties of filling and planarizing a patterned substrate when the organic film is used as a resist underlayer film.

Furthermore, since the compound has an amide structure in the molecule, it is possible to improve not only heat resistance but also adhesiveness to and film-formability on a substrate and so forth. In addition, by introducing an alkylene group between the amide structure and an aromatic ring, crystallinity is reduced, and not only thermal flowability but also film-formability is improved. Moreover, by appropriately selecting the main skeleton represented by W₁, it is also possible to adjust, in accordance with required performance, various physical properties of the organic film when used as a resist underlayer film, for example, optical characteristics and etching resistance.

### BRIEF DESCRIPTION OF DRAWINGS

FIG. 1 is an explanatory diagram of an example of an inventive patterning process according to a 3-layer resist process.
FIG. 2 is an explanatory diagram of a method for evaluating the filling property in Examples.
FIG. 3 is an explanatory diagram of a method for evaluating the planarizing property in Examples.
FIG. 4 is an explanatory diagram of a method for measuring the adhesiveness in Examples.

### DESCRIPTION OF EMBODIMENTS

As described above, the following have been desired: a material for forming an organic film in a fine patterning process by a multilayer resist method in a semiconductor device manufacturing process, where the material makes it possible to form an organic film excellent in film-formability and flatness even on a substrate to be processed having portions that are particularly difficult to planarize such as a wide trench structure; a patterning process in which the material is used; and an organic film compound.

The present inventors have found out that the inventive material for forming an organic film, containing a compound having a particular substituent on an aromatic ring, is useful for forming an organic film excellent in filling and planarizing properties, and completed the present invention.

That is, the present invention is a material for forming an organic film, comprising: an organic film compound represented by the following general formula (1); and an organic solvent, 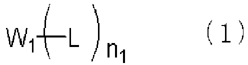 wherein W₁ represents an organic group having a valency of "n₁" and containing an aromatic ring, each L represents a substituent on the aromatic ring and is L₁, L₂, or both, represented by the following general formulae (2), and when a total number of the substituents L on the aromatic ring is "n₁", a number of L₁ is "x", and a number of L₂ is "y", "n₁", "x", and "y" satisfy relationships x + y = n₁, 2 ≤ n₁ ≤ 8, 0 ≤ y ≤ 8, and 0 ≤ x ≤ 8, 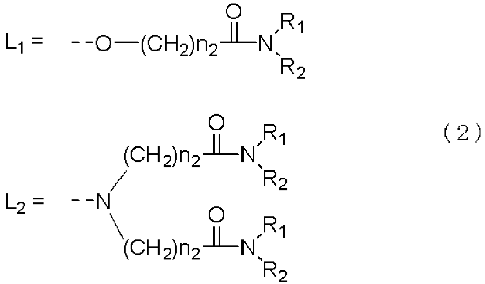 wherein "n₂" represents an integer of 1 to 3, R₁ represents one of the following formulae (3), and R₂ represents one of the following general formulae (4), 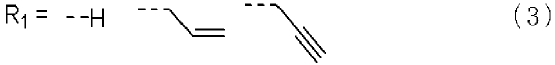 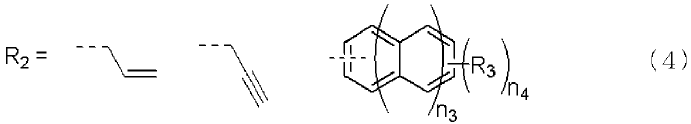 wherein R₃ represents one of the following formulae (5), "n₃" represents 0 or 1, and "n₄" represents 1 or 2.

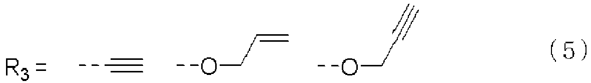

Hereinafter, the present invention will be described in detail, but the present invention is not limited thereto.

### <Material for Forming Organic Film>

The inventive material for forming an organic film contains: an organic film compound represented by the following general formula (1); and an organic solvent. In the following, the components contained in the inventive material for forming an organic film will be described in detail.

### <Organic Film Compound>

The inventive organic film compound is represented by the following general formula (1). 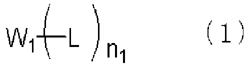 In the formula, W₁ represents an organic group having a valency of "n₁" and containing an aromatic ring, each L represents a substituent on the aromatic ring and is L₁, L₂, or both, represented by the following general formulae (2), and when a total number of the substituents L on the aromatic ring is "n₁", a number of L₁ is "x", and a number of L₂ is "y", "n₁", "x", and "y" satisfy relationships x + y = n₁, 2 ≤ n₁ ≤ 8, 0 ≤ y ≤ 8, and 0 ≤ x ≤ 8. 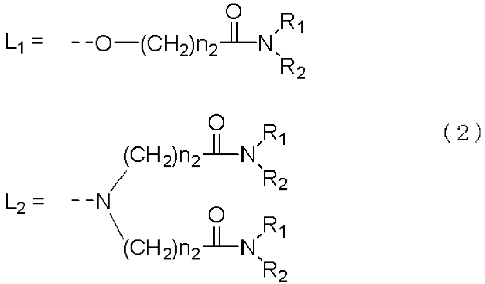 In the formulae, "n₂" represents an integer of 1 to 3, R₁ represents one of the following formulae (3), and R₂ represents one of the following general formulae (4) . 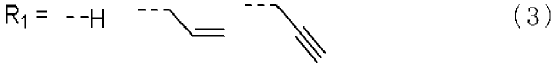 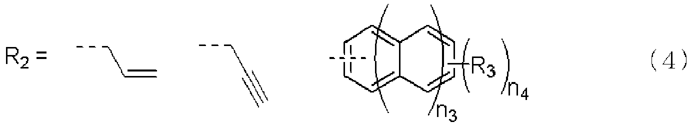 In the formula, R₃ represents one of the following formulae (5), "n₃" represents 0 or 1, and "n₄" represents 1 or 2.

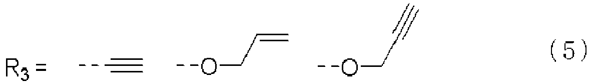

The W₁ in the general formula (1) represents an organic group having a valency of "n₁" and containing an aromatic ring, and specific examples include the structures shown below and so forth. These aromatic rings may have substituents other than L thereon, and examples include alkyl groups having 1 to 10 carbon atoms, alkynyl groups and alkenyl groups having 3 to 10 carbon atoms, aryl groups having 6 to 10 carbon atoms, a nitro group, halogen groups, a nitrile group, a sulfonyl group, alkoxycarbonyl groups having 2 to 10 carbon atoms, alkanoyloxy groups having 2 to 10 carbon atoms, etc.

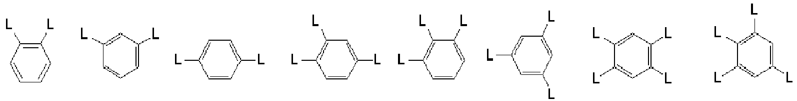

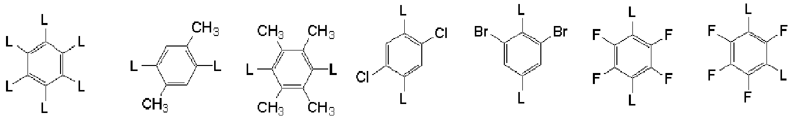

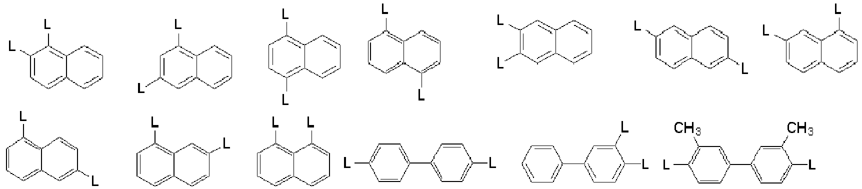

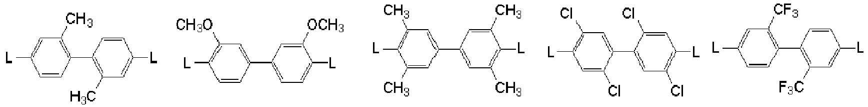

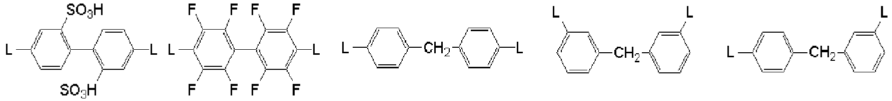

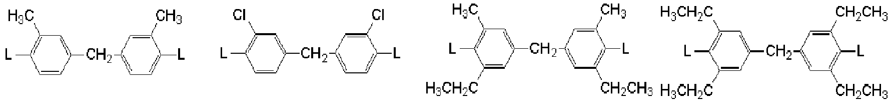

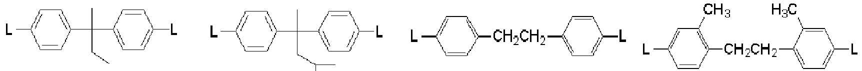

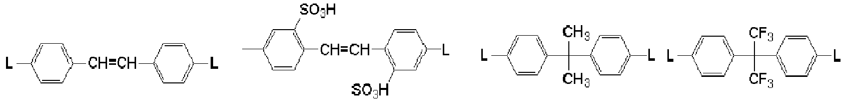

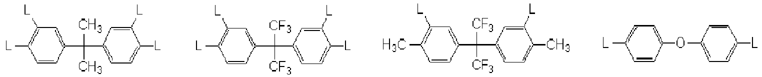

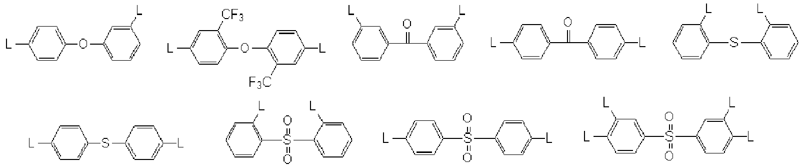

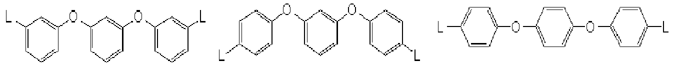

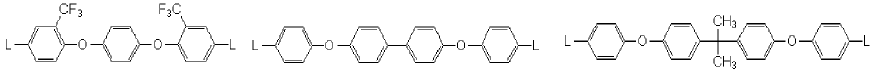

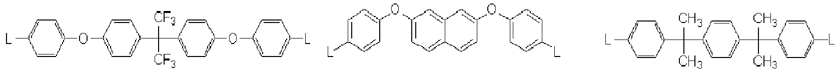

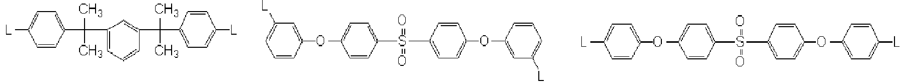

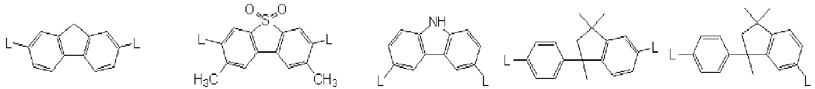

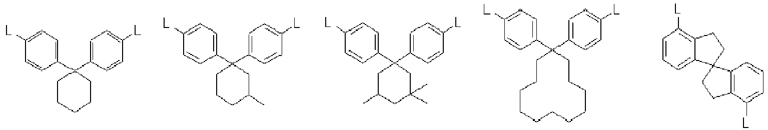

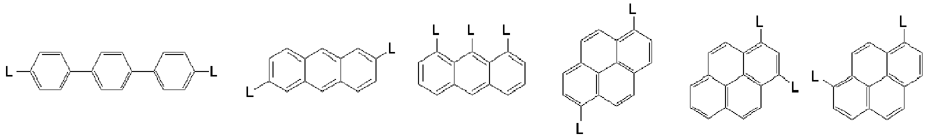

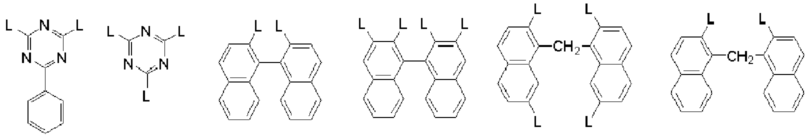

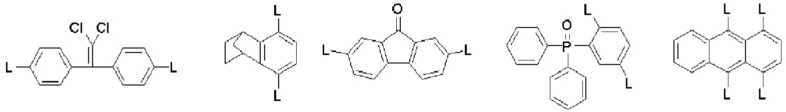

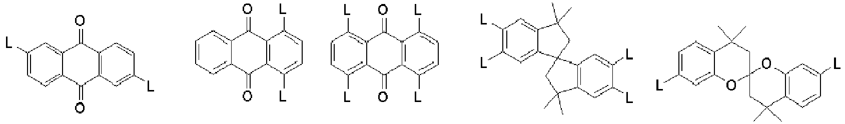

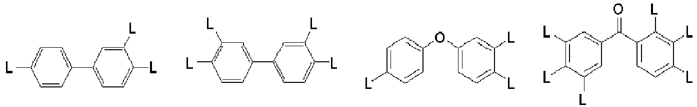

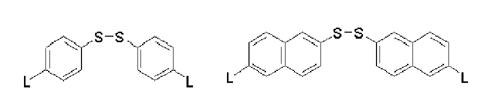

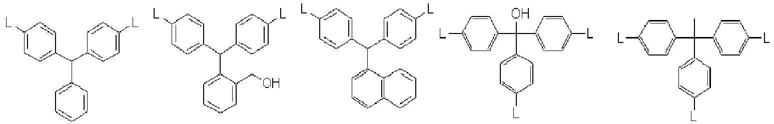

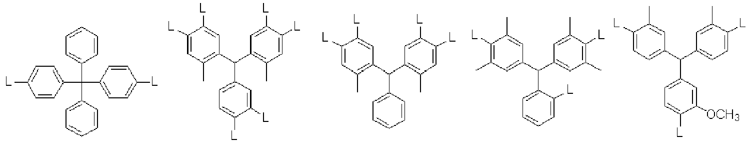

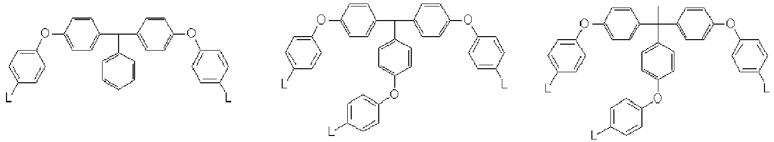

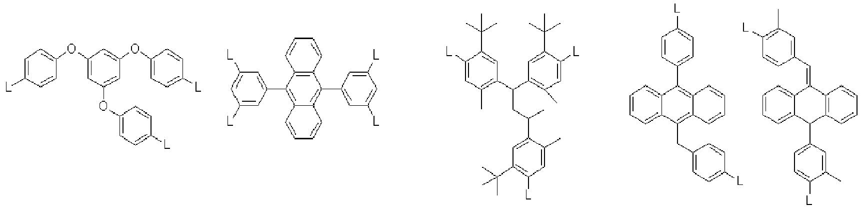

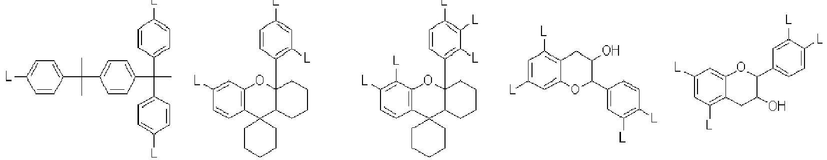

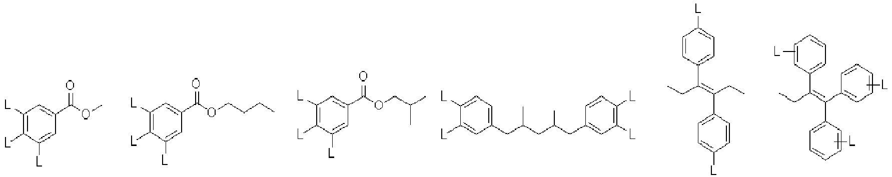

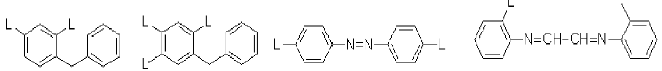

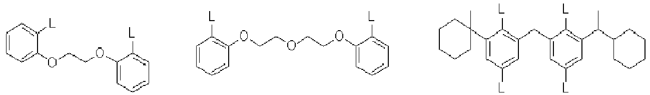

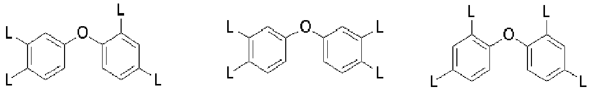

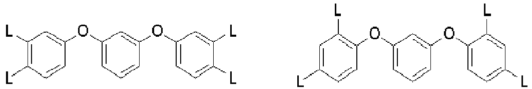

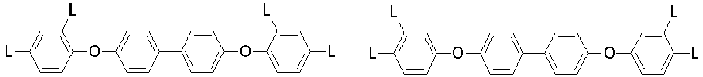

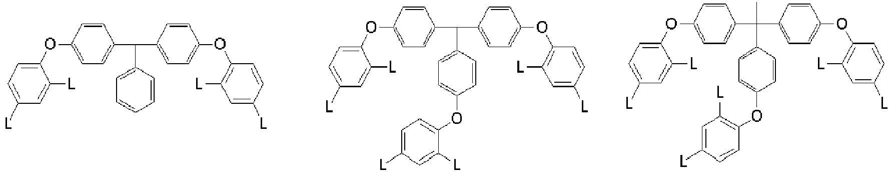

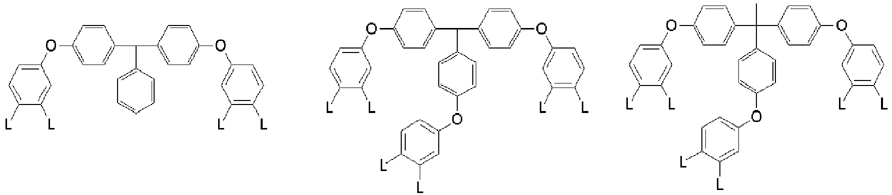

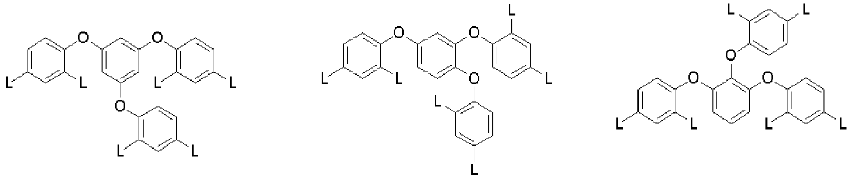

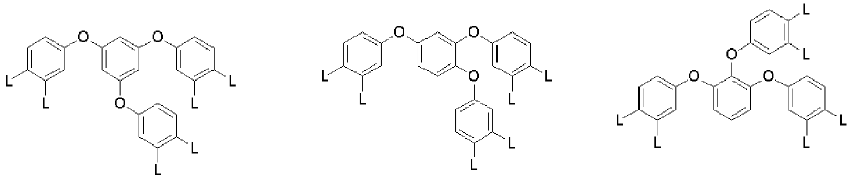

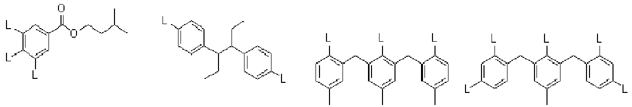

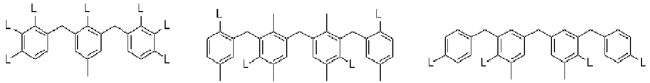

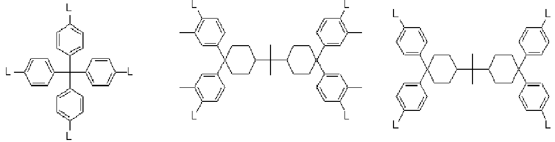

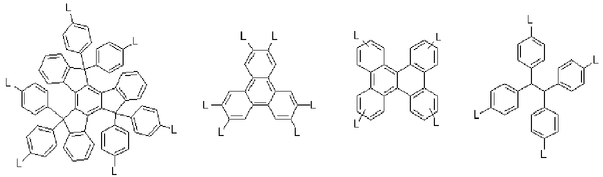

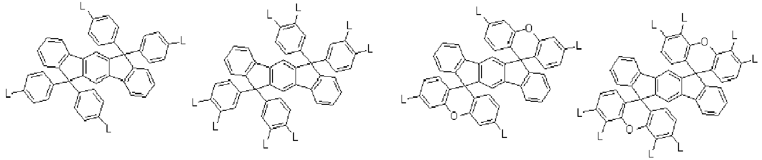

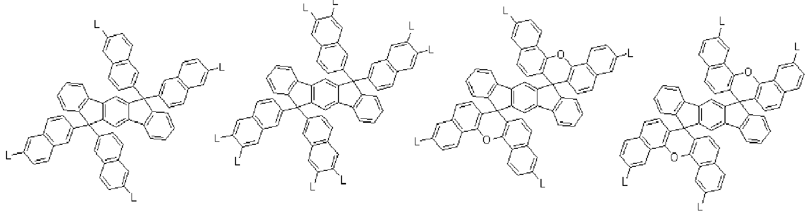

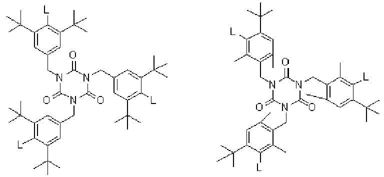

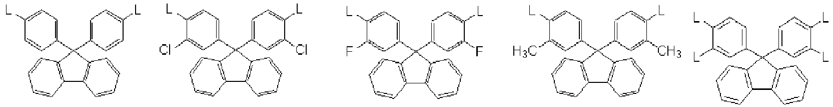

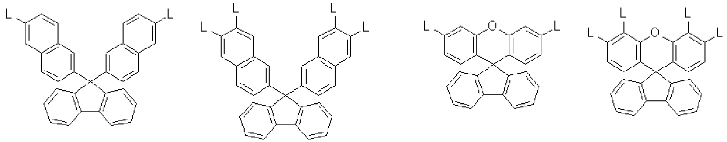

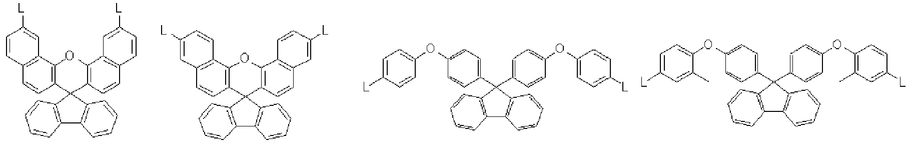

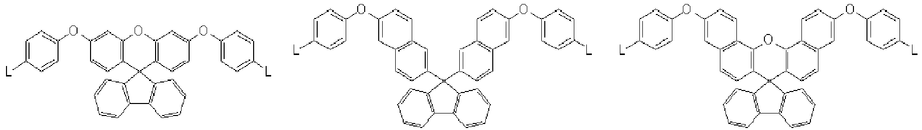

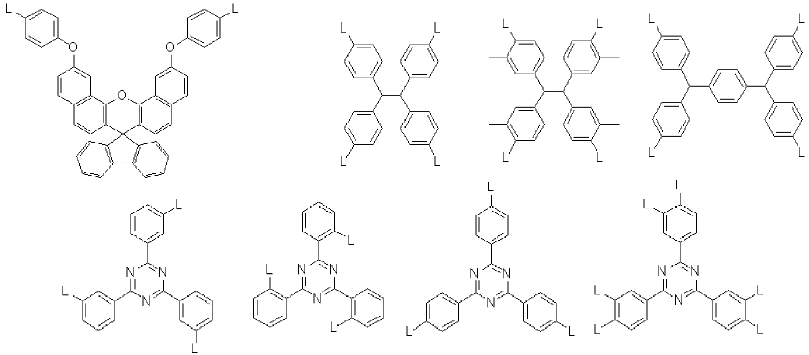

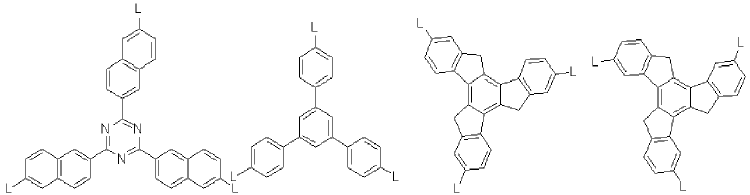

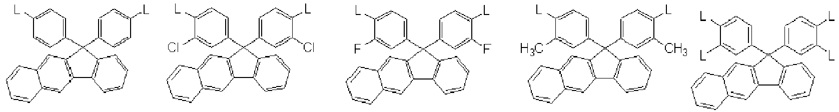

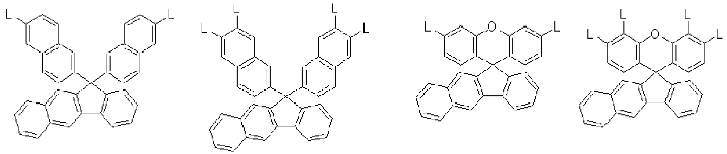

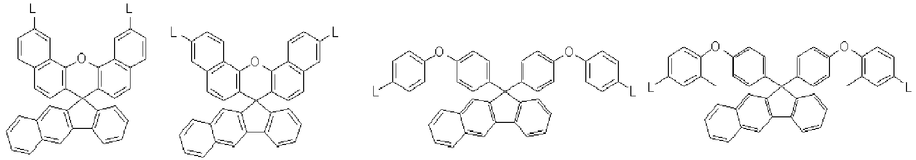

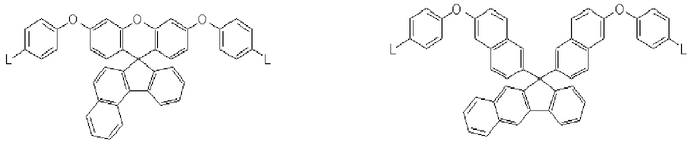

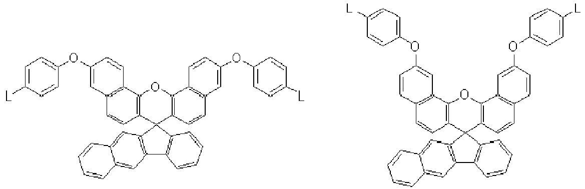

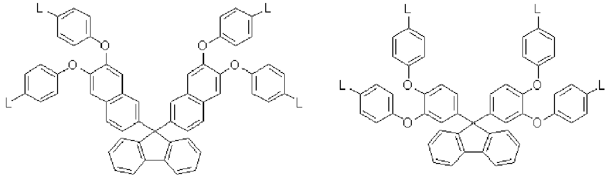

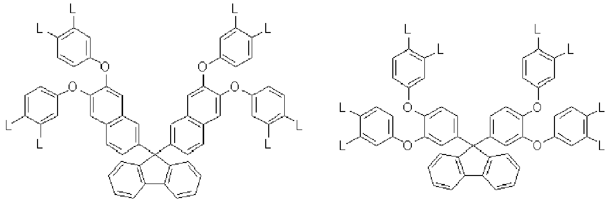

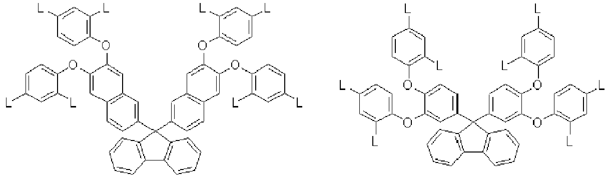

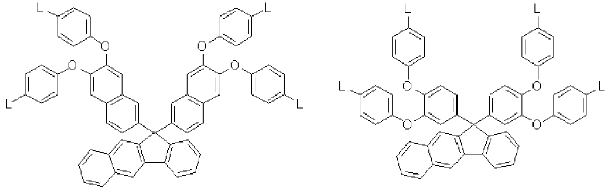

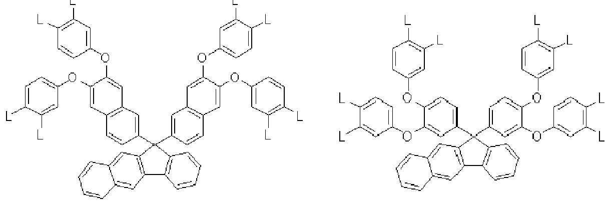

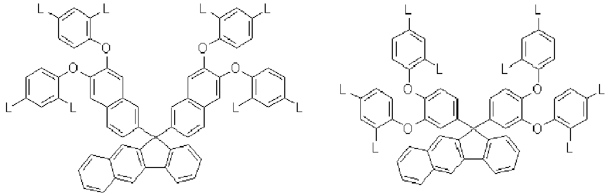

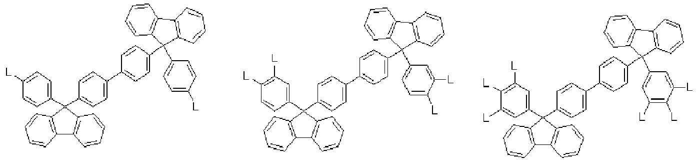

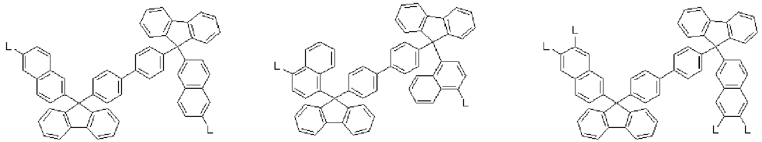

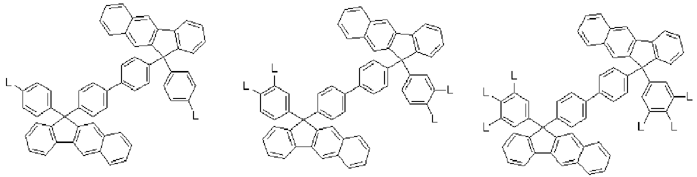

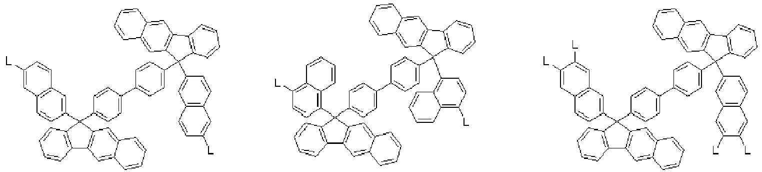

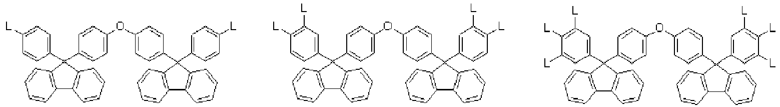

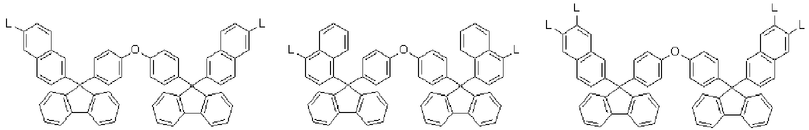

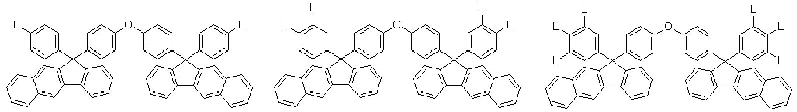

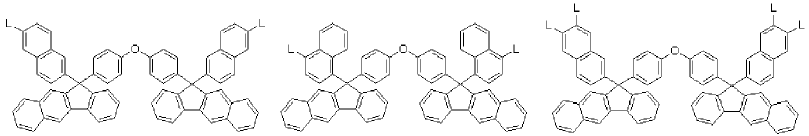

The total number "n₁" of the substituents L on the aromatic ring of the organic film compound represented by the general formula (1) preferably satisfies 3 ≤ n₁ ≤ 6.

Furthermore, the structural formula of the general formula (1) shown by W₁ and L is preferably a structural formula shown by the following general formula (8), that is, the substituents L on the aromatic ring are each preferably a substituent of an aromatic ring that is bonded to W₂, shown below, via an ether bond. W₂ represents an organic group having a valency of "n₆", "n₃" represents 1 or 2, and "n₆" represents an integer of 1 to 3. In this event, "n₁", which is the number of the substituents L on the aromatic ring in the general formula (1), is the product of "n₅" and "n₆", and satisfies the relationship 3 ≤ n₅ × n₆ ≤ 6. When a group linked to the W₂ is a substituent on an aromatic group bonded via an ether bond in this manner, thermal flowability can be provided without degrading heat resistance.

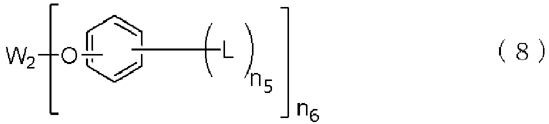

The following are preferable as the W₂ in the general formula (8). Such structures are preferable from the viewpoint of the availability of raw materials. 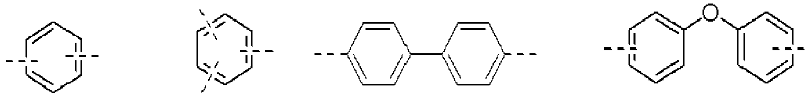 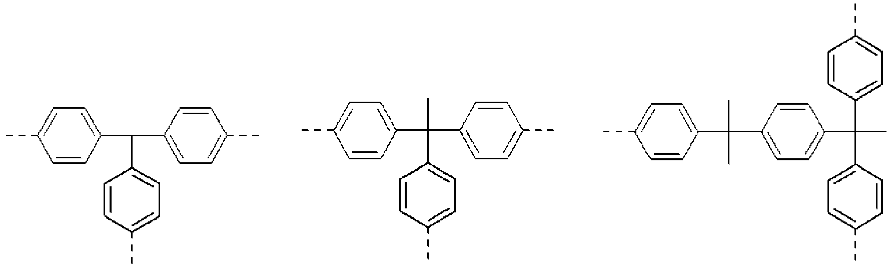 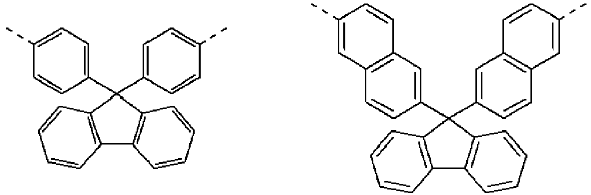 In the formulae, a broken line represents an attachment point.

In the L₁ or L₂, which are the substituents L on the aromatic ring, represented by the general formulae (2), the shown substitution is constituted by a substituent containing R₁ and R₂ via an oxygen atom or a nitrogen atom, and the structures of the substituents from the oxygen atom of the L₁ to the end and from the nitrogen atom of the L₂ to the end are identical. To express the terminal group structures, which are the identical structures, as R₄ for convenience, L₁ and L₂ can be shown as in the following general formulae (9). 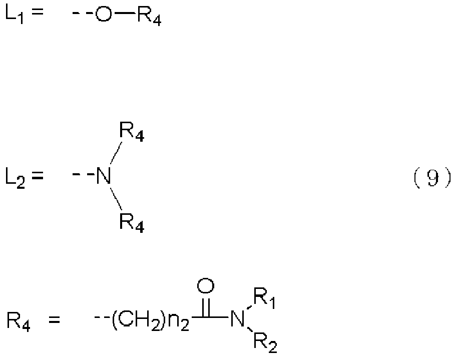 In the formulae, R₁, R₂, and "n₂" are as defined above.

As the R₄ in the L₁ and L₂ of the general formulae (9), the following structures can be given as examples. The alkylene chain linked between the attachment point in the R₄ and the amide bond is a linking group that contributes to the imparting of flowability and to film-formability, and by a combination with the curability of a crosslinking group having an unsaturated bond on the end, flatness and flowability are exhibited. By introducing an alkylene group, it is possible to alleviate crystallinity and provide thermal flowability and film-formability even when the skeleton represented by W₁ has a rigid structure. In particular, a structure in which n₂ = 1 and which contains a propargyl group or an ethynyl group is preferable from the viewpoint of heat resistance. Furthermore, a structure in which R₁ is not a hydrogen atom, that is, a structure having a tertiary amide structure having substituents other than hydrogen atoms as R₁ and R₂ are further preferable from the viewpoints of providing thermal flowability and complete solvent solubility. In the following formulae, "n₂" is as defined above, and a broken line represents an attachment point to the oxygen atom or nitrogen atom.

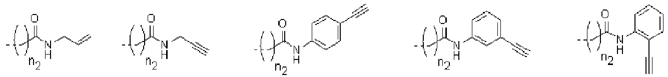

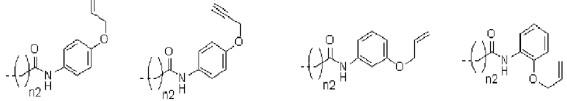

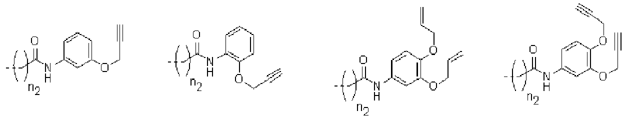

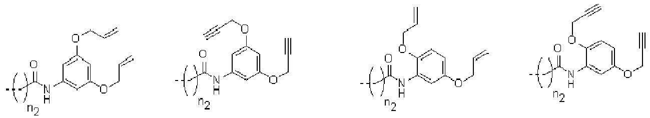

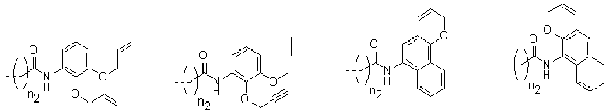

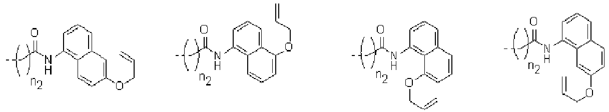

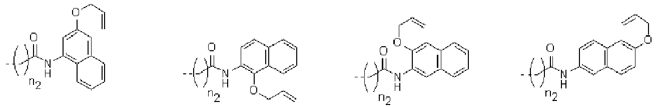

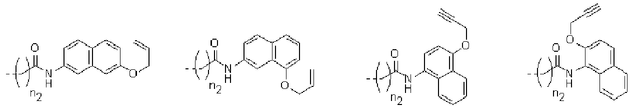

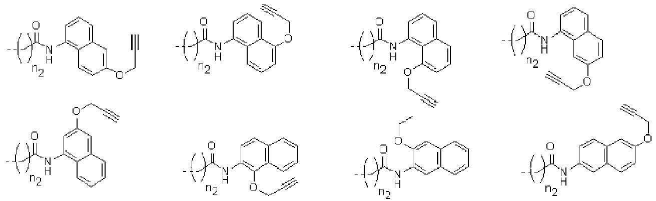

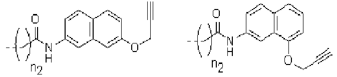

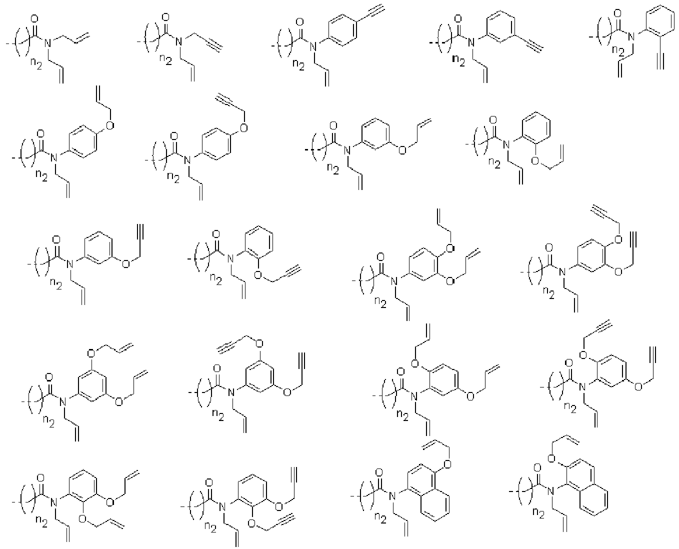

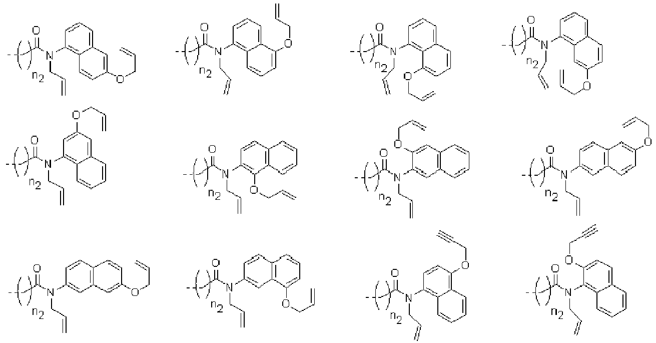

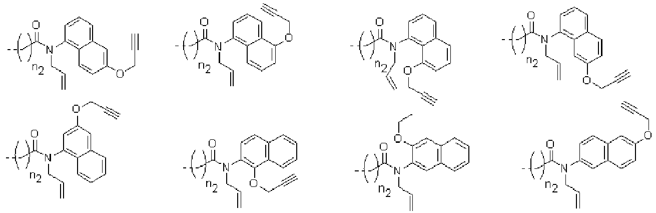

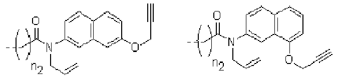

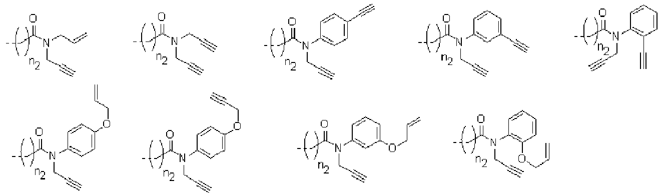

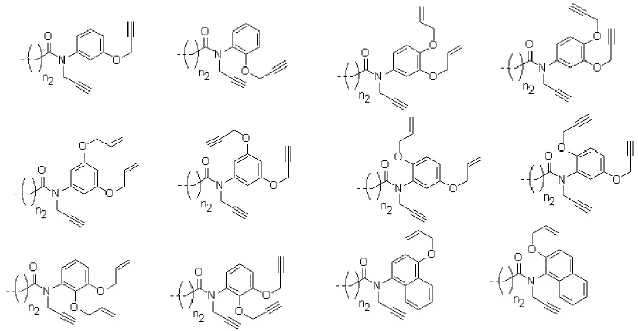

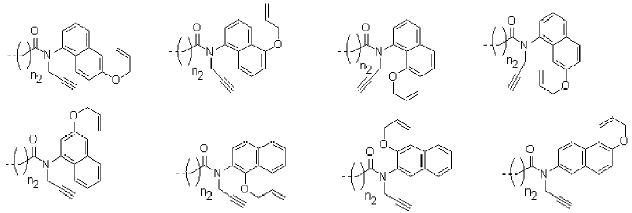

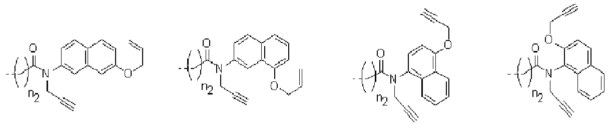

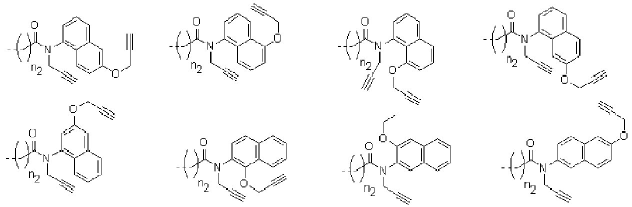

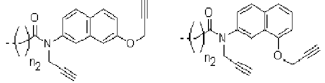

The L₁ and L₂ constituting the substituents L on the aromatic ring of the organic film compound represented by the general formula (1) are preferably represented by the following general formulae (6). The following are preferable from the viewpoints of the synthesis of the compound and the heat resistance of the obtained compound. 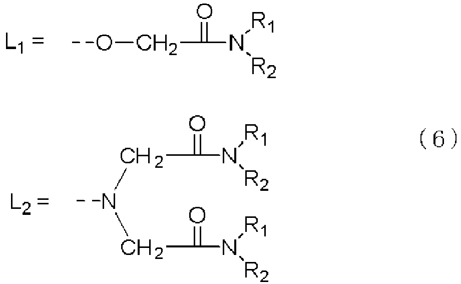 In the formulae, R₁ and R₂ are as defined above.

The R₁ and R₂ in the L₁ and L₂ constituting the substituents L on the aromatic ring of the organic film compound represented by the general formula (1) are preferably any of combinations shown by the following general formulae (7). Such combinations make it possible to form an organic film excellent in balanced heat resistance of thermosetting property and thermal flowability. 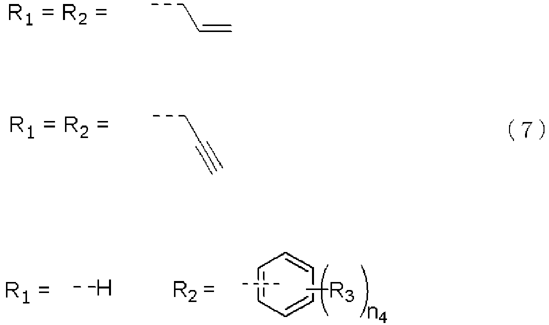 In the formula, R₃ and "n₄" are as defined above.

Furthermore, the substituents R₁ and R₂ on the L₁ and L₂ constituting the substituents L of the aromatic ring can be a combination of a plurality of structures. A specific method will be given in the manufacturing method, but the proportions of the substituents R₁ and R₂ on the L₁ and L₂ can be adjusted to any proportion by using a combination of reagents for introducing a plurality of terminal groups.

In addition, the organic film compound shown by the general formula (1) preferably satisfies 1.00 ≤ Mw/Mn ≤ 1.15, more preferably 1.00 ≤ Mw/Mn ≤ 1.10, where Mw is a weight-average molecular weight and Mn is a number-average molecular weight measured by gel permeation chromatography in terms of polystyrene. From the definition, Mw/Mn is 1.00 when the compound is a monomolecular compound. However, for reasons of separability in gel permeation chromatography, the measured value exceeds 1.00 in some cases. Generally, it is extremely difficult to bring Mw/Mn close to 1.00 in a polymer having a repeating unit unless a special polymerization method is used, there is a molecular weight distribution, and Mw/Mn becomes a value greater than 1. In the present invention, 1.00 ≤ Mw/Mn ≤ 1.15 has been defined as an index indicating monomerism in order to distinguish between a monomolecular compound and a polymer. In addition, in a case where a compound including a combination of terminal group structures is used as stated above, there is a distribution of Mw/Mn, unlike in the case of a single compound, and therefore, the above-described range of Mw/Mn has been set. Even in such a case, the distribution of Mw/Mn is not a large value, unlike in the case of a polymer.

By controlling the Mw/Mn of the organic film compound within such a range, an organic film excellent in filling property and flatness can be formed.

Thermosetting property, thermal flowability, and adhesiveness can be provided by the combination of the substituents introduced to the inventive organic film compound, and therefore, by using the inventive organic film compound, it is possible to form an organic film excellent in heat resistance, filling and planarizing properties, film-formability, etc.

### Method for Manufacturing Organic Film Compound

An example of a method for manufacturing the inventive organic film compound represented by the general formula (1) includes a method of obtaining the compound by a substitution reaction or the like using a base catalyst of a compound (modifier) having a leaving group X and having R₁ and R₂ as substituents and a so-called phenol, that is, a compound having a hydroxy group on an aromatic ring, or a so-called aniline, that is, a compound having an amino group as a substituent on an aromatic ring, as shown below. A single ingredient or two or more ingredients can be used in the reactions used in STEP 1 and STEP 2, and the ingredients can be selected appropriately and combined in accordance with required properties. In the following formulae, R₁, R₂, W₁, "n₁", and "n₂" are as defined above, and X represents a halogen atom, a tosylate group, or a mesylate group.

### Phenol (STEP 1)

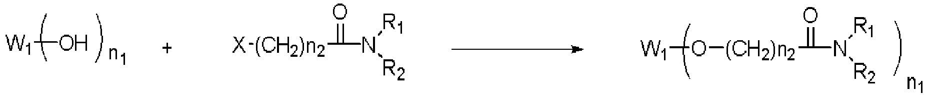

### Aniline (STEP 2)

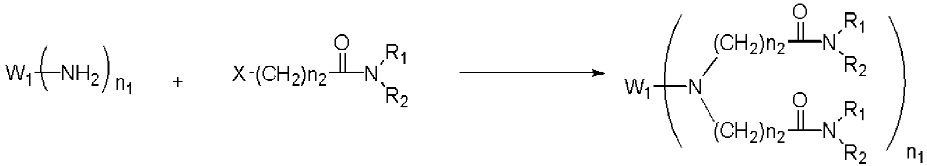

Furthermore, in a case where both a hydroxy group and an amino group are on an aromatic ring, the number of substituents in the ingredients can be adjusted so as to satisfy the relationships x + y = n₁, 2 ≤ n₁ ≤ 8, 0 ≤ x ≤ 8, and 0 ≤ y ≤ 8, where "x" is the number of hydroxy groups and "y" is the number of amino groups, as shown below (Pattern 1). In addition, the hydroxy group or amino group on the aromatic ring can be subjected to a reaction by using a plurality of modifiers at any proportion to control the introduction ratio of the substituents on the hydroxy group or amino group at any ratio. For example, in a case where the modifier has substituents having different combinations of two kinds of substituents R₁ₐ and R₂ₐ and substituents R_{1b} and R_{2b}, the introduction ratio can be controlled to any ratio in such a manner that a + b = 100%, where the introduction rate of the substituent having R₁ₐ and R₂ₐ is a% and the introduction rate of the substituent having R_{1b} and R_{2b} is b% (Pattern 2 and Pattern 3). In this manner, the introduction ratio can also be controlled in a case where both a hydroxy group and an amino group are contained as in Pattern 1. Furthermore, the introduction rate can also be controlled to any proportion so as to achieve a substituent introduction rate of 100% when three or more kinds of modifiers are used. In the following formulae, W₁, R₁, and R₂ are as defined above, and "x" and "y" represent integers that satisfy x + y = n₁. R₁ₐ, R₂ₐ, R_{1b}, and R_{2b} are either of the above-described R₁ and R₂, and satisfy the relationships R₁ₐ ≠ R_{1b} and R₂ₐ ≠ R_{2b}. When the proportion of the R₁ₐ and R₂ₐ contained as substituents included in "n₁" is a% and the proportion of the R_{1b} and R_{2b} contained as substituents included in "n₁" is b%, "a" and "b" satisfy a + b = 100%, n₁ₐ equals n₁ × a ÷ 100, n_{1b} equals n₁ × b ÷ 100, and "n₁ₐ", "n_{1b}", and "n₁" satisfy the relationship n₁ₐ + n_{1b} = n₁.

### Pattern 1

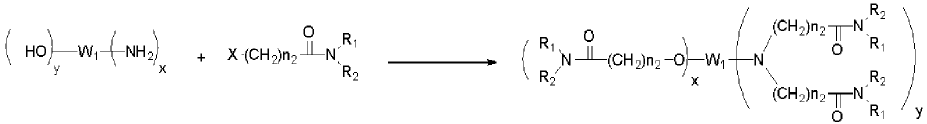

### Pattern 2

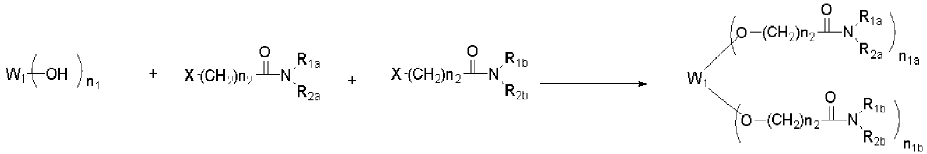

### Pattern 3

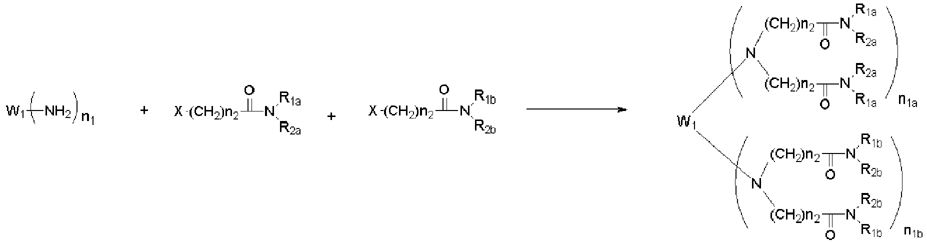

Examples of the base catalyst of the substitution reaction shown above include inorganic base compounds, such as sodium hydrogen carbonate, sodium carbonate, potassium carbonate, calcium carbonate, cesium carbonate, sodium hydroxide, potassium hydroxide, sodium hydride, and potassium phosphate; organic amine compounds, such as triethyl amine, pyridine, and N-methylmorpholine; and the like. One of these may be used, or two or more thereof may be used in combination. The amount of the catalyst to be used can be 0.1 to 20 mol, preferably 0.2 to 10 mol relative to the hydroxy groups or amino groups, which are substituents of the ingredients.

The solvent used in this event is not particularly limited, as long as the solvent is inert in the above reaction. Examples of the solvent include ether-based solvents, such as diethyl ether, tetrahydrofuran, and dioxane; aromatic solvents, such as benzene, toluene, and xylene; acetonitrile, dimethylsulfoxide, N,N-dimethylformamide, N-methylpyrrolidone, water, and the like. One of these or a mixture of two or more thereof can be used. These solvents can be used in a range of 0 to 2,000 parts by mass based on 100 parts by mass of the reactants. The reaction temperature is preferably -50°C to approximately the boiling point of the solvent, and room temperature to 150°C is even more preferable. Reaction time is appropriately selected from 0.1 to 100 hours.

Reaction methods include: a method of charging the phenol or aniline and the modifier into the solvent at once; a method of dispersing or dissolving each of the phenol or aniline and the modifier or a mixture thereof and charging the resultant dropwise; a method of dispersing or dissolving one of the phenol or aniline and the modifier in the solvent, and then charging the other one, dispersed or dissolved in the solvent, by adding dropwise; etc. In addition, in a case where multiple kinds of each of the phenol or aniline and the modifier are to be charged, it is possible to mix the compounds beforehand and allow the mixture to react, and it is also possible to allow the compounds to react individually in succession. In a case where a catalyst is to be used, methods include: a method of charging the phenol or aniline and the modifier at once; a method of dispersing or dissolving the catalyst beforehand, and then adding dropwise; etc. After completion of the reaction, the resultant can be diluted in an organic solvent in order to remove unreacted ingredients, the catalyst, etc. present in the system, and then a crystal can be precipitated to obtain a powder by liquid-liquid separation washing or with a poor solvent. In addition, after the dilution in the organic solvent, solvent substitution to a desired organic solvent can be performed, and the product can be collected as a solution. After further collecting the product as a powder by precipitating a crystal or the like, it is also possible to dissolve the powder in a desired organic solvent and collect the product as a solution.

The organic solvent in a case where liquid-liquid separation washing is performed is not particularly limited, as long as the organic solvent is capable of dissolving the target compounds and is separated into two layers even when mixed with water. Examples of the organic solvent include hydrocarbons, such as hexane, heptane, benzene, toluene, and xylene; esters, such as ethyl acetate, n-butyl acetate, and propylene glycol methyl ether acetate; ketones, such as methyl ethyl ketone, methyl amyl ketone, cyclohexanone, and methyl isobutyl ketone; ethers, such as diethyl ether, diisopropyl ether, methyl-t-butyl ether, and ethylcyclopentylmethyl ether; chlorinated solvents, such as methylene chloride, chloroform, dichloroethane, and trichloroethylene; mixtures thereof; etc. As washing water used in this event, generally, what is called deionized water or ultrapure water may be used. The washing may be performed one or more times, preferably approximately one to five times because washing ten times or more does not always produce the full washing effects thereof.

In the liquid-liquid separation washing, the washing may be performed with a basic aqueous solution to remove the acidic components in the system. The base specifically includes hydroxides of alkaline metals, carbonates of alkaline metals, hydroxides of alkali earth metals, carbonates of alkali earth metals, ammonia, organic ammonium, etc.

Further, in the liquid-liquid separation washing, the washing may be performed with an acidic aqueous solution to remove the metal impurities or basic components in the system. The acid specifically includes inorganic acids, such as hydrochloric acid, hydrobromic acid, sulfuric acid, nitric acid, phosphoric acid, and heteropoly acid; organic acids, such as oxalic acid, fumaric acid, maleic acid, trifluoroacetic acid, methanesulfonic acid, benzenesulfonic acid, p-toluenesulfonic acid, and trifluoromethanesulfonic acid; etc.

The liquid-liquid separation washing may be performed with either one of the basic aqueous solution and the acidic aqueous solution, or can be performed with a combination of the two. The liquid-liquid separation washing is preferably performed with the basic aqueous solution and the acidic aqueous solution in this order from the viewpoint of removing the metal impurities.

After the liquid-liquid separation washing with the basic aqueous solution and the acidic aqueous solution, washing with neutral water may be successively performed. The washing may be performed one or more times, preferably approximately one to five times. As the neutral water, deionized water, ultrapure water, or the like as mentioned above may be used. The washing may be performed one or more times, but if the washing is not performed sufficiently, the basic components and acidic components cannot be removed in some cases. The washing is preferably performed approximately one to five times because washing ten times or more does not always produce the full washing effects thereof.

Further, the reaction product after the liquid-liquid separation can also be collected as a powder by concentrating and drying up the solvent or crystallizing the reaction product under reduced pressure or normal pressure. Alternatively, the reaction product can also be retained in the state of solution with an appropriate concentration to improve the workability in preparing the material for forming an organic film. The concentration in this event is preferably 0.1 to 50 mass%, more preferably 0.5 to 30 mass%. With such a concentration, the viscosity is hardly increased, and therefore, workability does not become degraded; in addition, since the amount of the solvent is not excessive, it is economical.

The solvent in this event is not particularly limited, as long as the solvent is capable of dissolving the organic film compound. Specific examples of the solvent include ketones, such as cyclohexanone and methyl-2-amyl ketone; alcohols, such as 3-methoxybutanol, 3-methyl-3-methoxybutanol, 1-methoxy-2-propanol, and 1-ethoxy-2-propanol; ethers, such as propylene glycol monomethyl ether, ethylene glycol monomethyl ether, propylene glycol monoethyl ether, ethylene glycol monoethyl ether, propylene glycol dimethyl ether, and diethylene glycol dimethyl ether; and esters, such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate. One of these or a mixture of two or more thereof can be used.

To prepare the organic film compound used in the material for forming an organic film obtained by this method, one of the above-described various modifiers can be used or a combination of two or more thereof can be used according to the required performance. For example, those having a side chain structure that contributes to improvement of planarizing property, an aromatic ring structure that contributes to etching resistance and heat resistance, and so forth can be combined at any proportion. Therefore, a material for forming an organic film containing these organic film compounds can achieve not only higher filling and planarizing properties and film-formability but also higher etching resistance and optical characteristics.

As described above, the inventive organic film compound gives a material for forming an organic film that can exhibit excellent filling and planarizing properties and film-formability.

Note that one kind of the inventive organic film compound may be used, or two or more kinds thereof may be used in combination.

### Organic Solvent

The organic solvent that can be used in the inventive material for forming an organic film is not particularly limited as long as the solvent can dissolve the inventive organic film compound described above and optional components, such as a surfactant, plasticizer, acid generator, crosslinking agent, and other additives. Specifically, organic solvents with a boiling point of lower than 180°C can be used, such as those disclosed in paragraphs [0091] and [0092] of JP 2007-199653 A. Above all, propylene glycol monomethyl ether acetate, propylene glycol monomethyl ether, 2-heptanone, cyclopentanone, cyclohexanone, and a mixture of two or more thereof are preferably used.

Such a material for forming an organic film can be applied by spin-coating, and has heat resistance and high filling and planarizing properties because the inventive organic film compound described above is incorporated.

Further, the inventive material for forming an organic film may contain an organic solvent in which a high-boiling-point solvent having a boiling point of 180°C or higher is added to the aforementioned organic solvent having a boiling point of lower than 180°C (a mixture of an organic solvent having a boiling point of lower than 180°C and an organic solvent having a boiling point of 180°C or higher). The high-boiling-point organic solvent is not particularly limited to hydrocarbons, alcohols, ketones, esters, ethers, chlorinated solvents, and so forth as long as the high-boiling-point organic solvent is capable of dissolving the organic film compound and optional components, such as a surfactant, plasticizer, acid generator, crosslinking agent, and other additives. Specific examples of the high-boiling-point organic solvent include 1-octanol, 2-ethylhexanol, 1-nonanol, 1-decanol, 1-undecanol, ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, glycerin, n-nonyl acetate, ethylene glycol monohexyl ether, ethylene glycol mono-2-ethylhexyl ether, ethylene glycol monophenyl ether, ethylene glycol monobenzyl ether, diethylene glycol monoethyl ether, diethylene glycol monoisopropyl ether, diethylene glycol mono-n-butyl ether, diethylene glycol monoisobutyl ether, diethylene glycol monohexyl ether, diethylene glycol monophenyl ether, diethylene glycol monobenzyl ether, diethylene glycol diethyl ether, diethylene glycol dibutyl ether, diethylene glycol butylmethyl ether, triethylene glycol dimethyl ether, triethylene glycol monomethyl ether, triethylene glycol-n-butyl ether, triethylene glycol butylmethyl ether, triethylene glycol diacetate, tetraethylene glycol dimethyl ether, dipropylene glycol monomethyl ether, dipropylene glycol mono-n-propyl ether, dipropylene glycol mono-n-butyl ether, tripropylene glycol dimethyl ether, tripropylene glycol monomethyl ether, tripropylene glycol mono-n-propyl ether, tripropylene glycol mono-n-butyl ether, ethylene glycol monoethyl ether acetate, ethylene glycol monobutyl ether acetate, diethylene glycol monomethyl ether acetate, diethylene glycol monoethyl ether acetate, diethylene glycol monobutyl ether acetate, triacetin, propylene glycol diacetate, dipropylene glycol monomethyl ether acetate, dipropylene glycol methyl-n-propyl ether, dipropylene glycol methyl ether acetate, 1,4-butanediol diacetate, 1,3-butylene glycol diacetate, 1,6-hexanediol diacetate, triethylene glycol diacetate, γ-butyrolactone, dihexyl malonate, diethyl succinate, dipropyl succinate, dibutyl succinate, dihexyl succinate, dimethyl adipate, diethyl adipate, dibutyl adipate, and the like. One of these or a mixture thereof may be used.

The boiling point of the high-boiling-point solvent may be appropriately selected according to the temperature at which the material for forming an organic film is heated. The boiling point of the high-boiling-point solvent to be contained is preferably 180°C to 300°C, further preferably 200°C to 300°C. Such a boiling point prevents the evaporation rate at baking (heating) from becoming excessive, which would otherwise occur if the boiling point is too low. Thus, sufficient thermal flowability can be achieved. Meanwhile, with such a boiling point, the high-boiling-point solvent does not remain in the film without evaporating after baking due to the boiling point being too high; thus, the boiling point in these ranges does not adversely affect the film physical properties, such as etching resistance.

When the high-boiling-point solvent is used, the contained amount of the high-boiling-point solvent is preferably 1 to 30 parts by mass based on 100 parts by mass of the organic solvent having a boiling point of lower than 180°C. When the contained amount is within this range, there is no risk of sufficient thermal flowability not being provided at the time of baking due to the contained amount being too small or risk of the solvent remaining in the film and causing degradation in film physical properties, such as etching resistance, due to the contained amount being too large.

With such a material for forming an organic film, the above-described organic film compound is provided with thermal flowability by the addition of the high-boiling-point solvent, so that the material for forming an organic film also has high filling and planarizing properties.

In the present invention, the material for forming an organic film preferably further contains at least one of a surfactant and a plasticizer.

### Surfactant

In the inventive material for forming an organic film, a surfactant can be contained so as to enhance the coating property in spin-coating. As the surfactant, for example, those disclosed in paragraphs [0142] to [0147] of JP 2009-269953 A can be used.

One kind of the surfactant may be contained, or two or more kinds thereof may be contained in combination. When a surfactant is contained, the contained amount is preferably 0.01 to 10 parts by mass, more preferably 0.05 to 5 parts by mass based on 100 parts by mass of the organic film compound.

### Plasticizer

Further, in the inventive material for forming an organic film, a plasticizer can be contained so as to enhance the planarizing and filling properties further. The plasticizer is not particularly limited, and known various types of plasticizers can be widely used. Examples thereof include low-molecular-weight compounds, such as phthalic acid esters, adipic acid esters, phosphoric acid esters, trimellitic acid esters, and citric acid esters; and polymers such as polyethers, polyesters, and polyacetal-based polymers disclosed in JP 2013-253227 A. When a plasticizer is contained, the contained amount is preferably 1 to 100 parts by mass, more preferably 5 to 30 parts by mass based on 100 parts by mass of the organic film compound.

Furthermore, like the plasticizer, as an additive for providing the inventive material for forming an organic film with filling and planarizing properties, it is preferable to use, for example, liquid additives having polyethylene glycol or polypropylene glycol structures, or thermo-decomposable polymers having a weight loss ratio on heating from 30°C to 250°C of 40 mass% or more and a weight-average molecular weight of 300 to 200,000. The thermo-decomposable polymers preferably contain a repeating unit having an acetal structure shown by the following general formula (DP1) or (DP1a). When these thermo-decomposable polymers are contained, the contained amount is preferably 1 to 100 parts by mass, more preferably 5 to 30 parts by mass based on 100 parts by mass of the organic film compound. 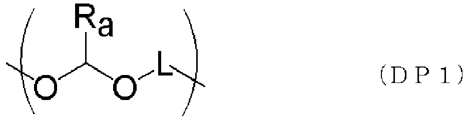 In the formula, Rₐ represents a hydrogen atom or a saturated or unsaturated monovalent organic group having 1 to 30 carbon atoms which may be substituted. L represents a saturated or unsaturated divalent organic group having 2 to 30 carbon atoms. 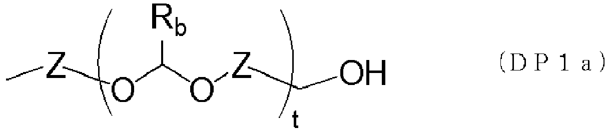 In the formula, R_{b} represents an alkyl group having 1 to 4 carbon atoms. Z represents a saturated or unsaturated divalent hydrocarbon group having 4 to 10 carbon atoms which may have an ether bond. "t" represents an average repeating unit number of 3 to 500.

Furthermore, it is possible to use the following flowability accelerator as an additive for improving filling and planarizing properties in the same manner as above. As the flowability accelerator, it is possible to use one or more compounds selected from the following general formulae (i) to (iii). When these flowability accelerators are contained, the contained amount is preferably 1 to 100 parts by mass, more preferably 5 to 30 parts by mass based on 100 parts by mass of the organic film compound. 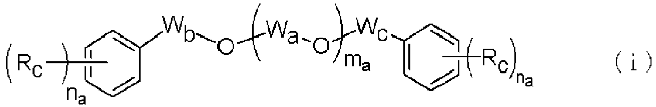 In the formula, each R_{c} independently represents a hydrogen atom, a hydroxy group, or an organic group having 1 to 10 carbon atoms and optionally being substituted. Wₐ represents a phenylene group or a divalent group represented by the following general formula (i-1). W_{b} and W_{c} each represent a single bond or a divalent group represented by one of the following formulae (i-2). "mₐ" represents an integer of 1 to 10, and "nₐ" represents an integer of 0 to 5. 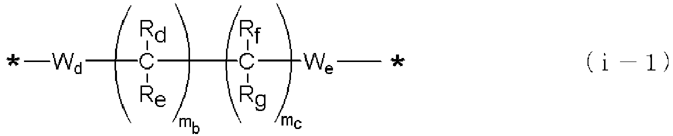 In the formula, "*" represents an attachment point, and R_{d}, Rₑ, R_{f}, and R_{g} each represent a hydrogen atom, a hydroxy group, or an organic group having 1 to 10 carbon atoms. W_{d} and Wₑ each independently represent a single bond or a carbonyl group. "m_{b}" and "m_{c}" each represent an integer of 0 to 10, and satisfy m_{b} + m_{c} ≥ 1. 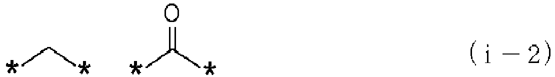 In the formulae, "*" represents an attachment point. 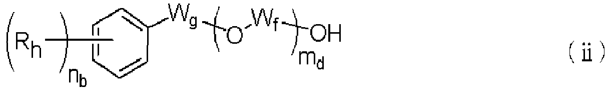 In the formulae, each Rₕ independently represents a hydrogen atom or an organic group having 1 to 10 carbon atoms and optionally being substituted. Wε represents a divalent group represented by the following general formula (ii-1). W_{g} represents a single bond or a divalent group represented by the following formula (ii-2). "m_{d}" represents an integer of 2 to 10, and "n_{b}" represents an integer of 0 to 5. 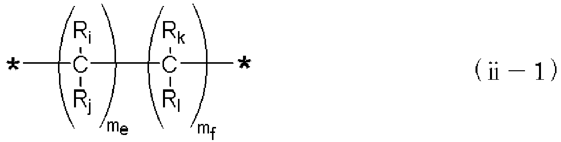 In the formula, "*" represents an attachment point, and Rᵢ, Rⱼ, Rₖ, and Rₗ each represent a hydrogen atom, a hydroxy group, or an organic group having 1 to 10 carbon atoms. "mₑ" and "m_{f}" each represent an integer of 0 to 10, and satisfy mₑ + mε ≥ 1. 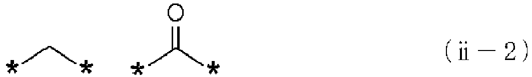 In the formulae, "*" represents an attachment point. 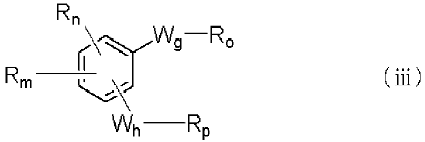 In the formula, Rₘ and Rₙ each represent a hydrogen atom, a hydroxy group, or an organic group having 1 to 10 carbon atoms and optionally being substituted, and may be bonded to each other to form a cyclic structure. Rₒ and R_{b} each represent an organic group having 1 to 10 carbon atoms, and Rₒ is a group that contains either one of an aromatic ring or a divalent group represented by the following general formula (iii-1). W_{g} and Wₕ each represent a single bond or a divalent group represented by the following formula (iii-2), and at least one of W_{g} and Wₕ is a divalent group represented by one of the following formulae (iii-2). 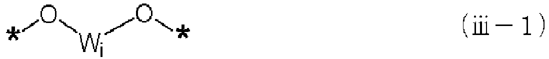 In the formula, "*" represents an attachment point, and Wᵢ represents an organic group having 1 to 4 carbon atoms. 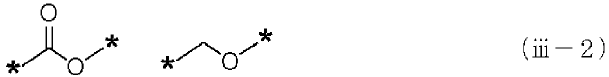 In the formulae, "*" represents an attachment point.

### Acid Generator

In the inventive material for forming an organic film, an acid generator can be contained so as to further promote the curing reaction. The acid generator includes a material that generates an acid by thermal decomposition and a material that generates an acid by light irradiation. Any acid generator can be contained. Specifically, materials disclosed in paragraphs [0061] to [0085] of JP 2007-199653 A can be contained, but the present invention is not limited thereto.

One of the acid generators or a combination of two or more thereof can be used. When an acid generator is contained, the contained amount is preferably 0.05 to 50 parts by mass, more preferably 0.1 to 10 parts by mass, based on 100 parts by mass of the above-described organic film compound.

### Crosslinking Agent

Moreover, in the inventive material for forming an organic film, a crosslinking agent can also be contained so as to increase the curability and to further suppress intermixing with a resist upper layer film. The crosslinking agent is not particularly limited, and known various types of crosslinking agents can be widely used. Examples thereof include melamine-based crosslinking agents, glycoluril-based crosslinking agents, benzoguanamine-based crosslinking agents, urea-based crosslinking agents, β-hydroxyalkylamide-based crosslinking agents, isocyanurate-based crosslinking agents, aziridine-based crosslinking agents, oxazoline-based crosslinking agents, epoxy-based crosslinking agents, and polynuclear phenol-based crosslinking agents.

Specific examples of the melamine-based crosslinking agents include hexamethoxymethylated melamine, hexabutoxymethylated melamine, alkoxy- and/or hydroxy-substituted derivatives thereof, and partial self-condensates thereof.

Specific examples of the glycoluril-based crosslinking agents include tetramethoxymethylated glycoluril, tetrabutoxymethylated glycoluril, alkoxy- and/or hydroxy-substituted derivatives thereof, and partial self-condensates thereof.

Specific examples of the benzoguanamine-based crosslinking agents include tetramethoxymethylated benzoguanamine, tetrabutoxymethylated benzoguanamine, alkoxy- and/or hydroxy-substituted derivatives thereof, and partial self-condensates thereof.

Specific examples of the urea-based crosslinking agents include dimethoxymethylated dimethoxyethyleneurea, alkoxy- and/or hydroxy-substituted derivatives thereof, and partial self-condensates thereof.

A specific example of the β-hydroxyalkylamide-based crosslinking agents includes N,N,N',N'-tetra(2-hydroxyethyl)adipic acid amide.

Specific examples of the isocyanurate-based crosslinking agents include triglycidyl isocyanurate and triallyl isocyanurate.

Specific examples of the aziridine-based crosslinking agents include 4,4'-bis(ethyleneiminocarbonylamino)diphenylmethane and 2,2-bishydroxymethylbutanol-tris[3-(1-aziridinyl)propionate].

Specific examples of the oxazoline-based crosslinking agents include 2,2'-isopropylidene bis(4-benzyl-2-oxazoline), 2,2'-isopropylidene bis(4-phenyl-2-oxazoline), 2,2'-methylenebis4,5-diphenyl-2-oxazoline, 2,2'-methylenebis-4-phenyl-2-oxazoline, 2,2'-methylenebis-4-tert-butyl-2-oxazoline, 2,2'-bis(2-oxazoline), 1,3-phenylenebis(2-oxazoline), 1,4-phenylenebis(2-oxazoline), and a 2-isopropenyloxazoline copolymer.

Specific examples of the epoxy-based crosslinking agents include diglycidyl ether, ethylene glycol diglycidyl ether, 1,4-butanediol diglycidyl ether, 1,4-cyclohexanedimethanol diglycidyl ether, poly(glycidyl methacrylate), trimethylolethane triglycidyl ether, trimethylolpropane triglycidyl ether, and pentaerythritol tetraglycidyl ether.

Specific examples of the polynuclear-phenol-based crosslinking agents include compounds shown by the following general formula (1A). 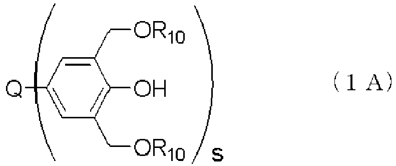 In the formula, Q represents a single bond or a hydrocarbon group with a valency of "s" having 1 to 20 carbon atoms. R₁₀ represents a hydrogen atom or an alkyl group having 1 to 20 carbon atoms. "s" represents an integer of 1 to 5.

Q represents a single bond or a hydrocarbon group with a valency of "s" having 1 to 20 carbon atoms. "s" represents an integer of 1 to 5, more preferably 2 or 3. Specific examples of Q include groups obtained by removing "s" hydrogen atoms from methane, ethane, propane, butane, isobutane, pentane, cyclopentane, hexane, cyclohexane, methyl pentane, methyl cyclohexane, dimethyl cyclohexane, trimethyl cyclohexane, benzene, toluene, xylene, ethyl benzene, ethyl isopropylbenzene, diisopropylbenzene, methylnaphthalene, ethyl naphthalene, and eicosane. R₁₀ represents a hydrogen atom or an alkyl group having 1 to 20 carbon atoms. Specific examples of the alkyl group having 1 to 20 carbon atoms include a methyl group, an ethyl group, a propyl group, an isopropyl group, a butyl group, an isobutyl group, a pentyl group, an isopentyl group, a hexyl group, an octyl group, an ethylhexyl group, a decyl group, and an eicosanyl group. A hydrogen atom or a methyl group is preferable.

Specific examples of the compound shown by the general formula (1A) include the following compounds. In particular, in view of enhancing the curability and film thickness uniformity of the organic film, hexamethoxymethylated compounds of triphenolmethane, triphenolethane, 1,1,1-tris(4-hydroxyphenyl)ethane, and tris(4-hydroxyphenyl)-1-ethyl-4-isopropylbenzene are preferable. In the formulae, R₁₀ is as defined above.

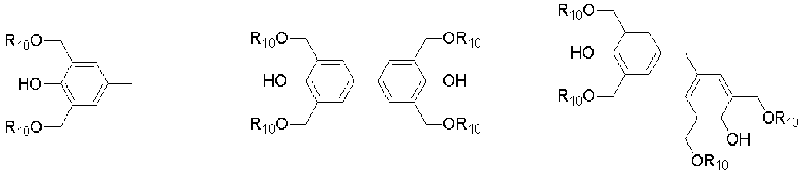

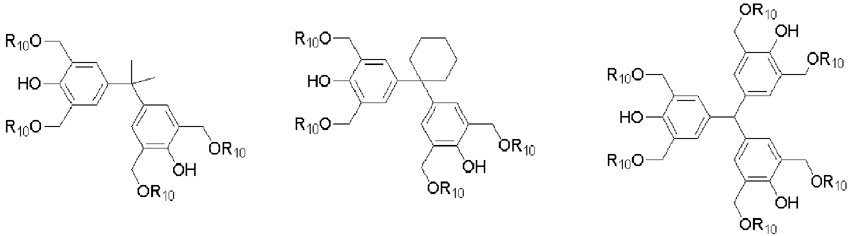

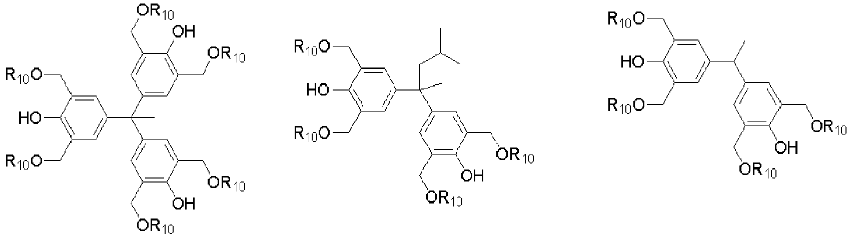

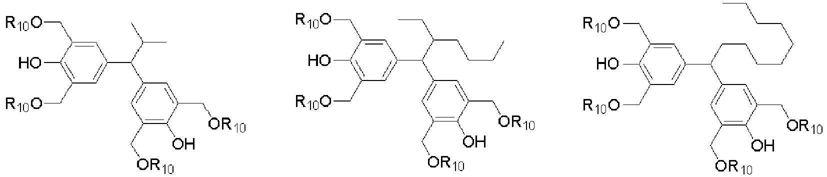

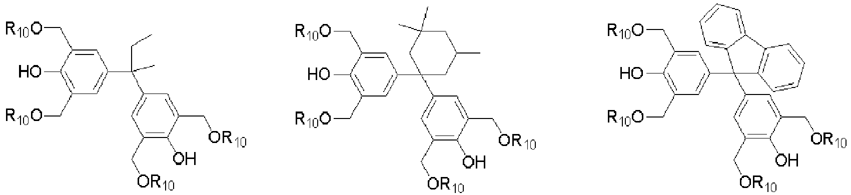

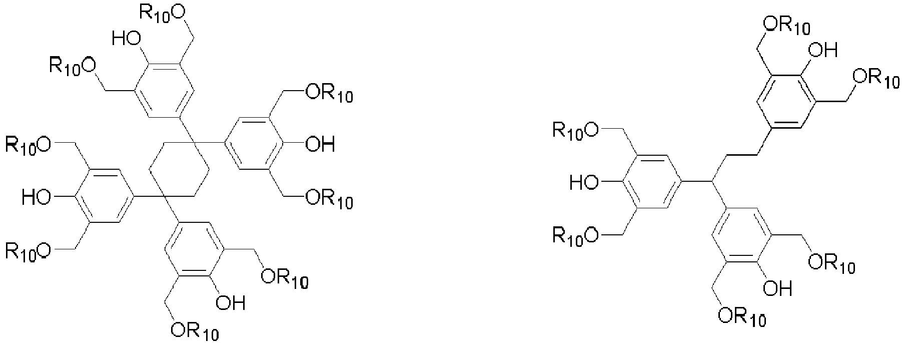

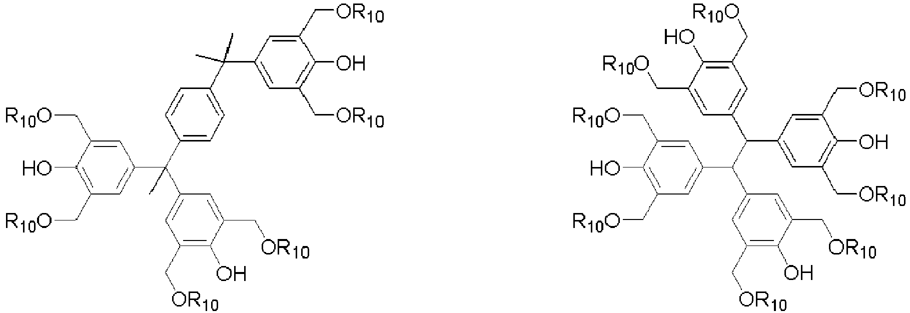

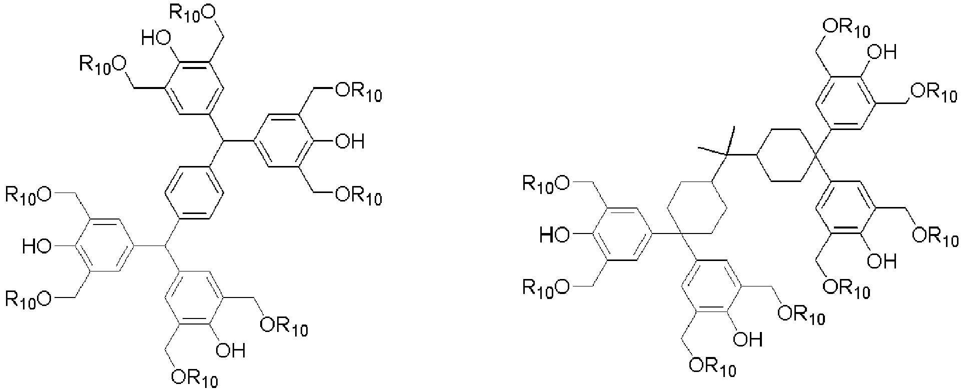

One kind of the crosslinking agent may be used, or a combination of two or more kinds thereof may be used. The crosslinking agent is preferably contained in an amount of 1 to 100 parts by mass, more preferably 5 to 50 parts by mass based on 100 parts by mass of the organic film compound.

### Other Components

The inventive material for forming an organic film may be further blended with a different compound or polymer. The blend compound or blend polymer mixed with the inventive material for forming an organic film serves to improve the film-formability by spin-coating and the filling property for a stepped substrate.

Examples of such a material include novolak resins of phenol, o-cresol, m-cresol, p-cresol, 2,3-dimethylphenol, 2,5-dimethylphenol, 3,4-dimethylphenol, 3,5-dimethylphenol, 2,4-dimethylphenol, 2,6-dimethylphenol, 2,3,5-trimethylphenol, 3,4,5-trimethylphenol, 2-tert-butylphenol, 3-tert-butylphenol, 4-tert-butylphenol, 2-phenylphenol, 3-phenylphenol, 4-phenylphenol, 3,5-diphenylphenol, 2-naphthylphenol, 3-naphthylphenol, 4-naphthylphenol, 4-tritylphenol, resorcinol, 2-methylresorcinol, 4-methylresorcinol, 5-methylresorcinol, catechol, 4-tert-butylcatechol, 2-methoxyphenol, 3-methoxyphenol, 2-propylphenol, 3-propylphenol, 4-propylphenol, 2-isopropylphenol, 3-isopropylphenol, 4-isopropylphenol, 2-methoxy-5-methylphenol, 2-tert-butyl-5-methylphenol, pyrogallol, thymol, isothymol, 4,4'-(9H-fluorene-9-ylidene)bisphenol, 2,2'dimethyl-4,4'-(9H-fluorene-9-ylidene)bisphenol, 2,2'diallyl-4,4'-(9H-fluorene-9-ylidene)bisphenol, 2,2'difluoro-4,4'-(9H-fluorene-9-ylidene)bisphenol, 2,2'diphenyl-4,4'-(9H-fluorene-9-ylidene)bisphenol, 2,2'dimethoxy-4,4'-(9H-fluorene-9-ylidene)bisphenol, 2,3,2',3'-tetrahydro-(1,1')-spirobiindene-6,6'-diol, 3,3,3',3'-tetramethyl-2,3,2',3'-tetrahydro-(1,1')-spirobiindene-6,6'-diol, 3,3,3',3',4,4'-hexamethyl-2,3,2',3'-tetrahydro-(1,1')-spirobiindene-6,6'-diol, 2,3,2',3'-tetrahydro-(1,1')-spirobiindene-5,5'-diol, 5,5'-dimethyl-3,3,3',3'-tetramethyl-2,3,2',3'-tetrahydro-(1,1')-spirobiindene-6,6'-diol, 1-naphthol, 2-naphthol, 2-methyl-1-naphthol, 4-methoxy-1-naphthol, and 7-methoxy-2-naphthol, dihydroxynaphthalenes, such as 1,5-dihydroxynaphthalene, 1,7-dihydroxynaphthalene, and 2,6-dihydroxynaphthalene, methyl 3-hydroxynaphthalene-2-carboxylate, indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, 5-vinylnorborna-2-ene, α-pinene, β-pinene, limonene, etc.; and polyhydroxystyrene, polystyrene, polyvinylnaphthalene, polyvinylanthracene, polyvinylcarbazole, polyindene, polyacenaphthylene, polynorbornene, polycyclodecene, polytetracyclododecene, polynortricyclene, poly(meth)acrylate, and copolymers thereof. It is also possible to blend a naphthol dicyclopentadiene copolymer disclosed in JP 2004-205685 A, a fluorene bisphenol novolak resin disclosed in JP 2005-128509 A, an acenaphthylene copolymer disclosed in JP 2005-250434 A, fullerene having a phenol group disclosed in JP 2006-227391 A, a bisphenol compound and a novolak resin thereof disclosed in JP 2006-293298 A, an adamantane phenol compound and a novolak resin thereof disclosed in JP 2006-285095 A, a bisnaphthol compound and a novolak resin thereof disclosed in JP 2010-122656 A, a fullerene resin compound disclosed in JP 2008-158002 A, or the like.

The blended amount of the blend compound or the blend polymer is preferably 0 to 1,000 parts by mass, more preferably 0 to 500 parts by mass based on 100 parts by mass of the organic film compound.

The material for forming an organic film can be used for an organic film material or a planarizing material for manufacturing a semiconductor device.

In addition, the inventive material for forming an organic film is extremely useful as an organic film material in a multilayer resist process such as a 2-layer resist process, a 3-layer resist process using a silicon-containing resist middle layer film, or a 4-layer resist process using a silicon-containing inorganic hard mask middle layer film and an organic antireflective coating.

### <Method for Forming Organic Film>

Using the above-described material for forming an organic film, it is possible to form an organic film which serves as an organic film in a multilayer resist film used in lithography or a planarizing film for manufacturing a semiconductor.

In the method for forming an organic film by using the inventive material for forming an organic film, a substrate to be processed (body to be processed) can be coated with the material for forming an organic film by a spin-coating method, etc. By employing a method like spin-coating method, favorable filling property can be obtained. After the spin-coating, baking (heating) is performed to evaporate the organic solvent and to promote the crosslinking reaction, thereby preventing the mixing with a resist upper layer film or a resist middle layer film. The baking is preferably performed at 100°C or higher to 600°C or lower for 10 to 600 seconds, more preferably at 200°C or higher to 500°C or lower for 10 to 300 seconds. In considering the influences of device damage and wafer deformation, the upper limit of the heating temperature in lithographic wafer process is preferably 600°C or lower, more preferably 500°C or lower.

Moreover, in the method for forming an organic film by using the inventive material for forming an organic film, after a substrate to be processed is coated with the inventive material for forming an organic film by the spin-coating method or the like as described above, an organic film can be formed by curing the material for forming an organic film by baking in an atmosphere with an oxygen concentration of 0.1% or more to 21% or less.

The inventive material for forming an organic film is baked in such an oxygen atmosphere, thereby enabling to obtain a fully cured organic film. The atmosphere during the baking may be in air. Nevertheless, it is preferable to introduce an inert gas such as N₂, Ar, or He to reduce oxygen amount from the viewpoint of preventing oxidation of the organic film. To prevent the oxidation, the oxygen concentration needs to be controlled, and is preferably 1,000 ppm or less, more preferably 100 ppm or less. Preventing the oxidation of the organic film during baking is preferable because an increase in absorption and a decrease in etching resistance are prevented.

Such a method for forming an organic film by using the inventive material for forming an organic film can achieve a flat cured film because of the excellent filling and planarizing properties regardless of unevenness of the substrate to be processed, and therefore, is extremely useful when forming a flat organic film on a substrate to be processed having a structure or step having a height of 30 nm or more.

Note that the thickness of the organic film or an organic film for a planarizing film for manufacturing a semiconductor device or the like is selected appropriately, but is preferably 30 to 20,000 nm, particularly preferably 50 to 15,000 nm.

### <Patterning Process>

The present invention provides a patterning process according to a 3-layer resist process using the material for forming an organic film described above. The patterning process is a method for forming a pattern in a substrate to be processed, and includes at least the following steps:
forming an organic film by using the above-described material for forming an organic film on a body to be processed;
forming a silicon-containing resist middle layer film by using a silicon-containing resist middle layer film material on the organic film;
forming a resist upper layer film by using a photoresist composition on the silicon-containing resist middle layer film;
forming a circuit pattern in the resist upper layer film;
transferring the pattern to the silicon-containing resist middle layer film by etching while using the resist upper layer film having the formed pattern as a mask;
transferring the pattern to the organic film by etching while using the silicon-containing resist middle layer film having the transferred pattern as a mask; and
further forming the pattern in the body to be processed by etching the body to be processed while using the organic film having the transferred pattern as a mask.

The silicon-containing resist middle layer film in the 3-layer resist process exhibits resistance to etching by an oxygen gas or a hydrogen gas. Thus, when the organic film is dry-etched while using the silicon-containing resist middle layer film as a mask in the 3-layer resist process, the dry etching is preferably performed using an etching gas mainly containing an oxygen gas or a hydrogen gas.

As the silicon-containing resist middle layer film in the 3-layer resist process, a polysiloxane-based middle layer film is also favorably used. The silicon-containing resist middle layer film having an antireflective effect can suppress the reflection. Particularly, for 193-nm light exposure, a material containing many aromatic groups and having high etching selectivity to a substrate is used as an organic film, so that the k-value and thus the substrate reflection are increased. However, the reflection can be suppressed by providing the silicon-containing resist middle layer film with absorption to achieve an appropriate k-value. Thus, the substrate reflection can be reduced to 0.5% or less. As the silicon-containing resist middle layer film having the antireflective effect, a polysiloxane is preferably used, the polysiloxane having anthracene for 248-nm and 157-nm light exposure, or a phenyl group or a light-absorbing group having a silicon-silicon bond for 193-nm light exposure in a pendant structure, and being crosslinked by an acid or heat.

In addition, a 4-layer resist process using an organic antireflective coating is also favorable, and in this case, the present invention provides a patterning process for forming a pattern in a body to be processed, including at least the following steps:
forming an organic film by using the above-described material for forming an organic film on a body to be processed;
forming a silicon-containing resist middle layer film by using a silicon-containing resist middle layer film material on the organic film;
forming an organic antireflective coating (BARC) on the silicon-containing resist middle layer film;
forming a resist upper layer film by using a photoresist composition on the BARC, so that a 4-layered film structure is constructed;
forming a circuit pattern in the resist upper layer film;
transferring the pattern to the BARC and the silicon-containing resist middle layer film by etching while using the resist upper layer film having the formed pattern as a mask;
transferring the pattern to the organic film by etching while using the silicon-containing resist middle layer film having the transferred pattern as a mask; and
further forming the pattern in the body to be processed by etching the body to be processed while using the organic film having the transferred pattern as a mask.

In addition, an inorganic hard mask middle layer film can be formed instead of the silicon-containing resist middle layer film, and in this case, the present invention provides a patterning process for forming a pattern in a body to be processed, including at least the following steps:
forming an organic film by using the above-described material for forming an organic film on a body to be processed;
forming an inorganic hard mask middle layer film selected from a silicon oxide film, a silicon nitride film, and a silicon oxynitride film on the organic film;
forming a resist upper layer film by using a photoresist composition on the inorganic hard mask middle layer film;
forming a circuit pattern in the resist upper layer film;
transferring the pattern to the inorganic hard mask middle layer film by etching while using the resist upper layer film having the formed pattern as a mask;
transferring the pattern to the organic film by etching while using the inorganic hard mask middle layer film having the transferred pattern as a mask; and
further forming the pattern in the body to be processed by etching the body to be processed while using the organic film having the transferred pattern as a mask.

In the case where an inorganic hard mask middle layer film is formed on the organic film as described above, a silicon oxide film, a silicon nitride film, and a silicon oxynitride film (SiON film) can be formed by a CVD method, an ALD method, or the like. The method for forming the silicon nitride film is disclosed in, for example, JP 2002-334869 A and WO 2004/066377 A1. The film thickness of the inorganic hard mask middle layer film is preferably 5 to 200 nm, more preferably 10 to 100 nm. As the inorganic hard mask middle layer film, a SiON film is most preferably used, being effective as an antireflective coating. When the SiON film is formed, the substrate temperature reaches 300 to 500°C. Hence, the organic film needs to withstand the temperature of 300 to 500°C. Since the materials for forming an organic film used in the present invention have high heat resistance and can withstand high temperatures of 300°C to 500°C, the combination of the inorganic hard mask middle layer film formed by a CVD method or an ALD method with the organic film formed by a spin-coating method is possible.

A 4-layer resist process using an organic antireflective coating is also favorable, and in this case, the present invention provides a patterning process for forming a pattern in a body to be processed, including at least the following steps:
forming an organic film by using the above-described material for forming an organic film on a body to be processed;
forming an inorganic hard mask middle layer film selected from a silicon oxide film, a silicon nitride film, and a silicon oxynitride film on the organic film;
forming an organic antireflective coating (BARC) on the inorganic hard mask middle layer film;
forming a resist upper layer film by using a photoresist composition on the BARC, so that a 4-layered film structure is constructed;
forming a circuit pattern in the resist upper layer film;
transferring the pattern to the BARC and the inorganic hard mask middle layer film by etching while using the resist upper layer film having the formed pattern as a mask;
transferring the pattern to the organic film by etching while using the inorganic hard mask middle layer film having the transferred pattern as a mask; and
further forming the pattern in the body to be processed by etching the body to be processed while using the organic film having the transferred pattern as a mask.

The photoresist film may be formed on the inorganic hard mask middle layer film as a resist upper layer film as described above, or alternatively, it is also possible to form an organic antireflective coating (BARC) on the inorganic hard mask middle layer film by spin-coating, and then form a photoresist film thereon. In particular, when a SiON film is used as the inorganic hard mask middle layer film, two antireflective coatings including the SiON film and the BARC make it possible to suppress the reflection even in liquid immersion exposure at a high NA exceeding 1.0. Another advantage of the BARC formation is having an effect of reducing trailing of the photoresist pattern immediately above the SiON film.

The resist upper layer film in the 3-layer resist process may be a positive type or a negative type, and any generally-used photoresist composition can be employed. After spin-coating of the photoresist composition, pre-baking is performed, preferably at 60 to 180°C for 10 to 300 seconds. Then, light exposure, post-exposure baking (PEB), and development are performed according to conventional methods to obtain the resist pattern. Note that the thickness of the resist upper layer film is not particularly limited, but is preferably 30 to 500 nm, and particularly preferably 50 to 400 nm.

In addition, examples of exposure light include a high-energy beam with a wavelength of 300 nm or less, specifically, excimer laser of 248 nm, 193 nm, and 157 nm, soft X-ray of 3 to 20 nm, electron beam, X-ray, and the like.

The pattern in the resist upper layer film is preferably formed by a lithography using light with a wavelength of 10 nm or more to 300 nm or less, a direct drawing with electron beam, nanoimprinting, or a combination thereof.

Furthermore, the development method in the patterning processes is preferably alkali development or development with an organic solvent.

Next, etching is performed while using the obtained resist upper layer film pattern as a mask. In the 3-layer resist process, the silicon-containing resist middle layer film and the inorganic hard mask middle layer film are etched using a fluorocarbon-based gas while using the resist upper layer film pattern as a mask. Thereby, a silicon-containing resist middle layer film pattern and an inorganic hard mask middle layer film pattern are formed.

Next, the organic film is etched while using the obtained silicon-containing resist middle layer film pattern and inorganic hard mask middle layer film pattern as masks.

Subsequently, the substrate to be processed (body to be processed) can be etched according to a conventional method. For example, the substrate to be processed made of SiO₂, SiN, or silica-based low-dielectric insulating film is etched mainly with a fluorocarbon-based gas; and p-Si, Al, or W is etched mainly with a chlorine- or bromine-based gas. When the substrate is processed by etching with a fluorocarbon-based gas, the silicon-containing resist middle layer film pattern in the 3-layer resist process is removed when the substrate is processed. When the substrate is etched with a chlorine- or bromine-based gas, the silicon-containing resist middle layer film pattern needs to be removed by additional dry etching with a fluorocarbon-based gas after the substrate processing.

An organic film obtained from the inventive material for forming an organic film has a characteristic of being excellent in etching resistance when etching these substrates to be processed.

The body to be processed is preferably a semiconductor device substrate or the semiconductor device substrate coated with any of a metal film, a metal carbide film, a metal oxide film, a metal nitride film, a metal oxycarbide film, and a metal oxynitride film. The body to be processed is not particularly limited, but more specifically, it is possible to use substrates made of Si, α-Si, p-Si, SiO₂, SiN, SiON, W, TiN, Al, or the like; the substrate coated with the metal film or the like as a layer to be processed; etc.

Examples of the layer to be processed include: various Low-k films made of Si, SiO₂, SiON, SiN, p-Si, α-Si, W, W-Si, Al, Cu, Al-Si, or the like; and stopper films thereof. Generally, the layer can be formed to have a thickness of 50 to 10,000 nm, in particular, 100 to 5,000 nm. Note that when the layer to be processed is formed, the substrate and the layer to be processed are formed from different materials.

Note that the metal is preferably silicon, titanium, tungsten, hafnium, zirconium, chromium, germanium, cobalt, copper, silver, gold, aluminum, indium, gallium, arsenic, palladium, iron, tantalum, iridium, manganese, molybdenum, ruthenium, or an alloy thereof.

Furthermore, as the substrate to be processed, a substrate to be processed having a structure or a step with a height of 30 nm or more is preferably used.

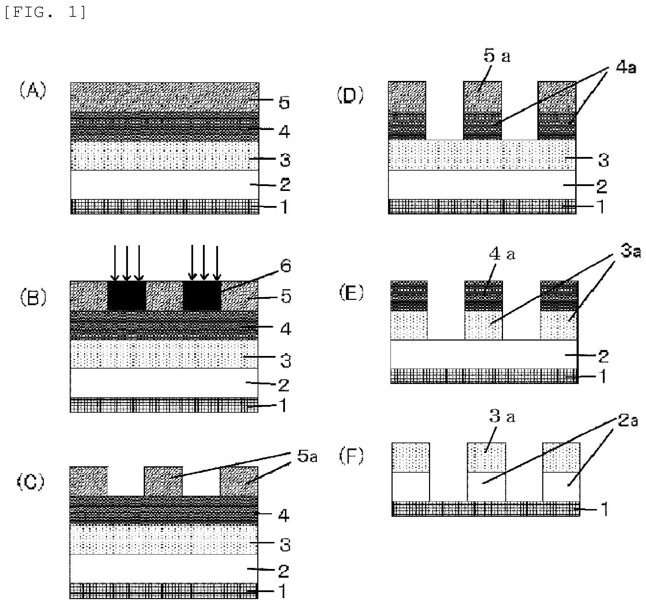

Hereinbelow, an example of the 3-layer resist process will be specifically described with reference to FIG. 1. As shown in FIG. 1 (A), in the 3-layer resist process, an organic film 3 is formed by using the inventive material for forming an organic film on a layer 2 to be processed that has been stacked on a substrate 1. Then, a silicon-containing resist middle layer film 4 is formed, and a resist upper layer film 5 is formed thereon.

Next, as shown in FIG. 1 (B), a predetermined portion 6 of the resist upper layer film 5 is exposed to light, followed by PEB and development to form a resist upper layer film pattern 5a as shown in FIG. 1 (C). While using the obtained resist upper layer film pattern 5a as a mask, the silicon-containing resist middle layer film 4 is etched with a CF-based gas. Thereby, a silicon-containing resist middle layer film pattern 4a is formed as shown in FIG. 1 (D). After the resist upper layer film pattern 5a is removed, the organic film 3 is etched with oxygen plasma while using the obtained silicon-containing resist middle layer film pattern 4a as a mask. Thereby, an organic film pattern 3a is formed as shown in FIG. 1 (E). Further, after the silicon-containing resist middle layer film pattern 4a is removed, the layer 2 to be processed is etched while using the organic film pattern 3a as a mask. Thus, a pattern 2a is formed as shown in FIG. 1 (F).

When an inorganic hard mask middle layer film is used, the silicon-containing resist middle layer film 4 is the inorganic hard mask middle layer film, and when a BARC is formed, the BARC is disposed between the silicon-containing resist middle layer film 4 and the resist upper layer film 5. The etching of the BARC may be performed continuously before the etching of the silicon-containing resist middle layer film 4. Alternatively, after the BARC is etched alone, the etching apparatus is changed, for example, and then the etching of the silicon-containing resist middle layer film 4 may be performed.

As described above, the inventive patterning processes make it possible to precisely form a fine pattern in a substrate to be processed in the multilayer resist processes.

### EXAMPLES

Hereinafter, the present invention will be more specifically described with reference to Synthesis Examples, Examples, and Comparative Examples. However, the present invention is not limited thereto. Note that, with respect to molecular weight and dispersity, weight-average molecular weight (Mw) and number-average molecular weight (Mn) were measured by gel permeation chromatography (GPC) using tetrahydrofuran as an eluent in terms of polystyrene, and dispersity (Mw/Mn) was calculated therefrom.

### Synthesis Examples: Synthesis of Organic Film Compounds (A1) to (A25) and Compounds (R1) to (R3) for Comparative Examples

For the synthesis of organic film compounds (A1) to (A25), compounds (B1) to (B12), having amino groups and/or hydroxy groups, and modifiers (C1) to (C8) shown below were used. Meanwhile, for the synthesis of compound (R3) for a Comparative Example, starting material compound (D1) for a Comparative Example was used.

Compounds having amino groups and/or hydroxy groups:

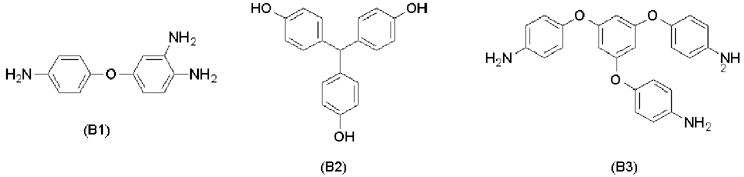

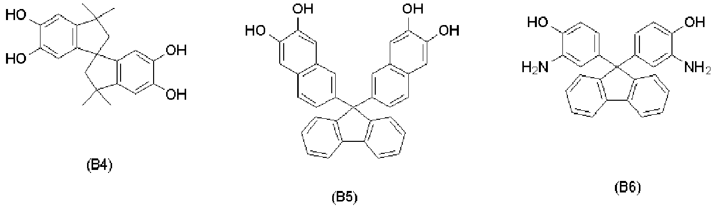

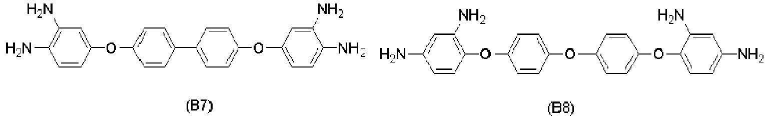

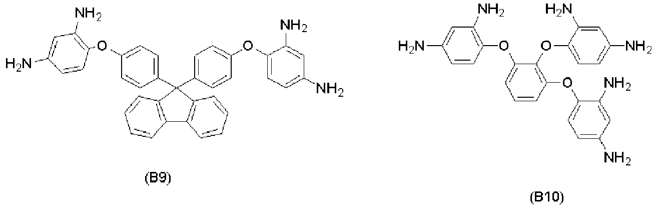

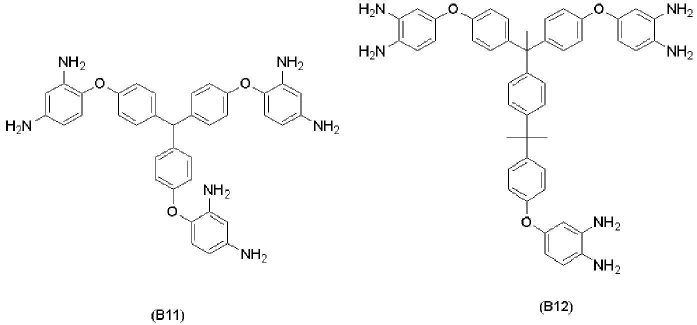

### Modifiers:

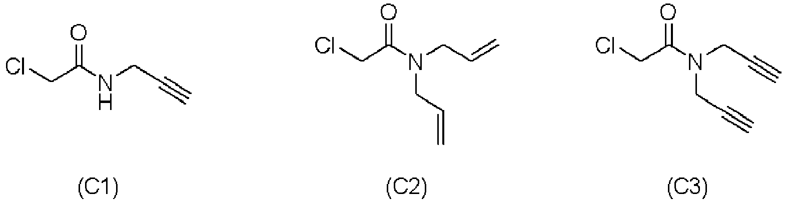

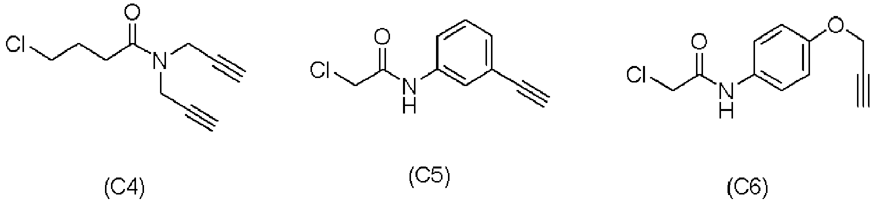

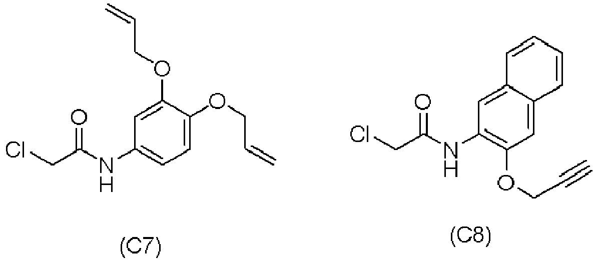

### Starting material compound for Comparative Example:

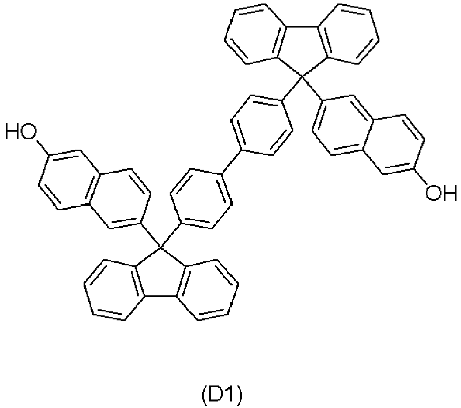

### (Synthesis Example 1)

### Synthesis of organic film compound (A1)

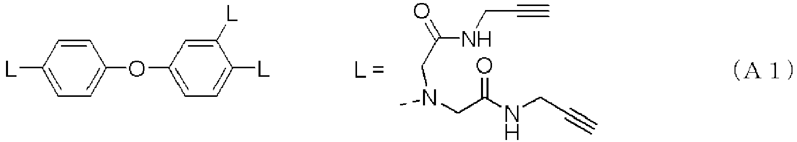

Under a nitrogen atmosphere, 10.0 g of the compound (B1), 38.5 g of potassium carbonate, and 140 g of DMF (dimethylformamide) were added together to form a homogeneous dispersion at an inner temperature of 50°C. A solution obtained by mixing and homogenizing 45.8 g of the modifier (C1) and 180 g of DMF beforehand was slowly added dropwise, and the reaction was allowed to proceed at an inner temperature of 50°C for 24 hours. After cooling to room temperature, 500 g of MIBK (methyl isobutyl ketone) and 500 g of pure water were added to form a homogeneous solution, and then the aqueous layer was removed. Furthermore, the organic layer was washed twice with 200 g of a 3.0% aqueous solution of nitric acid and five times with 200 g of pure water, and then the organic layer was evaporated under reduced pressure to dry up. To the residue, 110 g of THF was added to form a homogeneous solution. Thereafter, the solution was dropped into 700 g of hexane to precipitate a crystal. The precipitated crystal was separated by filtration, washed twice with 200 g of hexane, and collected. The collected crystal was vacuum dried at 70°C to obtain organic film compound (A1).

When the weight-average molecular weight (Mw) and dispersity (Mw/Mn) were measured by GPC, the following results were obtained.
(A1): Mw = 840, Mw/Mn = 1.02

### (Synthesis Example 2)

### Synthesis of organic film compound (A2)

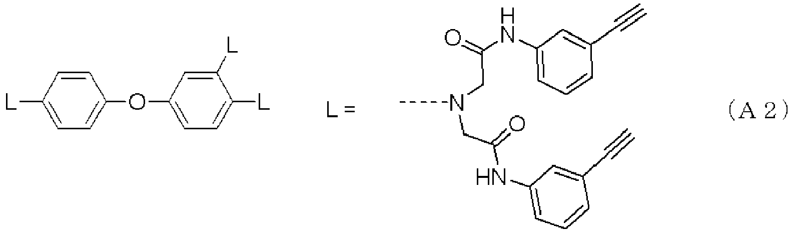

Under a nitrogen atmosphere, 10.0 g of the compound (B1), 38.5 g of potassium carbonate, and 140 g of DMF were added together to form a homogeneous dispersion at an inner temperature of 50°C. A solution obtained by mixing and homogenizing 67.5 g of the modifier (C5) and 270 g of DMF beforehand was slowly added dropwise, and the reaction was allowed to proceed at an inner temperature of 50°C for 24 hours. After cooling to room temperature, 500 g of MIBK and 500 g of pure water were added to form a homogeneous solution, and then the aqueous layer was removed. Furthermore, the organic layer was washed twice with 200 g of a 3.0% aqueous solution of nitric acid and five times with 200 g of pure water, and then the organic layer was evaporated under reduced pressure to dry up. To the residue, 170 g of THF was added to form a homogeneous solution. Thereafter, the solution was dropped into 1000 g of methanol to precipitate a crystal. The precipitated crystal was separated by filtration, washed twice with 300 g of methanol, and collected. The collected crystal was vacuum dried at 70°C to obtain organic film compound (A2).

When the weight-average molecular weight (Mw) and dispersity (Mw/Mn) were measured by GPC, the following results were obtained.
(A2): Mw = 1,200, Mw/Mn = 1.01

### (Synthesis Example 3)

### Synthesis of organic film compound (A3)

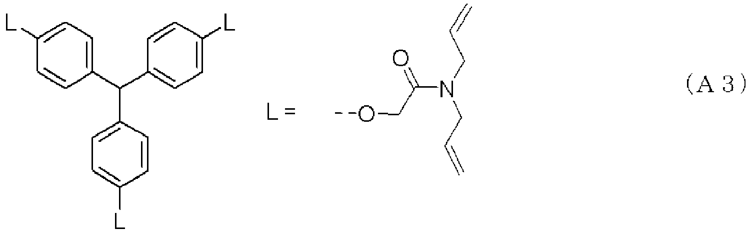

Under a nitrogen atmosphere, 10.0 g of the compound (B2), 14.2 g of potassium carbonate, and 75 g of DMF were added together to form a homogeneous dispersion at an inner temperature of 50°C. A solution obtained by mixing and homogenizing 22.3 g of the modifier (C2) and 70 g of DMF beforehand was slowly added dropwise, and the reaction was allowed to proceed at an inner temperature of 50°C for 24 hours. After cooling to room temperature, 300 g of MIBK and 300 g of pure water were added to form a homogeneous solution, and then the aqueous layer was removed. Furthermore, the organic layer was washed twice with 100 g of a 3.0% aqueous solution of nitric acid and five times with 100 g of pure water, and then the organic layer was evaporated under reduced pressure to dry up. To the residue, 70 g of THF was added to form a homogeneous solution. Thereafter, the solution was dropped into 400 g of hexane to precipitate a crystal. The precipitated crystal was separated by filtration, washed twice with 100 g of hexane, and collected. The collected crystal was vacuum dried at 70°C to obtain organic film compound (A3) .

When the weight-average molecular weight (Mw) and dispersity (Mw/Mn) were measured by GPC, the following results were obtained.
(A3): Mw = 710, Mw/Mn = 1.01

### (Synthesis Example 4)

### Synthesis of organic film compound (A4)

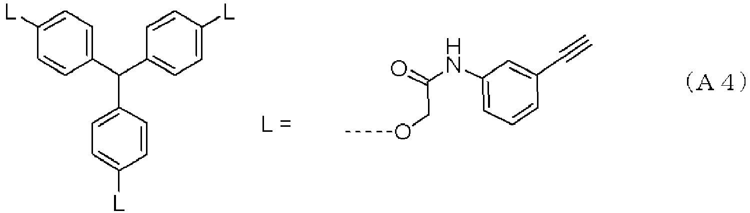

Under a nitrogen atmosphere, 10.0 g of the compound (B2), 14.2 g of potassium carbonate, and 75 g of DMF were added together to form a homogeneous dispersion at an inner temperature of 50°C. A solution obtained by mixing and homogenizing 24.8 g of the modifier (C5) and 75 g of DMF beforehand was slowly added dropwise, and the reaction was allowed to proceed at an inner temperature of 50°C for 24 hours. After cooling to room temperature, 300 g of MIBK and 300 g of pure water were added to form a homogeneous solution, and then the aqueous layer was removed. Furthermore, the organic layer was washed twice with 100 g of a 3.0% aqueous solution of nitric acid and five times with 100 g of pure water, and then the organic layer was evaporated under reduced pressure to dry up. To the residue, 80 g of THF was added to form a homogeneous solution. Thereafter, the solution was dropped into 500 g of methanol to precipitate a crystal. The precipitated crystal was separated by filtration, washed twice with 100 g of methanol, and collected. The collected crystal was vacuum dried at 70°C to obtain organic film compound (A4).

When the weight-average molecular weight (Mw) and dispersity (Mw/Mn) were measured by GPC, the following results were obtained.
(A4): Mw = 780, Mw/Mn = 1.02

### (Synthesis Example 5)

### Synthesis of organic film compound (A5)

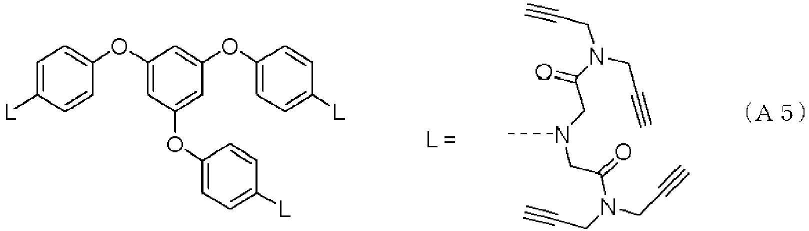

Under a nitrogen atmosphere, 10.0 g of the compound (B3), 20.8 g of potassium carbonate, and 120 g of DMF were added together to form a homogeneous dispersion at an inner temperature of 50°C. A solution obtained by mixing and homogenizing 31.9 g of the modifier (C3) and 100 g of DMF beforehand was slowly added dropwise, and the reaction was allowed to proceed at an inner temperature of 50°C for 24 hours. After cooling to room temperature, 300 g of MIBK and 300 g of pure water were added to form a homogeneous solution, and then the aqueous layer was removed. Furthermore, the organic layer was washed twice with 100 g of a 3.0% aqueous solution of nitric acid and five times with 100 g of pure water, and then the organic layer was evaporated under reduced pressure to dry up. To the residue, 90 g of THF was added to form a homogeneous solution. Thereafter, the solution was dropped into 550 g of hexane to precipitate a crystal. The precipitated crystal was separated by filtration, washed twice with 100 g of hexane, and collected. The collected crystal was vacuum dried at 70°C to obtain organic film compound (A5) .

When the weight-average molecular weight (Mw) and dispersity (Mw/Mn) were measured by GPC, the following results were obtained.
(A5): Mw = 1,260, Mw/Mn = 1.02

### (Synthesis Example 6)

### Synthesis of organic film compound (A6)

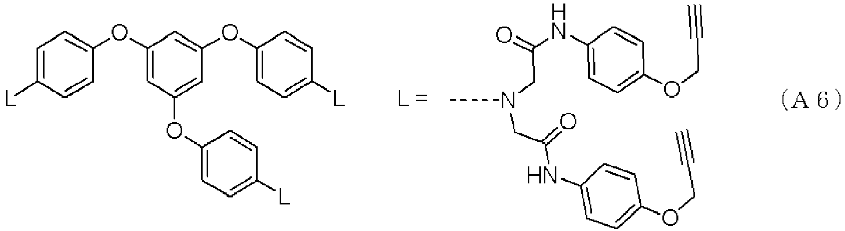

Under a nitrogen atmosphere, 10.0 g of the compound (B3), 20.8 g of potassium carbonate, and 120 g of DMF were added together to form a homogeneous dispersion at an inner temperature of 50°C. A solution obtained by mixing and homogenizing 42.0 g of the modifier (C6) and 130 g of DMF beforehand was slowly added dropwise, and the reaction was allowed to proceed at an inner temperature of 50°C for 24 hours. After cooling to room temperature, 400 g of MIBK and 400 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 120 g of THF was added to form a homogeneous solution. Thereafter, the solution was dropped into 600 g of methanol to precipitate a crystal. The precipitated crystal was separated by filtration, washed twice with 150 g of methanol, and collected. The collected crystal was vacuum dried at 70°C to obtain organic film compound (A6).

When the weight-average molecular weight (Mw) and dispersity (Mw/Mn) were measured by GPC, the following results were obtained.
(A6): Mw = 1,630, Mw/Mn = 1.01

### (Synthesis Example 7)

### Synthesis of organic film compound (A7)

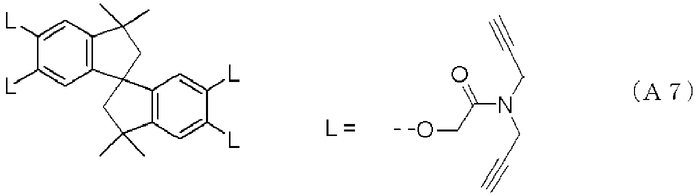

Under a nitrogen atmosphere, 10.0 g of the compound (B4), 16.2 g of potassium carbonate, and 80 g of DMF were added together to form a homogeneous dispersion at an inner temperature of 50°C. A solution obtained by mixing and homogenizing 24.9 g of the modifier (C3) and 75 g of DMF beforehand was slowly added dropwise, and the reaction was allowed to proceed at an inner temperature of 50°C for 24 hours. After cooling to room temperature, 200 g of MIBK and 200 g of pure water were added to form a homogeneous solution, and then the aqueous layer was removed. Furthermore, the organic layer was washed twice with 100 g of a 3.0% aqueous solution of nitric acid and five times with 100 g of pure water, and then the organic layer was evaporated under reduced pressure to dry up. To the residue, 75 g of THF was added to form a homogeneous solution. Thereafter, the solution was dropped into 400 g of hexane to precipitate a crystal. The precipitated crystal was separated by filtration, washed twice with 100 g of hexane, and collected. The collected crystal was vacuum dried at 70°C to obtain organic film compound (A7) .

When the weight-average molecular weight (Mw) and dispersity (Mw/Mn) were measured by GPC, the following results were obtained.
(A7): Mw = 890, Mw/Mn = 1.01

### (Synthesis Example 8)

### Synthesis of organic film compound (A8)

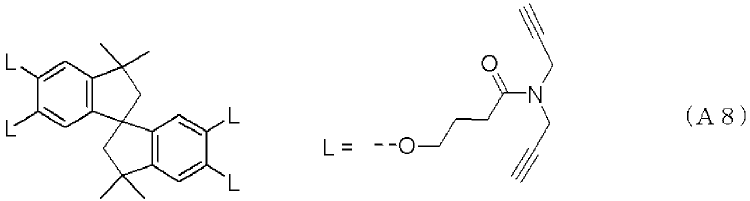

Under a nitrogen atmosphere, 10.0 g of the compound (B4), 16.2 g of potassium carbonate, and 80 g of DMF were added together to form a homogeneous dispersion at an inner temperature of 50°C. A solution obtained by mixing and homogenizing 29.0 g of the modifier (C4) and 90 g of DMF beforehand was slowly added dropwise, and the reaction was allowed to proceed at an inner temperature of 50°C for 24 hours. After cooling to room temperature, 200 g of MIBK and 200 g of pure water were added to form a homogeneous solution, and then the aqueous layer was removed. Furthermore, the organic layer was washed twice with 100 g of a 3.0% aqueous solution of nitric acid and five times with 100 g of pure water, and then the organic layer was evaporated under reduced pressure to dry up. To the residue, 90 g of THF was added to form a homogeneous solution. Thereafter, the solution was dropped into 450 g of hexane to precipitate a crystal. The precipitated crystal was separated by filtration, washed twice with 100 g of hexane, and collected. The collected crystal was vacuum dried at 70°C to obtain organic film compound (A8) .

When the weight-average molecular weight (Mw) and dispersity (Mw/Mn) were measured by GPC, the following results were obtained.
(A8): Mw = 980, Mw/Mn = 1.02

### (Synthesis Example 9)

### Synthesis of organic film compound (A9)

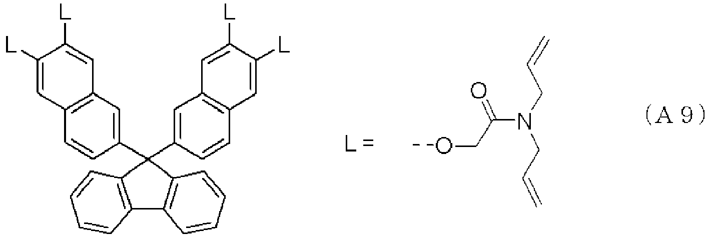

Under a nitrogen atmosphere, 10.0 g of the compound (B5), 11.5 g of potassium carbonate, and 60 g of DMF were added together to form a homogeneous dispersion at an inner temperature of 50°C. A solution obtained by mixing and homogenizing 18.0 g of the modifier (C2) and 75 g of DMF beforehand was slowly added dropwise, and the reaction was allowed to proceed at an inner temperature of 50°C for 24 hours. After cooling to room temperature, 200 g of MIBK and 200 g of pure water were added to form a homogeneous solution, and then the aqueous layer was removed. Furthermore, the organic layer was washed twice with 100 g of a 3.0% aqueous solution of nitric acid and five times with 100 g of pure water, and then the organic layer was evaporated under reduced pressure to dry up. To the residue, 65 g of THF was added to form a homogeneous solution. Thereafter, the solution was dropped into 400 g of hexane to precipitate a crystal. The precipitated crystal was separated by filtration, washed twice with 100 g of hexane, and collected. The collected crystal was vacuum dried at 70°C to obtain organic film compound (A9) .

When the weight-average molecular weight (Mw) and dispersity (Mw/Mn) were measured by GPC, the following results were obtained.
(A9): Mw = 1,080, Mw/Mn = 1.02

### (Synthesis Example 10)

### Synthesis of organic film compound (A10)

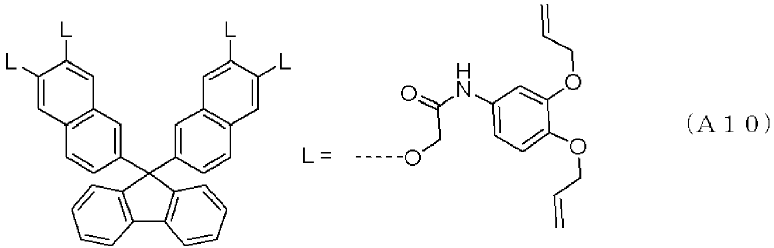

Under a nitrogen atmosphere, 10.0 g of the compound (B5), 11.5 g of potassium carbonate, and 60 g of DMF were added together to form a homogeneous dispersion at an inner temperature of 50°C. A solution obtained by mixing and homogenizing 29.2 g of the modifier (C7) and 90 g of DMF beforehand was slowly added dropwise, and the reaction was allowed to proceed at an inner temperature of 50°C for 24 hours. After cooling to room temperature, 300 g of MIBK and 200 g of pure water were added to form a homogeneous solution, and then the aqueous layer was removed. Furthermore, the organic layer was washed twice with 100 g of a 3.0% aqueous solution of nitric acid and five times with 100 g of pure water, and then the organic layer was evaporated under reduced pressure to dry up. To the residue, 90 g of THF was added to form a homogeneous solution. Thereafter, the solution was dropped into 400 g of methanol to precipitate a crystal. The precipitated crystal was separated by filtration, washed twice with 100 g of methanol, and collected. The collected crystal was vacuum dried at 70°C to obtain organic film compound (A10).

When the weight-average molecular weight (Mw) and dispersity (Mw/Mn) were measured by GPC, the following results were obtained.
(A10): Mw = 1,540, Mw/Mn = 1.03

### (Synthesis Example 11)

### Synthesis of organic film compound (A11)

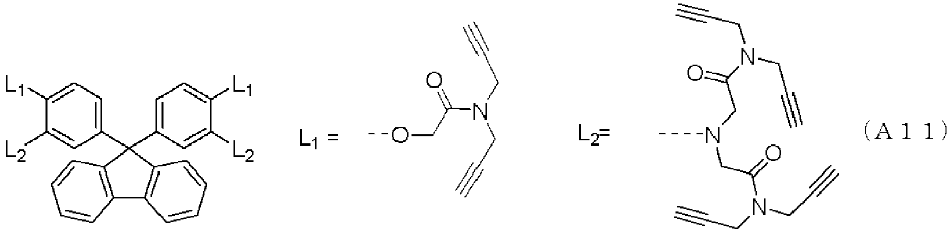

Under a nitrogen atmosphere, 10.0 g of the compound (B6), 21.8 g of potassium carbonate, and 100 g of DMF were added together to form a homogeneous dispersion at an inner temperature of 50°C. A solution obtained by mixing and homogenizing 33.4 g of the modifier (C3) and 130 g of DMF beforehand was slowly added dropwise, and the reaction was allowed to proceed at an inner temperature of 50°C for 24 hours. After cooling to room temperature, 400 g of MIBK and 300 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 100 g of THF was added to form a homogeneous solution. Thereafter, the solution was dropped into 400 g of hexane to precipitate a crystal. The precipitated crystal was separated by filtration, washed twice with 100 g of hexane, and collected. The collected crystal was vacuum dried at 70°C to obtain organic film compound (A11).

When the weight-average molecular weight (Mw) and dispersity (Mw/Mn) were measured by GPC, the following results were obtained.
(A11): Mw = 1,220, Mw/Mn = 1.03

### (Synthesis Example 12)

### Synthesis of organic film compound (A12)

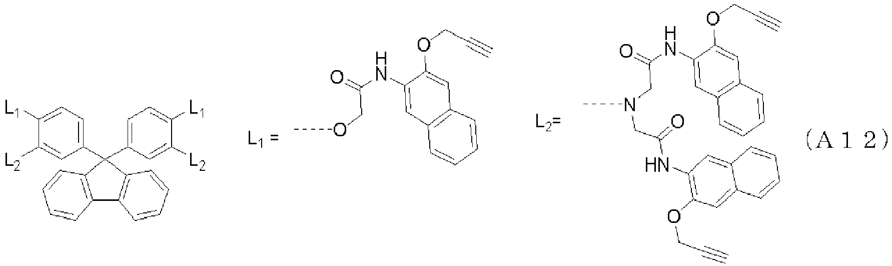

Under a nitrogen atmosphere, 10.0 g of the compound (B6), 21.8 g of potassium carbonate, and 100 g of DMF were added together to form a homogeneous dispersion at an inner temperature of 50°C. A solution obtained by mixing and homogenizing 54.0 g of the modifier (C8) and 160 g of DMF beforehand was slowly added dropwise, and the reaction was allowed to proceed at an inner temperature of 50°C for 24 hours. After cooling to room temperature, 400 g of MIBK and 300 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 140 g of THF was added to form a homogeneous solution. Thereafter, the solution was dropped into 500 g of methanol to precipitate a crystal. The precipitated crystal was separated by filtration, washed twice with 200 g of methanol, and collected. The collected crystal was vacuum dried at 70°C to obtain organic film compound (A12).

When the weight-average molecular weight (Mw) and dispersity (Mw/Mn) were measured by GPC, the following results were obtained.
(A12): Mw = 1,920, Mw/Mn = 1.03

### (Synthesis Example 13)

### Synthesis of organic film compound (A13)

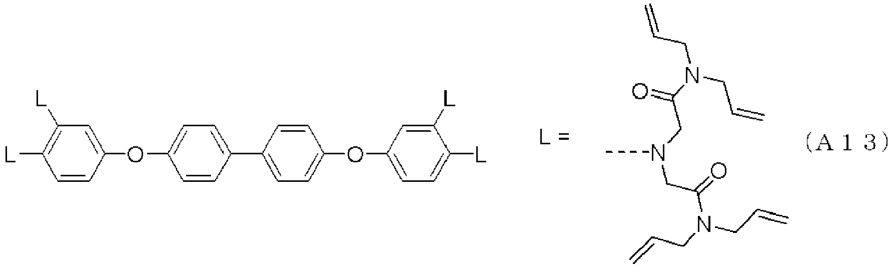

Under a nitrogen atmosphere, 10.0 g of the compound (B7), 27.8 g of potassium carbonate, and 120 g of DMF were added together to form a homogeneous dispersion at an inner temperature of 50°C. A solution obtained by mixing and homogenizing 43.6 g of the modifier (C2) and 130 g of DMF beforehand was slowly added dropwise, and the reaction was allowed to proceed at an inner temperature of 50°C for 24 hours. After cooling to room temperature, 400 g of MIBK and 400 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 110 g of THF was added to form a homogeneous solution. Thereafter, the solution was dropped into 500 g of hexane to precipitate a crystal. The precipitated crystal was separated by filtration, washed twice with 150 g of hexane, and collected. The collected crystal was vacuum dried at 70°C to obtain organic film compound (A13).

When the weight-average molecular weight (Mw) and dispersity (Mw/Mn) were measured by GPC, the following results were obtained.
(A13): Mw = 1,550, Mw/Mn = 1.01

### (Synthesis Example 14)

### Synthesis of organic film compound (A14)

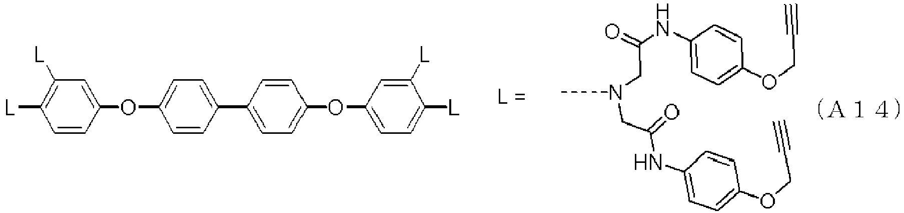

Under a nitrogen atmosphere, 10.0 g of the compound (B7), 27.8 g of potassium carbonate, and 120 g of DMF were added together to form a homogeneous dispersion at an inner temperature of 50°C. A solution obtained by mixing and homogenizing 56.1 g of the modifier (C6) and 170 g of DMF beforehand was slowly added dropwise, and the reaction was allowed to proceed at an inner temperature of 50°C for 24 hours. After cooling to room temperature, 500 g of MIBK and 400 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 140 g of THF was added to form a homogeneous solution. Thereafter, the solution was dropped into 600 g of methanol to precipitate a crystal. The precipitated crystal was separated by filtration, washed twice with 150 g of methanol, and collected. The collected crystal was vacuum dried at 70°C to obtain organic film compound (A14).

When the weight-average molecular weight (Mw) and dispersity (Mw/Mn) were measured by GPC, the following results were obtained.
(A14): Mw = 1,900, Mw/Mn = 1.02

### (Synthesis Example 15)

### Synthesis of organic film compound (A15)

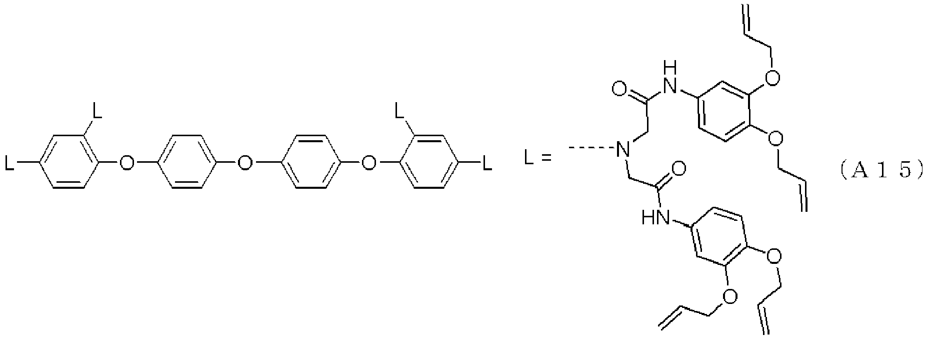

Under a nitrogen atmosphere, 10.0 g of the compound (B8), 26.7 g of potassium carbonate, and 130 g of DMF were added together to form a homogeneous dispersion at an inner temperature of 50°C. A solution obtained by mixing and homogenizing 68.0 g of the modifier (C7) and 200 g of DMF beforehand was slowly added dropwise, and the reaction was allowed to proceed at an inner temperature of 50°C for 24 hours. After cooling to room temperature, 500 g of MIBK and 400 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 170 g of THF was added to form a homogeneous solution. Thereafter, the solution was dropped into 1000 g of methanol to precipitate a crystal. The precipitated crystal was separated by filtration, washed twice with 300 g of methanol, and collected. The collected crystal was vacuum dried at 70°C to obtain organic film compound (A15).

When the weight-average molecular weight (Mw) and dispersity (Mw/Mn) were measured by GPC, the following results were obtained.
(A15): Mw = 2,450, Mw/Mn = 1.02

### (Synthesis Example 16)

### Synthesis of organic film compound (A16)

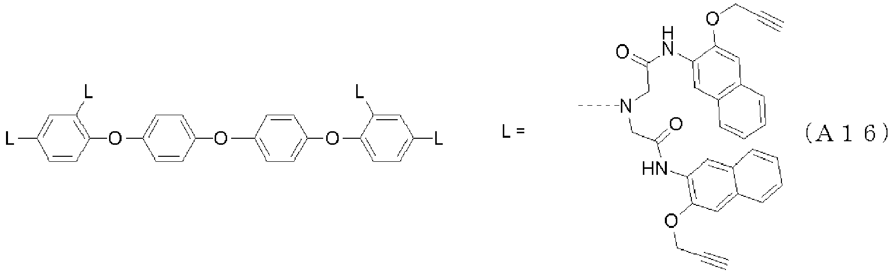

Under a nitrogen atmosphere, 10.0 g of the compound (B8), 26.7 g of potassium carbonate, and 130 g of DMF were added together to form a homogeneous dispersion at an inner temperature of 50°C. A solution obtained by mixing and homogenizing 66.0 g of the modifier (C8) and 200 g of DMF beforehand was slowly added dropwise, and the reaction was allowed to proceed at an inner temperature of 50°C for 24 hours. After cooling to room temperature, 500 g of MIBK and 400 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 170 g of THF was added to form a homogeneous solution. Thereafter, the solution was dropped into 1000 g of methanol to precipitate a crystal. The precipitated crystal was separated by filtration, washed twice with 300 g of methanol, and collected. The collected crystal was vacuum dried at 70°C to obtain organic film compound (A16).

When the weight-average molecular weight (Mw) and dispersity (Mw/Mn) were measured by GPC, the following results were obtained.
(A16): Mw = 2,380, Mw/Mn = 1.03

### (Synthesis Example 17)

### Synthesis of organic film compound (A17)

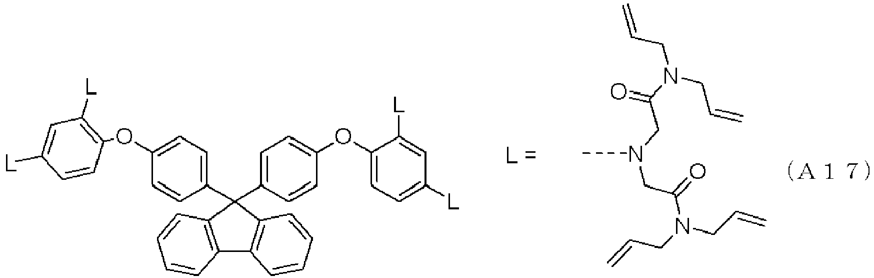

Under a nitrogen atmosphere, 10.0 g of the compound (B9), 19.7 g of potassium carbonate, and 90 g of DMF were added together to form a homogeneous dispersion at an inner temperature of 50°C. A solution obtained by mixing and homogenizing 30.9 g of the modifier (C2) and 90 g of DMF beforehand was slowly added dropwise, and the reaction was allowed to proceed at an inner temperature of 50°C for 24 hours. After cooling to room temperature, 200 g of MIBK and 200 g of pure water were added to form a homogeneous solution, and then the aqueous layer was removed. Furthermore, the organic layer was washed twice with 100 g of a 3.0% aqueous solution of nitric acid and five times with 100 g of pure water, and then the organic layer was evaporated under reduced pressure to dry up. To the residue, 90 g of THF was added to form a homogeneous solution. Thereafter, the solution was dropped into 400 g of hexane to precipitate a crystal. The precipitated crystal was separated by filtration, washed twice with 100 g of hexane, and collected. The collected crystal was vacuum dried at 70°C to obtain organic film compound (A17).

When the weight-average molecular weight (Mw) and dispersity (Mw/Mn) were measured by GPC, the following results were obtained.
(A17): Mw = 1,730, Mw/Mn = 1.01

### (Synthesis Example 18)

### Synthesis of organic film compound (A18)

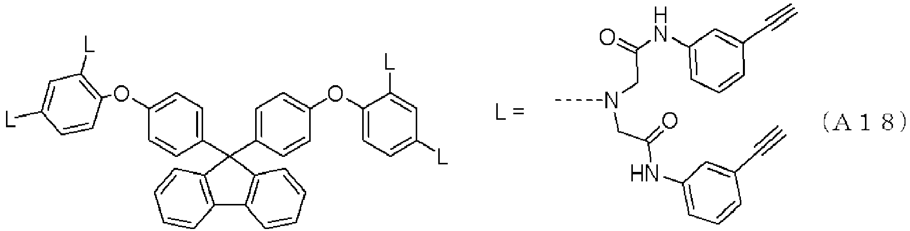

Under a nitrogen atmosphere, 10.0 g of the compound (B9), 19.7 g of potassium carbonate, and 90 g of DMF were added together to form a homogeneous dispersion at an inner temperature of 50°C. A solution obtained by mixing and homogenizing 34.4 g of the modifier (C5) and 100 g of DMF beforehand was slowly added dropwise, and the reaction was allowed to proceed at an inner temperature of 50°C for 24 hours. After cooling to room temperature, 200 g of MIBK and 200 g of pure water were added to form a homogeneous solution, and then the aqueous layer was removed. Furthermore, the organic layer was washed twice with 100 g of a 3.0% aqueous solution of nitric acid and five times with 100 g of pure water, and then the organic layer was evaporated under reduced pressure to dry up. To the residue, 100 g of THF was added to form a homogeneous solution. Thereafter, the solution was dropped into 400 g of methanol to precipitate a crystal. The precipitated crystal was separated by filtration, washed twice with 100 g of methanol, and collected. The collected crystal was vacuum dried at 70°C to obtain organic film compound (A18).

When the weight-average molecular weight (Mw) and dispersity (Mw/Mn) were measured by GPC, the following results were obtained.
(A18): Mw = 1,880, Mw/Mn = 1.02

### (Synthesis Example 19)

### Synthesis of organic film compound (A19)

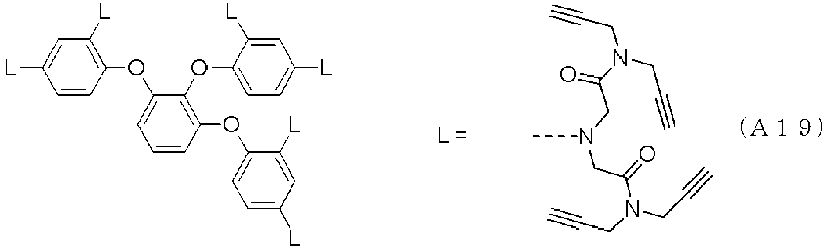

Under a nitrogen atmosphere, 10.0 g of the compound (B10), 37.3 g of potassium carbonate, and 150 g of DMF were added together to form a homogeneous dispersion at an inner temperature of 50°C. A solution obtained by mixing and homogenizing 57.2 g of the modifier (C3) and 170 g of DMF beforehand was slowly added dropwise, and the reaction was allowed to proceed at an inner temperature of 50°C for 24 hours. After cooling to room temperature, 400 g of MIBK and 400 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 140 g of THF was added to form a homogeneous solution. Thereafter, the solution was dropped into 600 g of hexane to precipitate a crystal. The precipitated crystal was separated by filtration, washed twice with 200 g of hexane, and collected. The collected crystal was vacuum dried at 70°C to obtain organic film compound (A19) .

When the weight-average molecular weight (Mw) and dispersity (Mw/Mn) were measured by GPC, the following results were obtained.
(A19): Mw = 2,050, Mw/Mn = 1.03

### (Synthesis Example 20)

### Synthesis of organic film compound (A20)

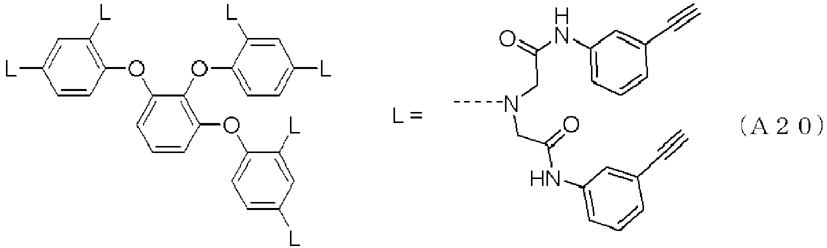

Under a nitrogen atmosphere, 10.0 g of the compound (B10), 37.3 g of potassium carbonate, and 150 g of DMF were added together to form a homogeneous dispersion at an inner temperature of 50°C. A solution obtained by mixing and homogenizing 65.3 g of the modifier (C5) and 200 g of DMF beforehand was slowly added dropwise, and the reaction was allowed to proceed at an inner temperature of 50°C for 24 hours. After cooling to room temperature, 500 g of MIBK (methyl isobutyl ketone) and 500 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 160 g of THF was added to form a homogeneous solution. Thereafter, the solution was dropped into 1,000 g of methanol to precipitate a crystal. The precipitated crystal was separated by filtration, washed twice with 300 g of methanol, and collected. The collected crystal was vacuum dried at 70°C to obtain organic film compound (A20).

When the weight-average molecular weight (Mw) and dispersity (Mw/Mn) were measured by GPC, the following results were obtained.
(A20): Mw = 2,460, Mw/Mn = 1.02

### (Synthesis Example 21)

### Synthesis of organic film compound (A21)

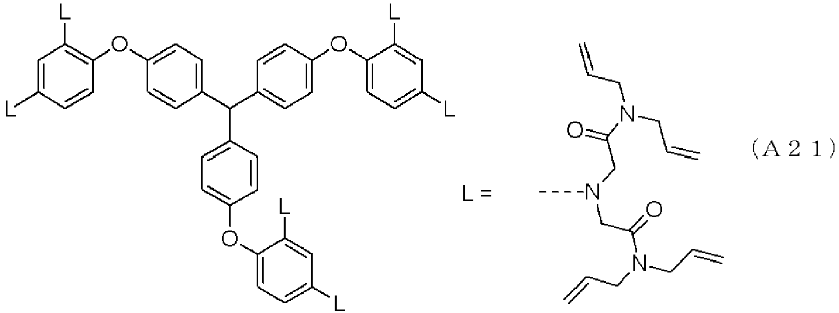

Under a nitrogen atmosphere, 10.0 g of the compound (B11), 27.2 g of potassium carbonate, and 110 g of DMF were added together to form a homogeneous dispersion at an inner temperature of 50°C. A solution obtained by mixing and homogenizing 42.7 g of the modifier (C2) and 130 g of DMF beforehand was slowly added dropwise, and the reaction was allowed to proceed at an inner temperature of 50°C for 24 hours. After cooling to room temperature, 400 g of MIBK and 400 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 110 g of THF was added to form a homogeneous solution. Thereafter, the solution was dropped into 500 g of hexane to precipitate a crystal. The precipitated crystal was separated by filtration, washed twice with 200 g of hexane, and collected. The collected crystal was vacuum dried at 70°C to obtain organic film compound (A21).

When the weight-average molecular weight (Mw) and dispersity (Mw/Mn) were measured by GPC, the following results were obtained.
(A21): Mw = 2,310, Mw/Mn = 1.03

### (Synthesis Example 22)

### Synthesis of organic film compound (A22)

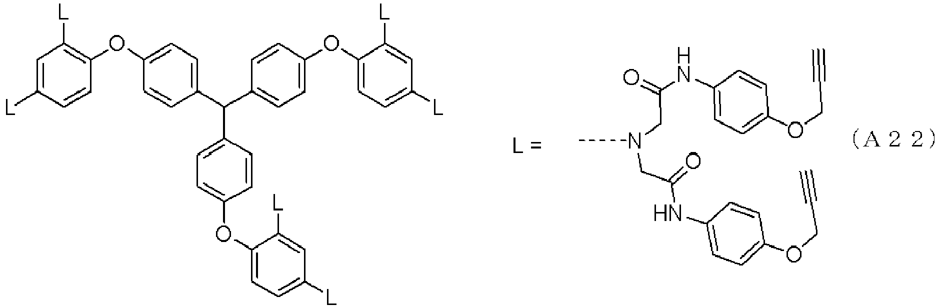

Under a nitrogen atmosphere, 10.0 g of the compound (B11), 27.2 g of potassium carbonate, and 110 g of DMF were added together to form a homogeneous dispersion at an inner temperature of 50°C. A solution obtained by mixing and homogenizing 54.9 g of the modifier (C6) and 160 g of DMF beforehand was slowly added dropwise, and the reaction was allowed to proceed at an inner temperature of 50°C for 24 hours. After cooling to room temperature, 500 g of MIBK and 500 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 140 g of THF was added to form a homogeneous solution. Thereafter, the solution was dropped into 600 g of methanol to precipitate a crystal. The precipitated crystal was separated by filtration, washed twice with 200 g of methanol, and collected. The collected crystal was vacuum dried at 70°C to obtain organic film compound (A22).

When the weight-average molecular weight (Mw) and dispersity (Mw/Mn) were measured by GPC, the following results were obtained.
(A22): Mw = 2,940, Mw/Mn = 1.02

### (Synthesis Example 23)

### Synthesis of organic film compound (A23)

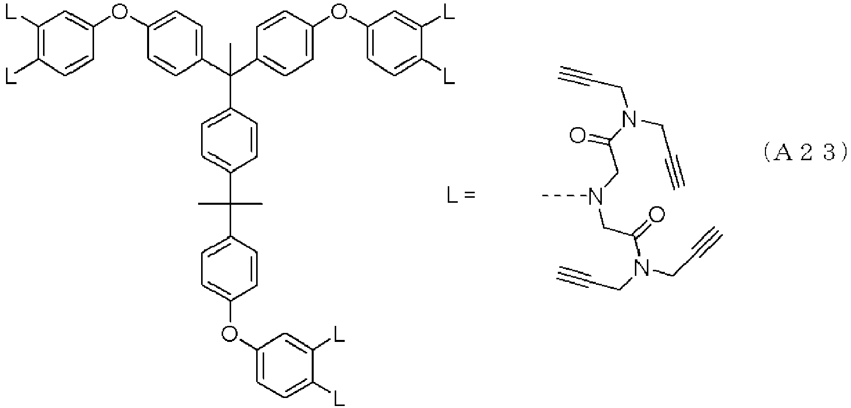

Under a nitrogen atmosphere, 10.0 g of the compound (B12), 22.3 g of potassium carbonate, and 100 g of DMF were added together to form a homogeneous dispersion at an inner temperature of 50°C. A solution obtained by mixing and homogenizing 34.3 g of the modifier (C3) and 110 g of DMF beforehand was slowly added dropwise, and the reaction was allowed to proceed at an inner temperature of 50°C for 24 hours. After cooling to room temperature, 400 g of MIBK and 300 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 100 g of THF was added to form a homogeneous solution. Thereafter, the solution was dropped into 500 g of hexane to precipitate a crystal. The precipitated crystal was separated by filtration, washed twice with 200 g of hexane, and collected. The collected crystal was vacuum dried at 70°C to obtain organic film compound (A23).

When the weight-average molecular weight (Mw) and dispersity (Mw/Mn) were measured by GPC, the following results were obtained.
(A23): Mw = 2,360, Mw/Mn = 1.03

### (Synthesis Example 24)

### Synthesis of organic film compound (A24)

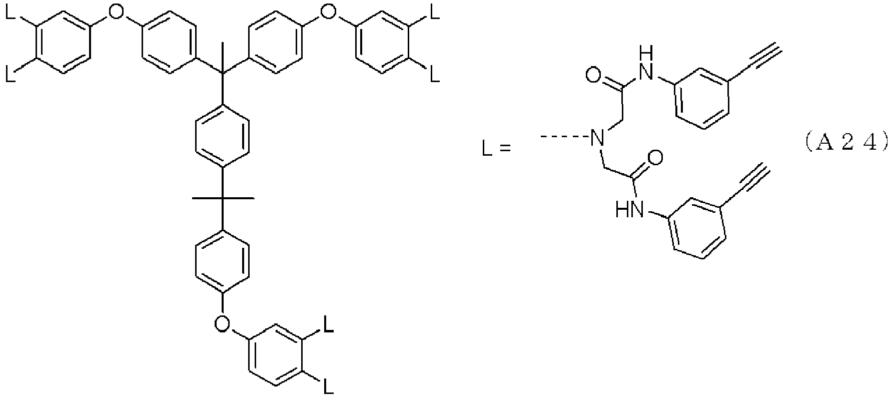

Under a nitrogen atmosphere, 10.0 g of the compound (B12), 22.3 g of potassium carbonate, and 100 g of DMF were added together to form a homogeneous dispersion at an inner temperature of 50°C. A solution obtained by mixing and homogenizing 39.1 g of the modifier (C5) and 120 g of DMF beforehand was slowly added dropwise, and the reaction was allowed to proceed at an inner temperature of 50°C for 24 hours. After cooling to room temperature, 400 g of MIBK and 300 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 110 g of THF was added to form a homogeneous solution. Thereafter, the solution was dropped into 500 g of methanol to precipitate a crystal. The precipitated crystal was separated by filtration, washed twice with 200 g of methanol, and collected. The collected crystal was vacuum dried at 70°C to obtain organic film compound (A24).

When the weight-average molecular weight (Mw) and dispersity (Mw/Mn) were measured by GPC, the following results were obtained.
(A24): Mw = 2,840, Mw/Mn = 1.04

### (Synthesis Example 25)

### Synthesis of organic film compound (A25)

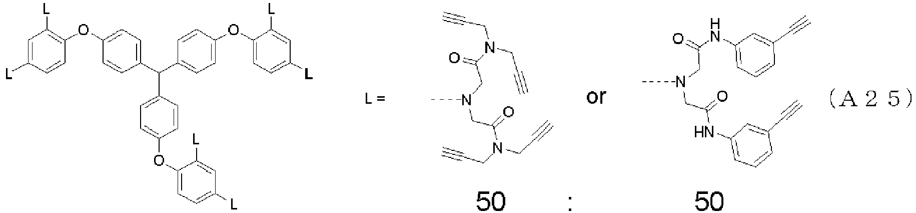

Under a nitrogen atmosphere, 10.0 g of the compound (B11), 27.2 g of potassium carbonate, and 110 g of DMF were added together to form a homogeneous dispersion at an inner temperature of 50°C. A solution obtained by mixing and homogenizing 16.7 g of the modifier (C3) and 50 g of DMF beforehand was slowly added dropwise, and the reaction was allowed to proceed at an inner temperature of 50°C for 8 hours. After that, a solution obtained by mixing and homogenizing 23.8 g of the modifier (C5) and 75 g of DMF beforehand was slowly added dropwise, and the reaction was allowed to proceed for a further 16 hours. After cooling to room temperature, 500 g of MIBK (methyl isobutyl ketone) and 500 g of pure water were added to form a homogeneous solution, and then the aqueous layer was removed. Furthermore, the organic layer was washed twice with 150 g of a 3.0% aqueous solution of nitric acid and five times with 150 g of pure water, and then the organic layer was evaporated under reduced pressure to dry up. To the residue, 100 g of THF was added to form a homogeneous solution. Thereafter, the solution was dropped into 500 g of hexane to precipitate a crystal. The precipitated crystal was separated by filtration, washed twice with 200 g of hexane, and collected. The collected crystal was vacuum dried at 70°C to obtain organic film compound (A25).

When the weight-average molecular weight (Mw) and dispersity (Mw/Mn) were measured by GPC, the following results were obtained.
(A25): Mw = 1,830, Mw/Mn = 1.09

### (Comparative Synthesis Example 1)

### Synthesis of compound (R1) for Comparative Example

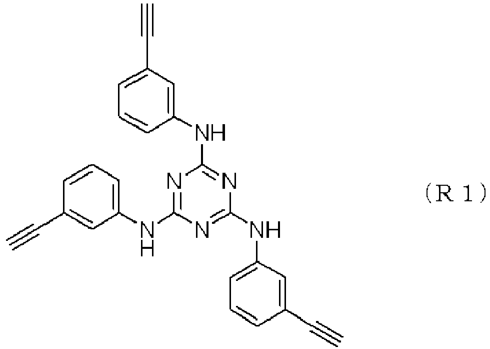

Under a nitrogen atmosphere, 13.7 g of m-ethynylaniline, 13.3 g of triethyl amine, and 150 g of THF were stirred at room temperature to form a homogeneous solution. A solution obtained by mixing 10 g of THF and 10.0 g of cyanuric chloride beforehand to form a homogeneous solution was slowly added dropwise, and then the reaction was allowed to proceed under reflux for 5 hours. After cooling to room temperature, 150 g of methyl isobutyl ketone and 100 g of pure water were added to form a homogeneous solution, and then the aqueous layer was removed. Furthermore, the organic layer was washed twice with 50 g of a 3.0% aqueous solution of nitric acid and five times with 50 g of pure water, and then the organic layer was evaporated under reduced pressure to dry up. To the residue, 60 g of THF was added to form a homogeneous solution. Thereafter, a crystal was precipitated with 200 g of hexane. The precipitated crystal was separated by filtration, washed twice with 100 g of hexane, and collected. The collected crystal was vacuum dried at 70°C to obtain compound (R1) for a Comparative Example.

When the weight-average molecular weight (Mw) and dispersity (Mw/Mn) were measured by GPC, the following results were obtained.
(R1): Mw = 430, Mw/Mn = 1.01

### (Comparative Synthesis Example 2)

### Synthesis of compound (R2) for Comparative Example

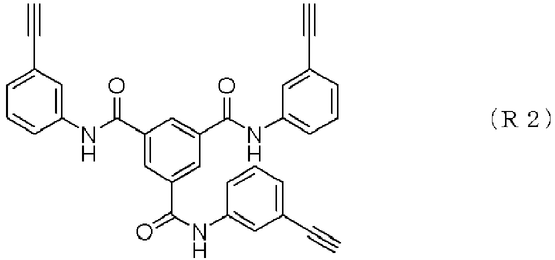

Under a nitrogen atmosphere, 13.7 g of m-ethynylaniline, 13.3 g of triethyl amine, and 150 g of THF were stirred in an ice bath to form a homogeneous solution. A solution obtained by mixing 10 g of THF and 10.0 g of trimesic acid chloride beforehand to form a homogeneous solution was slowly added dropwise, and the reaction was allowed to proceed in an ice bath for 1 hour and at room temperature for 3 hours. After cooling to room temperature, 150 g of methyl isobutyl ketone and 100 g of pure water were added to form a homogeneous solution, and then the aqueous layer was removed. Furthermore, the organic layer was washed twice with 50 g of a 3.0% aqueous solution of nitric acid and five times with 50 g of pure water, and then the organic layer was evaporated under reduced pressure to dry up. To the residue, 60 g of THF was added to form a homogeneous solution. Thereafter, a crystal was precipitated with 200 g of methanol. The precipitated crystal was separated by filtration, washed twice with 100 g of methanol, and collected. The collected crystal was vacuum dried at 70°C to obtain compound (R2) for a Comparative Example.

When the weight-average molecular weight (Mw) and dispersity (Mw/Mn) were measured by GPC, the following results were obtained.
(R2): Mw = 520, Mw/Mn = 1.01

### (Comparative Synthesis Example 3)

### Synthesis of compound (R3) for Comparative Example

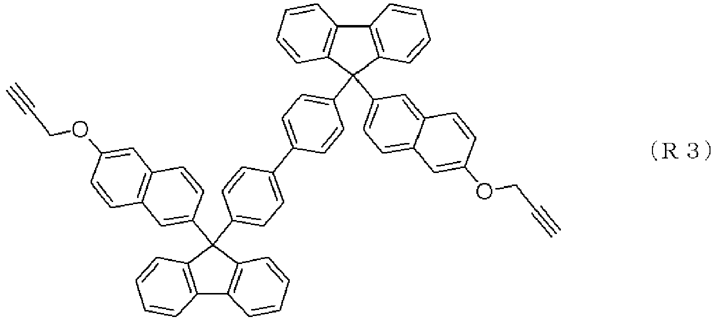

Under a nitrogen atmosphere, a homogeneous dispersion of 10.00 g of the starting material compound (D1) for a Comparative Example, 4.76 g of potassium carbonate, and 50 g of DMF was formed at an inner temperature of 50°C. 3.72 g of propargyl bromide was slowly added dropwise, and the reaction was allowed to proceed at an inner temperature of 50°C for 16 hours. After cooling to room temperature, 100 g of methyl isobutyl ketone and 50 g of pure water were added to form a homogeneous solution, and then the aqueous layer was removed. Furthermore, the organic layer was washed twice with 30 g of a 3.0% aqueous solution of nitric acid and five times with 30 g of pure water, and then the organic layer was evaporated under reduced pressure to dry up. To the residue, 30 g of THF was added, and a crystal was precipitated with 100 g of methanol. The precipitated crystal was separated by filtration, washed twice with 60 g of methanol, and collected. The collected crystal was vacuum dried at 70°C to obtain compound (R3) for a Comparative Example.

When the weight-average molecular weight (Mw) and dispersity (Mw/Mn) were measured by GPC, the following results were obtained.
(R3): Mw = 960, Mw/Mn = 1.07

A list of the Mw and Mw/Mn results for the organic film compounds (A1) to (A25) used in the Examples and the compounds (R1) to (R3) for the Comparative Examples is shown in Tables 1 to 4.

**[Table 1]**

| Synthesis Example | Compound | Mw | Mw/Mn |
|---|---|---|---|
| 1 | 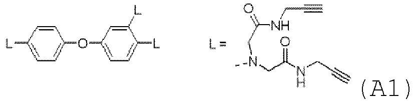 | 840 | 1.02 |
| 2 | 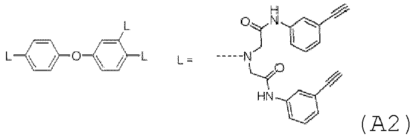 | 1,200 | 1.01 |
| 3 | 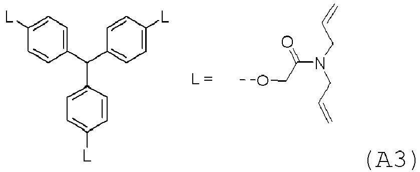 | 710 | 1.01 |
| 4 | 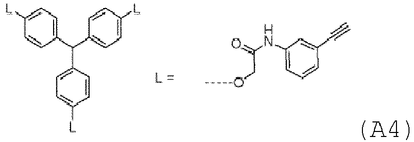 | 780 | 1.02 |
| 5 | 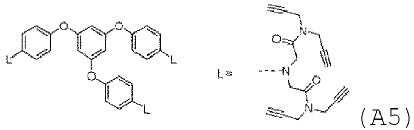 | 1,260 | 1.02 |
| 6 | 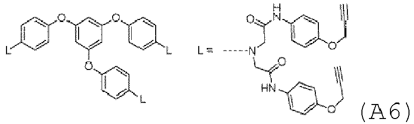 | 1,630 | 1.01 |
| 7 | 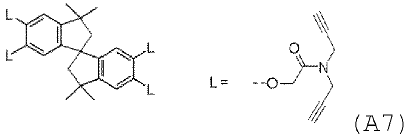 | 890 | 1.01 |
| 8 | 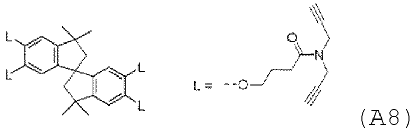 | 980 | 1.02 |

**[Table 2]**

| Synthesis Example | Compound | Mw | Mw/Mn |
|---|---|---|---|
| 9 | 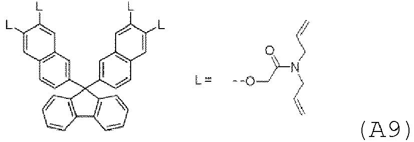 | 1,080 | 1.02 |
| 10 | 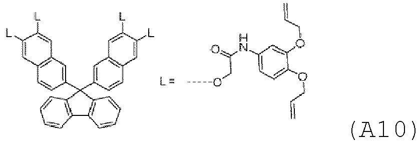 | 1,540 | 1.03 |
| 11 | 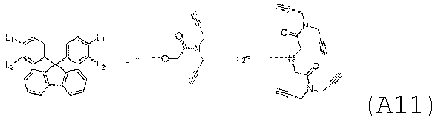 | 1,220 | 1.03 |
| 12 | 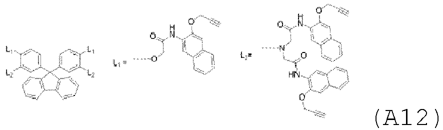 | 1,920 | 1.03 |
| 13 | 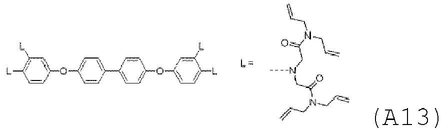 | 1,550 | 1.01 |
| 14 | 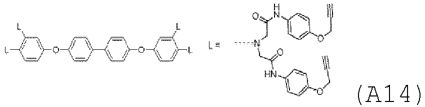 | 1,900 | 1.02 |
| 15 | 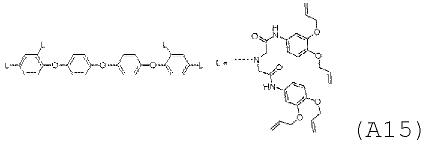 | 2,450 | 1.02 |
| 16 | 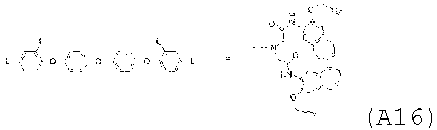 | 2,380 | 1.03 |

**[Table 3]**

| Synthesis Example | Compound | Mw | Mw/Mn |
|---|---|---|---|
| 17 | 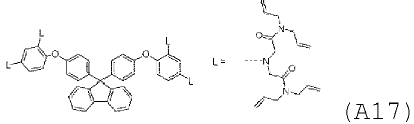 | 1,730 | 1.01 |
| 18 | 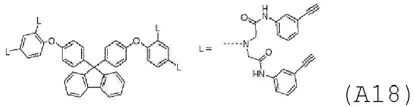 | 1,880 | 1.02 |
| 19 | 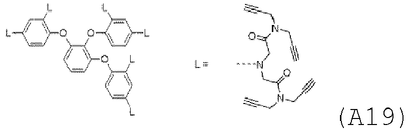 | 2,050 | 1.03 |
| 20 | 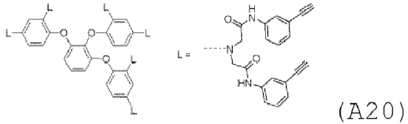 | 2,460 | 1.02 |
| 21 | 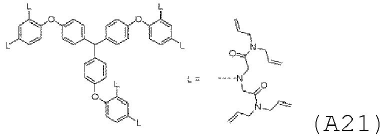 | 2, 310 | 1.03 |
| 22 | 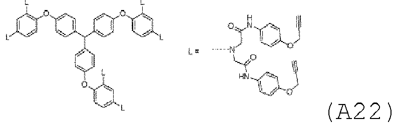 | 2,940 | 1.02 |
| 23 | 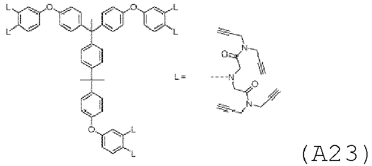 | 2,360 | 1.03 |
| 24 | 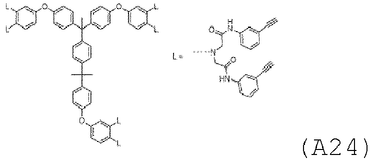 | 2,840 | 1.04 |

**[Table 4]**

| Synthesis Example | Compound | Mw | Mw/Mn |
|---|---|---|---|
| 25 | 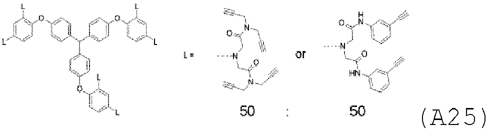 | 1,830 | 1.09 |
| Comparative Synthesis Example 1 | 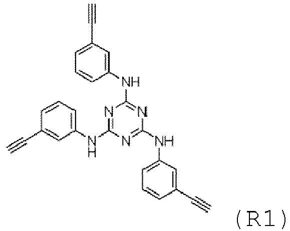 | 430 | 1.01 |
| Comparative Synthesis Example 2 | 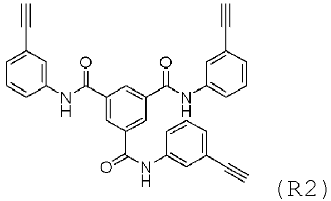 | 520 | 1.01 |
| Comparative Synthesis Example 3 | 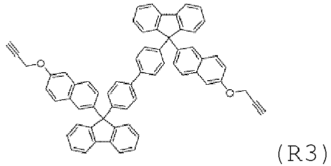 | 960 | 1.07 |

### Preparation of Materials (UDL-1 to -29, Comparative UDL-1 to -3) for Forming Organic Film

According to proportions shown in Table 5, the following were dissolved using propylene glycol monomethyl ether acetate (PGMEA) containing 0.1 mass% of PF-6320 (manufactured by Omnova Solutions, Inc.): the organic film compounds (A1) to (A25) and compounds (R1) to (R3) for the Comparative Examples; and (S1) 1,6-diacetoxyhexane, having a boiling point of 260°C, and (S2) tripropylene glycol monomethyl ether, having a boiling point of 242°C, as high-boiling-point solvents. The resulting solutions were filtered through a 0.1-µm filter made of a fluorinated resin. Thus, materials (UDL-1 to -29, comparative UDL-1 to -3) for forming an organic film were prepared.

**[Table 5]**

| Material for forming organic film | Compound | High-boiling-point solvent | PGMEA |
|---|---|---|---|
| | (parts by mass) | (parts by mass) | (parts by mass) |
| UDL-1 | A1(10) | - | 90 |
| UDL-2 | A2(10) | - | 90 |
| UDL-3 | A3(10) | - | 90 |
| UDL-4 | A4(10) | - | 90 |
| UDL-5 | A5(10) | - | 90 |
| UDL-6 | A6(10) | - | 90 |
| UDL-7 | A7(10) | - | 90 |
| UDL-8 | A8(10) | - | 90 |
| UDL-9 | A9(10) | - | 90 |
| UDL-10 | A10(10) | - | 90 |
| UDL-11 | A11(10) | - | 90 |
| UDL-12 | A12(10) | - | 90 |
| UDL-13 | A13(10) | - | 90 |
| UDL-14 | A14(10) | - | 90 |
| UDL-15 | A15(10) | - | 90 |
| UDL-16 | A16(10) | - | 90 |
| UDL-17 | A17(10) | - | 90 |
| UDL-18 | A18(10) | - | 90 |
| UDL-19 | A19(10) | - | 90 |
| UDL-20 | A20(10) | - | 90 |
| UDL-21 | A21(10) | - | 90 |
| UDL-22 | A22(10) | - | 90 |
| UDL-23 | A23(10) | - | 90 |
| UDL-24 | A24(10) | - | 90 |
| UDL-25 | A25(10) | - | 90 |
| UDL-26 | A1(10) | S1(10) | 80 |
| UDL-27 | A3(10) | 32(10) | 80 |
| UDL-28 | A14(10) | S1(10) | 80 |
| UDL-29 | A18(10) | 32(10) | 80 |
| Comparative UDL-1 | R1(10) | - | 90 |
| Comparative UDL-2 | R2(10) | - | 90 |
| Comparative UDL-3 | R3 (10) | - | 90 |

### Example 1: Solvent Resistance Measurement (Examples 1-1 to 1-29, Comparative Examples 1-1 to 1-3)

A silicon substrate was coated with one of the UDL-1 to -29 and comparative UDL-1 to -3 prepared above and baked in the atmosphere at the temperature shown in Table 6 for 60 seconds. Then, the film thickness was measured. A PGMEA solvent was dispensed on each film and allowed to stand for 30 seconds. The resultant was spin-dried and baked at 100°C for 60 seconds to evaporate the PGMEA, and the film thicknesses before and after the PGMEA treatment were measured. Using the film thickness "a" after the film formation and the film thickness "b" after the PGMEA treatment, the film remaining percentage was determined. Table 6 shows the results.

**[Table 6]**

| | Material for forming organic film | Baking temperature | Film thickness after film formation: a (Å) | Film thickness after PGMEA treatment: b (Å) | b/a × 100 (%) |
|---|---|---|---|---|---|
| Example 1-1 | UDL-1 | 400°C × 60 seconds | 2000 | 1996 | 99.8 |
| Example 1-2 | UDL-2 | 400°C × 60 seconds | 1991 | 1987 | 99.8 |
| Example 1-3 | UDL-3 | 400°C × 60 seconds | 1995 | 1993 | 99.9 |
| Example 1-4 | UDL-4 | 400°C × 60 seconds | 1997 | 1997 | 100 |
| Example 1-5 | UDL-3 | 400°C × 60 seconds | 2008 | 2006 | 99.9 |
| Example 1-6 | UDL-6 | 400°C × 60 seconds | 2012 | 2008 | 99.8 |
| Example 1-7 | UDL-7 | 400°C × 60 seconds | 2000 | 1998 | 99.9 |
| Example 1-8 | UDL-8 | 400°C × 60 seconds | 2003 | 1997 | 99.7 |
| Example 1-9 | UDL-9 | 400°C × 60 seconds | 1993 | 1987 | 99.7 |
| Example 1-10 | UDL-10 | 400°C × 60 seconds | 2005 | 2001 | 99.8 |
| Example 1-11 | UDL-11 | 400°C × 60 seconds | 2009 | 2007 | 99.9 |
| Example 1-12 | UDL-12 | 400°C × 60 seconds | 2001 | 1995 | 99.7 |
| Example 1-13 | UDL-13 | 400°C × 60 seconds | 1989 | 1983 | 99.7 |
| Example 1-14 | UDL-14 | 400°C × 60 seconds | 2002 | 1998 | 99.8 |
| Example 1-15 | UDL-15 | 400°C × 60 seconds | 2002 | 1996 | 99.7 |
| Example 1-16 | UDL-16 | 400°C × 60 seconds | 2003 | 2003 | 100 |
| Example 1-17 | UDL-17 | 400°C × 60 seconds | 1998 | 1992 | 99.7 |
| Example 1-18 | UDL-18 | 400°C × 60 seconds | 2011 | 2003 | 99.7 |
| Example 1-19 | UDL-19 | 400°C × 60 seconds | 1997 | 1997 | 100 |
| Example 1-20 | UDL-20 | 400°C × 60 seconds | 1998 | 1992 | 99.7 |
| Example 1-21 | UDL-21 | 400°C × 60 seconds | 1995 | 1991 | 99.8 |
| Example 1-22 | UDL-22 | 400°C × 60 seconds | 1992 | 1990 | 99.9 |
| Example 1-23 | UDL-23 | 400°C × 60 seconds | 2011 | 2011 | 100 |
| Example 1-24 | UDL-24 | 400°C × 60 seconds | 1997 | 1993 | 99.8 |
| Example 1-25 | UDL-23 | 400°C × 60 seconds | 2004 | 2000 | 99.8 |
| Example 1-26 | UDL-26 | 400°C × 60 seconds | 2002 | 1998 | 99.8 |
| Example 1-27 | UDL-27 | 400°C × 60 seconds | 2000 | 1998 | 99.9 |
| Example 1-28 | UDL-28 | 400°C × 60 seconds | 2006 | 2002 | 99.8 |
| Example 1-29 | UDL-29 | 400°C × 60 seconds | 2013 | 2007 | 99.7 |
| Comparative Example 1-1 | Comparative UDL-1 | 400°C × 60 seconds | 2015 | 2009 | 99.7 |
| Comparative Example 1-2 | Comparative UDL-2 | 400°C × 60 seconds | 2015 | 2015 | 100 |
| Comparative Example 1-3 | Comparative UDL-3 | 400°C × 60 seconds | 1998 | 1994 | 99.8 |

As shown in Table 6, the organic films (Examples 1-1 to 1-29) using the inventive organic film compounds had a film remaining percentage after the PGMEA treatment of more than 99.5%, and it can be observed that a crosslinking reaction was caused by the heat treatment, and sufficient solvent resistance was exhibited.

### Example 2: Heat Resistance Evaluation (Examples 2-1 to 2-29, Comparative Examples 2-1 to 2-3)

A silicon substrate was coated with one of the materials (UDL-1 to -29, comparative UDL-1 to -3) for forming an organic film and baked in the atmosphere at the temperature shown in Table 7 for 60 seconds to form a coating film of about 200 nm. The film thickness "a" was measured. The substrate was further baked at 400°C for 20 minutes under such a nitrogen stream that the oxygen concentration was controlled to 0.2% or less. Then, the film thickness "b" was measured. The film remaining percentage was calculated from the film thickness "a" after the baking and the film thickness "b" after the baking. Table 7 shows the results.

**[Table 7]**

| | Material for forming organic film | Baking temperature | Film thickness after baking: a (Å) | Film thickness after baking: b (Å) | b/a × 100 |
|---|---|---|---|---|---|
| | | | | | (%) |
| Example 2-1 | UDL-1 | 400°C × 60 seconds | 2000 | 1914 | 95.7 |
| Example 2-2 | UDL-2 | 400°C × 60 seconds | 1991 | 1971 | 99 |
| Example 2-3 | UDL-3 | 400°C × 60 seconds | 1995 | 1899 | 95.2 |
| Example 2-4 | UDL-4 | 400°C × 60 seconds | 1997 | 1981 | 99.2 |
| Example 2-5 | UDL-3 | 400°C × 60 seconds | 2008 | 1992 | 99.2 |
| Example 2-6 | UDL-6 | 400°C × 60 seconds | 2012 | 1919 | 95.4 |
| Example 2-7 | UDL-7 | 400°C × 60 seconds | 2000 | 1996 | 99.8 |
| Example 2-8 | UDL-8 | 400°C × 60 seconds | 2003 | 1907 | 95.2 |
| Example 2-9 | UDL-9 | 400°C × 60 seconds | 1993 | 1899 | 95.3 |
| Example 2-10 | UDL-10 | 400°C × 60 seconds | 2003 | 1995 | 99.5 |
| Example 2-11 | UDL-11 | 400°C × 60 seconds | 2009 | 1985 | 98.8 |
| Example 2-12 | UDL-12 | 400°C × 60 seconds | 2001 | 1997 | 99.8 |
| Example 2-13 | UDL-13 | 400°C × 60 seconds | 1989 | 1892 | 95.1 |
| Example 2-14 | UDL-14 | 400°C × 60 seconds | 2002 | 1994 | 99.6 |
| Example 2-15 | UDL-15 | 400°C × 60 seconds | 2002 | 1990 | 99.4 |
| Example 2-16 | UDL-16 | 400°C × 60 seconds | 2005 | 1987 | 99.1 |
| Example 2-17 | UDL-17 | 400°C × 60 seconds | 1998 | 1912 | 95.7 |
| Example 2-18 | UDL-18 | 400°C × 60 seconds | 2011 | 2007 | 99.8 |
| Example 2-19 | UDL-19 | 400°C × 60 seconds | 1997 | 1973 | 98.8 |
| Example 2-20 | UDL-20 | 400°C × 60 seconds | 1998 | 1994 | 99.8 |
| Example 2-21 | UDL-21 | 400°C × 60 seconds | 1995 | 1897 | 95.1 |
| Example 2-22 | UDL-22 | 400°C × 60 seconds | 1992 | 1988 | 99.8 |
| Example 2-23 | UDL-23 | 400°C × 60 seconds | 2011 | 1997 | 99.3 |
| Example 2-24 | UDL-24 | 400°C × 60 seconds | 1997 | 1995 | 99.9 |
| Example 2-25 | UDL-23 | 400°C × 60 seconds | 2004 | 1992 | 99.4 |
| Example 2-26 | UDL-26 | 400°C × 60 seconds | 2002 | 1916 | 95.7 |
| Example 2-27 | UDL-27 | 400°C × 60 seconds | 2000 | 1904 | 95.2 |
| Example 2-28 | UDL-28 | 400°C × 60 seconds | 2006 | 1998 | 99.6 |
| Example 2-29 | UDL-29 | 400°C × 60 seconds | 2013 | 2009 | 99.8 |
| Comparative Example 2-1 | Comparative UDL-1 | 400°C × 60 seconds | 2015 | 2003 | 99.4 |
| Comparative Example 2-2 | Comparative UDL-2 | 400°C × 60 seconds | 2015 | 1997 | 99.1 |
| Comparative Example 2-3 | Comparative UDL-3 | 400°C × 60 seconds | 1998 | 1988 | 99.5 |

As shown in Table 7, the organic films where the inventive organic film compounds were used (Examples 2-1 to 2-29) had a film thickness decrease of less than 5% even after baking at 400°C over a long time. This indicates that the inventive materials for forming an organic film were excellent in heat resistance. In particular, the compounds in which n₂ = 1 in the structures represented by the general formulae (2) and in which a propargyl group or an ethynyl group has been introduced provide films that maintain a film remaining percentage of 98% or more, and this indicates particularly excellent heat resistance.

### Example 3: Filling Property Evaluation (Examples 3-1 to 3-29, Comparative Examples 3-1 to 3-3)

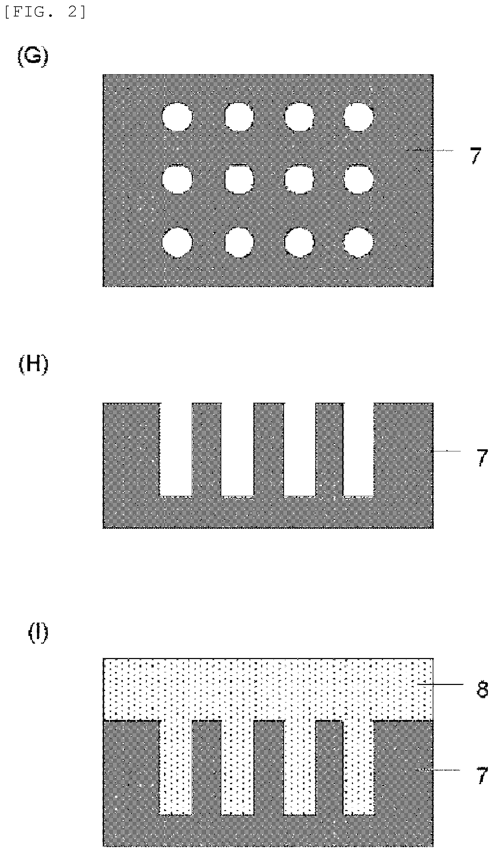

The materials (UDL-1 to -29, comparative UDL-1 to -3) for forming an organic film prepared above were each applied respectively onto an SiO₂ wafer substrate having a dense hole pattern (hole diameter: 0.16 um, hole depth: 0.50 um, distance between the centers of two adjacent holes: 0.32 µm) and baked in the atmosphere at the temperature shown in Table 8 for 60 seconds to form an organic film. The substrate used was a base substrate 7 (SiO₂ wafer substrate) having a dense hole pattern as shown in FIG. 2 (G) (top view) and (H) (sectional view). The film-formability and filling property on each of the obtained wafer substrates were evaluated.

Regarding film-formability, each of the obtained wafers was visually observed. The film-formability was evaluated as good when no dewetting was observed, and evaluated as poor when dewetting was observed. Regarding filling property, the sectional profile was observed with a scanning electron microscope (SEM) to check whether or not the holes were filled with the organic film 8 without voids (space). Table 8 shows the results. In this evaluation, when an organic film material having good filling property is used, the holes are filled with the organic film without voids as shown in FIG. 2 (I), and the material is evaluated as good. If an organic film material having poor filling property is used, voids occur inside the holes, and the material is evaluated as poor.

**[Table 8]**

| | Material for forming organic film | Baking temperature | Film-formability | Filling property |
|---|---|---|---|---|
| Example 3-1 | UDL-1 | 400°C × 60 seconds | Good | Good |
| Example 3-2 | UDL-2 | 400°C × 60 seconds | Good | Good |
| Example 3-3 | UDL-3 | 400°C × 60 seconds | Good | Good |
| Example 3-4 | UDL-4 | 400°C × 60 seconds | Good | Good |
| Example 3-5 | UDL-5 | 400°C × 60 seconds | Good | Good |
| Example 3-6 | UDL-6 | 400°C × 60 seconds | Good | Good |
| Example 3-7 | UDL-7 | 400°C × 60 seconds | Good | Good |
| Example 3-8 | UDL-8 | 400°C × 60 seconds | Good | Good |
| Example 3-9 | UDL-9 | 400°C × 60 seconds | Good | Good |
| Example 3-10 | UDL-10 | 400°C × 60 seconds | Good | Good |
| Example 3-11 | UDL-11 | 400°C × 60 seconds | Good | Good |
| Example 3-12 | UDL-12 | 400°C × 60 seconds | Good | Good |
| Example 3-13 | UDL-13 | 400°C × 60 seconds | Good | Good |
| Example 3-14 | UDL-14 | 400°C × 60 seconds | Good | Good |
| Example 3-15 | UDL-15 | 400°C × 60 seconds | Good | Good |
| Example 3-16 | UDL-16 | 400°C × 60 seconds | Good | Good |
| Example 3-17 | UDL-17 | 400°C × 60 seconds | Good | Good |
| Example 3-18 | UDL-18 | 400°C × 60 seconds | Good | Good |
| Example 3-19 | UDL-19 | 400°C × 60 seconds | Good | Good |
| Example 3-20 | UDL-20 | 400°C × 60 seconds | Good | Good |
| Example 3-21 | UDL-21 | 400°C × 60 seconds | Good | Good |
| Example 3-22 | UDL-22 | 400°C × 60 seconds | Good | Good |
| Example 3-23 | UDL-23 | 400°C × 60 seconds | Good | Good |
| Example 3-24 | UDL-24 | 400°C × 60 seconds | Good | Good |
| Example 3-25 | UDL-25 | 400°C × 60 seconds | Good | Good |
| Example 3-26 | UDL-26 | 400°C × 60 seconds | Good | Good |
| Example 3-27 | UDL-27 | 400°C × 60 seconds | Good | Good |
| Example 3-28 | UDL-28 | 400°C × 60 seconds | Good | Good |
| Example 3-29 | UDL-29 | 400°C × 60 seconds | Good | Good |
| Comparative Example 3-1 | Comparative UDL-1 | 400°C × 60 seconds | Poor | - |
| Comparative Example 3-2 | Comparative UDL-2 | 400°C × 60 seconds | Poor | - |
| Comparative Example 3-3 | Comparative UDL-3 | 400°C × 60 seconds | Good | Good |

As shown by Examples 3-1 to 3-29 in Table 8, it was revealed that every material for forming an organic film using the inventive organic film compound was capable of forming a film on the above-described SiO₂ wafer substrate having a dense hole pattern, was able to fill the hole patterns without voids, and was excellent in filling property. It was also possible to form a film on the substrate in Comparative Example 3-3, and it was possible to fill the hole pattern without voids. On the other hand, in Comparative Example 3-1, the compound had no amide structure, and in Comparative Example 3-2, the compound had a structure having no alkylene group between an amide structure and an aromatic ring, and therefore, film-formability was poor, and film formation failure (dewetting) occurred on the substrate. It is thought that the structures of both of the compounds used in these materials for forming an organic film had high thermal flowability but adhesion to the substrate was weak because the compound did not have an amide structure, or cohesion was strong due to high crystallinity caused by the compound not having an alkylene group between the amide structure and the aromatic ring, and that the film formation failure thus occurred.

### Example 4: Planarizing Property Evaluation (Examples 4-1 to 4-29, Comparative Examples 4-1 to 4-3)

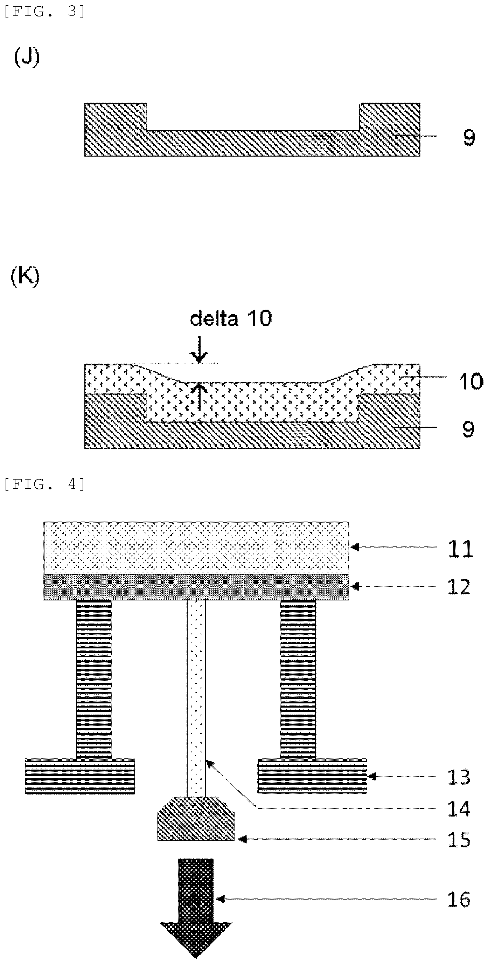

The materials (UDL-1 to -29, comparative UDL-1 to -3) for forming an organic film prepared above were each applied respectively onto a base substrate 9 (SiO₂ wafer substrate) having a giant isolated trench pattern (trench width: 50 um, trench depth: 0.115 um) shown in FIG. 3 (J) and baked in the atmosphere at the temperature shown in Table 9 for 60 seconds. Then, a step (delta 10) between the trench portion and the non-trench portion of an organic film 10 shown in FIG. 3 (K) was observed with Alpha-Step D-600 (stylus profiler) manufactured by KLA-Tencor Corporation. Table 9 shows the results. In this evaluation, the evaluation S was given when the step was less than 60 nm, A when 60 nm or more and less than 80 nm, B when 80 nm or more and less than 100 nm, C when 100 nm or more and less than 115 nm, and D when 115 nm or more. In this evaluation, the smaller the step, the better the planarizing property. Note that, in this evaluation, a trench pattern having a depth of 0.115 um was generally planarized using a material for forming an organic film at a film thickness of approximately 0.2 um. This is a severe evaluation condition to evaluate the planarizing property.

**[Table 9]**

| | Material for forming organic film | Baking temperature | Film-formability | Flatness |
|---|---|---|---|---|
| Example 4-1 | UDL-1 | 400°C × 60 seconds | Good | A |
| Example 4-2 | UDL-2 | 400°C × 60 seconds | Good | A |
| Example 4-3 | UDL-3 | 400°C × 60 seconds | Good | A |
| Example 4-4 | UDL-4 | 400°C × 60 seconds | Good | B |
| Example 4-5 | UDL-5 | 400°C × 60 seconds | Good | S |
| Example 4-6 | UDL-6 | 400°C × 60 seconds | Good | A |
| Example 4-7 | UDL-7 | 400°C × 60 seconds | Good | A |
| Example 4-8 | UDL-8 | 400°C × 60 seconds | Good | A |
| Example 4-9 | UDL-9 | 400°C × 60 seconds | Good | A |
| Example 4-10 | UDL-10 | 400°C × 60 seconds | Good | B |
| Example 4-11 | UDL-11 | 400°C × 60 seconds | Good | A |
| Example 4-12 | UDL-12 | 400°C × 60 seconds | Good | C |
| Example 4-13 | UDL-13 | 400°C × 60 seconds | Good | S |
| Example 4-14 | UDL-14 | 400°C × 60 seconds | Good | A |
| Example 4-15 | UDL-15 | 400°C × 60 seconds | Good | A |
| Example 4-16 | UDL-16 | 400°C × 60 seconds | Good | B |
| Example 4-17 | UDL-17 | 400°C × 60 seconds | Good | S |
| Example 4-18 | UDL-18 | 400°C × 60 seconds | Good | A |
| Example 4-19 | UDL-19 | 400°C × 60 seconds | Good | S |
| Example 4-20 | UDL-20 | 400°C × 60 seconds | Good | A |
| Example 4-21 | UDL-21 | 400°C × 60 seconds | Good | S |
| Example 4-22 | UDL-22 | 400°C × 60 seconds | Good | A |
| Example 4-23 | UDL-23 | 400°C × 60 seconds | Good | S |
| Example 4-24 | UDL-24 | 400°C × 60 seconds | Good | A |
| Example 4-25 | UDL-25 | 400°C × 60 seconds | Good | S |
| Example 4-26 | UDL-26 | 400°C × 60 seconds | Good | S |
| Example 4-27 | UDL-27 | 400°C × 60 seconds | Good | A |
| Example 4-28 | UDL-28 | 400°C × 60 seconds | Good | S |
| Example 4-29 | UDL-29 | 400°C × 60 seconds | Good | S |
| Comparative Example 4-1 | Comparative UDL-1 | 400°C × 60 seconds | Poor | - |
| Comparative Example 4-2 | Comparative UDL-2 | 400°C × 60 seconds | Poor | - |
| Comparative Example 4-3 | Comparative UDL-3 | 400°C × 60 seconds | Good | D |

As shown by Examples 4-1 to 4-29 in Table 9, every material for forming an organic film had better film-formability when the inventive organic film compounds were used compared with Comparative Examples 4-1 and 4-2, and moreover, had smaller steps in the organic film between the trench portion and the non-trench portion compared with Comparative Example 4-3, showing that planarizing property was excellent. In particular, the compounds having an ether bond as shown in the general formula (8) and compounds having a tertiary amide structure had excellent thermal flowability, and therefore, had improved planarizing property, and furthermore, the organic film materials containing a compound having both of these characteristics had the best planarizing property. In addition, the materials for forming an organic film containing a high-boiling-point solvent were provided with thermal flowability, and therefore, planarizing property was better than that of the organic film materials containing no high-boiling-point solvent (Examples 4-26 to 4-29). On the other hand, in Comparative Examples 4-1 and 4-2, film formation failure (dewetting) occurred on the substrate with the giant isolated trench pattern, and this is thought to be for the same reason film formation failure occurred on the SiO₂ wafer substrates having the dense hole pattern. Meanwhile, the compound in Comparative Example 4-3 had poor thermal flowability, and resulted in low planarizing property.

### Example 5: Adhesiveness Test (Examples 5-1 to 5-29, Comparative Examples 5-1 to 5-3)

The materials (UDL-1 to -29, comparative UDL-1 to -3) for forming an organic film were respectively applied onto SiO₂ wafer substrates and baked using a hot plate in the atmosphere at the temperature shown in Table 10 for 60 seconds. Thus, organic films each with a film thickness of 200 nm were formed. This wafer with an organic film was cut into a 1 × 1 cm square, and an aluminum pin with epoxy adhesive was fastened to the cut wafer with a dedicated jig. Thereafter, the assembly was heated with an oven at 150°C for 1 hour to bond the aluminum pin to the substrate. After cooling to room temperature, initial adhesiveness was evaluated based on the resistance force by a thin-film adhesion strength measurement apparatus (Sebastian Five-A).

FIG. 4 shows an explanatory diagram of the adhesiveness measurement method. In FIG. 4, reference number 11 denotes a silicon wafer (substrate), 12 denotes a cured film, 14 denotes an aluminum pin with adhesive, 13 denotes a support, 15 denotes a grip, and 16 denotes a tensile direction. Each adhesive force is an average of 12 measurement points, and a larger value indicates that the organic film has higher adhesiveness with respect to the substrate. The adhesiveness was evaluated by comparing the obtained values. Table 10 shows the results.

**[Table 10]**

| | Material for forming organic film | Baking temperature | Adhesion (mN) |
|---|---|---|---|
| Example 5-1 | UDL-1 | 400°C × 60 seconds | 660 |
| Example 5-2 | UDL-2 | 400°C × 60 seconds | 680 |
| Example 5-3 | UDL-3 | 400°C × 60 seconds | 650 |
| Example 5-4 | UDL-4 | 400°C × 60 seconds | 690 |
| Example 5-5 | UDL-5 | 400°C × 60 seconds | 590 |
| Example 5-6 | UDL-6 | 400°C × 60 seconds | 690 |
| Example 5-7 | UDL-7 | 400°C × 60 seconds | 630 |
| Example 5-8 | UDL-8 | 400°C × 60 seconds | 600 |
| Example 5-9 | UDL-9 | 400°C × 60 seconds | 620 |
| Example 5-10 | UDL-10 | 400°C × 60 seconds | 660 |
| Example 5-11 | UDL-11 | 400°C × 60 seconds | 600 |
| Example 5-12 | UDL-12 | 400°C × 60 seconds | 690 |
| Example 5-13 | UDL-13 | 400°C × 60 seconds | 610 |
| Example 5-14 | UDL-14 | 400°C × 60 seconds | 670 |
| Example 5-15 | UDL-15 | 400°C × 60 seconds | 690 |
| Example 5-16 | UDL-16 | 400°C × 60 seconds | 670 |
| Example 5-17 | UDL-17 | 400°C × 60 seconds | 570 |
| Example 5-18 | UDL-18 | 400°C × 60 seconds | 690 |
| Example 5-19 | UDL-19 | 400°C × 60 seconds | 640 |
| Example 5-20 | UDL-20 | 400°C × 60 seconds | 670 |
| Example 5-21 | UDL-21 | 400°C × 60 seconds | 630 |
| Example 5-22 | UDL-22 | 400°C × 60 seconds | 670 |
| Example 5-23 | UDL-23 | 400°C × 60 seconds | 630 |
| Example 5-24 | UDL-24 | 400°C × 60 seconds | 670 |
| Example 5-25 | UDL-25 | 400°C × 60 seconds | 570 |
| Example 5-26 | UDL-26 | 400°C × 60 seconds | 660 |
| Example 5-27 | UDL-27 | 400°C × 60 seconds | 650 |
| Example 5-28 | UDL-28 | 400°C × 60 seconds | 670 |
| Example 5-29 | UDL-29 | 400°C × 60 seconds | 690 |
| Comparative Example 5-1 | Comparative UDL-1 | 400°C × 60 seconds | 410 |
| Comparative Example 5-2 | Comparative UDL-2 | 400°C × 60 seconds | 660 |
| Comparative Example 5-3 | Comparative UDL-3 | 400°C × 60 seconds | 370 |

As shown in Examples 5-1 to 5-29 in Table 10, the materials for forming an organic film, which contain the inventive organic film compound having an amide structure, showed higher adhesive force compared with Comparative Examples 5-1 and 5-3. Meanwhile, the compound used in comparative ULD-2, which caused film formation failure in Comparative Example 3-2 and Comparative Example 4-2, had an amide structure, and therefore, had adhesiveness equivalent to that of the inventive organic film compounds as shown in Comparative Example 5-2. From these results, too, it is thought that the inventive materials for forming an organic film have excellent thermal flowability, and also exhibit excellent film-formability by the high adhesion due to the amide structure and the effect of alleviating crystallinity due to an alkylene group being between an amide structure and an aromatic ring.

### Example 6: Patterning Test (Examples 6-1 to 6-29, Comparative Example 6-1)

The materials (UDL-1 to -29, comparative UDL-3) for forming an organic film were respectively applied onto a base substrate 9 (SiO₂ wafer substrate) having a giant isolated trench pattern (trench width: 50 um, trench depth: 0.115 um) shown in FIG. 3 (J). Then, an organic film (resist underlayer film) was formed by baking under the conditions shown in Table 14 in the atmosphere so that the film thickness on the Bare-Si substrate was 200 nm. A silicon-containing resist middle layer film material (SOG-1) was applied onto the organic film and baked at 220°C for 60 seconds to form a resist middle layer film having a film thickness of 35 nm. A resist upper layer film material (SL resist for ArF) was applied thereon and baked at 105°C for 60 seconds to form a resist upper layer film having a film thickness of 100 nm. A liquid immersion top coat (TC-1) was applied onto the resist upper layer film and baked at 90°C for 60 seconds to form a top coat having a film thickness of 50 nm. Note that regarding comparative UDL-1 and -2, which caused film formation failure in Comparative Examples 4-1 and 4-2, it was not possible to carry out the patterning test.

The resist upper layer film material (SL resist for ArF) was prepared by: dissolving a polymer (RP1), an acid generator (PAG1), and a basic compound (Amine1) into a solvent containing 0.1 mass% FC-430 (manufactured by Sumitomo 3M Ltd.) in proportions shown in Table 11; and filtering the solution through a 0.1-pm filter made of a fluorinated resin.

**[Table 11]**

| | Polymer | Acid generator | Basic compound | Solvent |
|---|---|---|---|---|
| | (parts by mass) | (parts by mass) | (parts by mass) | (parts by mass) |
| SL resist for ArF | RP1 (100) | PAG1 (6.6) | Amine1 (0.8) | PGMEA (2500) |

The structural formulae of the polymer (RP1), acid generator (PAG1), and basic compound (Amine1) used are shown below.

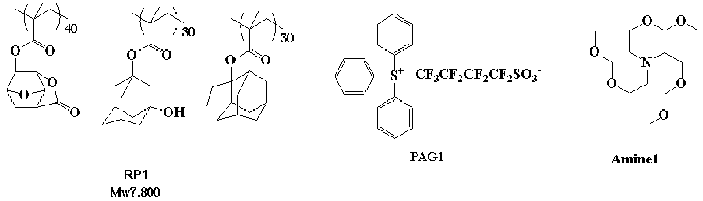

The liquid immersion top coat material (TC-1) was prepared by: dissolving a top coat polymer (PP1) into organic solvents in proportions shown in Table 12; and filtering the solution through a 0.1-µm filter made of a fluorinated resin.

**[Table 12]**

| | Top coat polymer | Organic solvent |
|---|---|---|
| | (parts by mass) | (parts by mass) |
| TC-1 | PP1 | Diisoamyl ether (2700) |
| | (100) | 2-methyl-1-butanol (270) |

The structural formula of the used top coat polymer (PP1) is shown below.

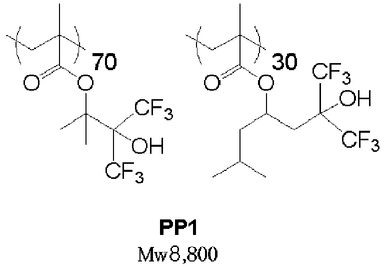

The silicon-containing resist middle layer film material (SOG-1) was prepared by: dissolving a polymer shown by an ArF silicon-containing middle layer film polymer (SiP1) and a thermal crosslinking catalyst (CAT1) into an organic solvent containing 0.1 mass% FC-4430 (manufactured by Sumitomo 3M Ltd.) in proportions shown in Table 13; and filtering the solution through a filter made of a fluorinated resin with a pore size of 0.1 µm.

**[Table 13]**

| | Polymer | Thermally crosslinking catalyst | Organic solvent |
|---|---|---|---|
| | (parts by mass) | (parts by mass) | (parts by mass) |
| SOG-1 | SiP1 (100) | CAT1 (1) | Propylene glycol monoethyl ether (4000) |

The structural formulae of the used ArF silicon-containing middle layer film polymer (SiP1) and crosslinking catalyst (CAT1) are shown below.

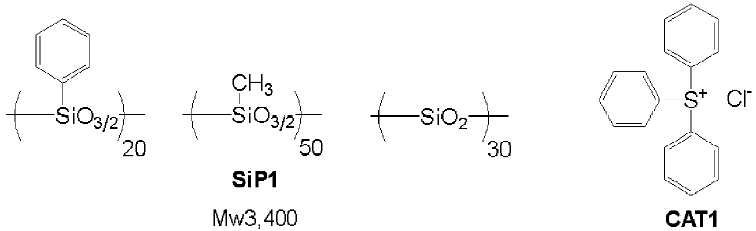

Next, the resulting substrate was exposed to light with an ArF liquid immersion exposure apparatus (NSR-S610C manufactured by Nikon Corporation, NA: 1.30, σ: 0.98/0.65, 35° s-polarized dipole illumination, 6% halftone phase shift mask) while changing the exposure, baked (PEB) at 100°C for 60 seconds, and developed with a 2.38 mass% aqueous solution of tetramethylammonium hydroxide (TMAH) for 30 seconds. Thus, a positive line and space pattern (resist upper layer film pattern) was obtained with a pitch of 100 nm and a resist line width of 50 nm to 30 nm.

Next, using an etching apparatus Telius manufactured by Tokyo Electron Limited, the silicon-containing middle layer film (SOG-1 film) was processed by dry etching while using the resist upper layer film pattern as a mask; the organic film was processed while using the SOG-1 film as a mask; and the SiO₂ wafer substrate was processed while using the organic film as a mask.

The etching conditions were as follows.

Conditions for transferring the resist upper layer film pattern to the SOG-1 film.

| | |
|---|---|
| Chamber pressure: | 10.0 Pa |
| RF power: | 1,500 W |
| CF₄ gas flow rate: | 15 sccm |
| O₂ gas flow rate: | 75 sccm |
| Time: | 15 sec |

Conditions for transferring the pattern from the SOG-1 film to the organic film.

| | |
|---|---|
| Chamber pressure: | 2.0 Pa |
| RF power: | 500 W |
| Ar gas flow rate: | 75 sccm |
| O₂ gas flow rate: | 45 sccm |
| Time: | 120 sec |

Conditions for transferring the pattern from the organic film to the SiO₂ wafer substrate.

| | |
|---|---|
| Chamber pressure: | 2.0 Pa |
| RF power: | 2,200 W |
| C₅F₁₂ gas flow rate: | 20 sccm |
| C₂F₆ gas flow rate: | 10 sccm |
| Ar gas flow rate: | 300 sccm |
| O₂ gas flow rate: | 60 sccm |
| Time: | 90 sec |

The pattern cross sections were observed with an electron microscope (S-4700) manufactured by Hitachi, Ltd., the profiles were compared, and summarized in Table 14.

**[Table 14]**

| | Material for forming organic film | Baking temperature | Pattern profile after etching for transferring to substrate |
|---|---|---|---|
| Example 6-1 | UDL-1 | 400°C × 60 seconds | Vertical profile |
| Example 6-2 | UDL-2 | 400°C × 60 seconds | Vertical profile |
| Example 6-3 | UDL-3 | 400°C × 60 seconds | Vertical profile |
| Example 6-4 | UDL-4 | 400°C × 60 seconds | Vertical profile |
| Example 6-5 | UDL-5 | 400°C × 60 seconds | Vertical profile |
| Example 6-6 | UDL-6 | 400°C × 60 seconds | Vertical profile |
| Example 6-7 | UDL-7 | 400°C × 60 seconds | Vertical profile |
| Example 6-8 | UDL-8 | 400°C × 60 seconds | Vertical profile |
| Example 6-9 | UDL-9 | 400°C × 60 seconds | Vertical profile |
| Example 6-10 | UDL-10 | 400°C × 60 seconds | Vertical profile |
| Example 6-11 | UDL-11 | 400°C × 60 seconds | Vertical profile |
| Example 6-12 | UDL-12 | 400°C × 60 seconds | Vertical profile |
| Example 6-13 | UDL-13 | 400°C × 60 seconds | Vertical profile |
| Example 6-14 | UDL-14 | 400°C × 60 seconds | Vertical profile |
| Example 6-15 | UDL-15 | 400°C × 60 seconds | Vertical profile |
| Example 6-16 | UDL-16 | 400°C × 60 seconds | Vertical profile |
| Example 6-17 | UDL-17 | 400°C × 60 seconds | Vertical profile |
| Example 6-18 | UDL-18 | 400°C × 60 seconds | Vertical profile |
| Example 6-19 | UDL-19 | 400°C × 60 seconds | Vertical profile |
| Example 6-20 | UDL-20 | 400°C × 60 seconds | Vertical profile |
| Example 6-21 | UDL-21 | 400°C × 60 seconds | Vertical profile |
| Example 6-22 | UDL-22 | 400°C × 60 seconds | Vertical profile |
| Example 6-23 | UDL-23 | 400°C × 60 seconds | Vertical profile |
| Example 6-24 | UDL-24 | 400°C × 60 seconds | Vertical profile |
| Example 6-25 | UDL-25 | 400°C × 60 seconds | Vertical profile |
| Example 6-26 | UDL-26 | 400°C × 60 seconds | Vertical profile |
| Example 6-27 | UDL-27 | 400°C × 60 seconds | Vertical profile |
| Example 6-28 | UDL-28 | 400°C × 60 seconds | Vertical profile |
| Example 6-29 | UDL-29 | 400°C × 60 seconds | Vertical profile |
| Comparative Example 6-1 | Comparative UDL-3 | 400°C × 60 seconds | Pattern collapse |

As shown in Table 14, it was confirmed from the results of the inventive materials for forming an organic film in Examples 6-1 to 6-29 that the resist upper layer film pattern was favorably transferred to the final substrate in each case, and that the inventive materials for forming an organic film are suitably used in fine processing according to the multilayer resist method. On the other hand, in Comparative Example 6-1, steps on the patterned substrate were large, as indicated by the results of Comparative Example 4-3, and there arose portions where the focus was out of place at the time of exposure. This caused a defect in the pattern profile of the resist upper layer film, and therefore, pattern collapse had partly occurred during pattern processing.

From the above, it was revealed that the inventive organic film compounds and the inventive materials for forming an organic film, containing the organic film compounds, have good film-formability, good adhesiveness, and excellent filling and planarizing properties. Thus, the inventive compounds and materials are extremely useful for organic films (resist underlayer films) used in multilayer resist methods. Moreover, the inventive patterning process using these materials can form a fine pattern with high precision even when the body to be processed is a stepped substrate.

The present description includes the following embodiments.
[1]: A material for forming an organic film, comprising: an organic film compound represented by the following general formula (1); and an organic solvent, 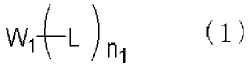 wherein W₁ represents an organic group having a valency of "n₁" and containing an aromatic ring, each L represents a substituent on the aromatic ring and is L₁, L₂, or both, represented by the following general formulae (2), and when a total number of the substituents L on the aromatic ring is "n₁", a number of L₁ is "x", and a number of L₂ is "y", "n₁", "x", and "y" satisfy relationships x + y = n₁, 2 ≤ n₁ ≤ 8, 0 ≤ y ≤ 8, and 0 ≤ x ≤ 8, 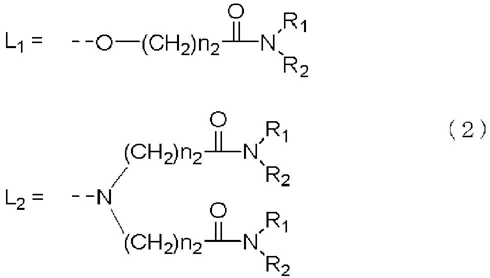 wherein "n₂" represents an integer of 1 to 3, R₁ represents one of the following formulae (3), and R₂ represents one of the following general formulae (4), 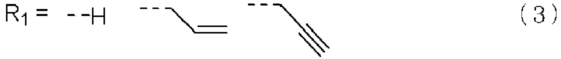 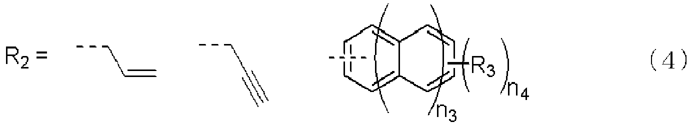 wherein R₃ represents one of the following formulae (5), "n₃" represents 0 or 1, and "n₄" represents 1 or 2. 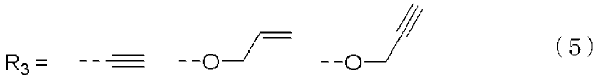
[2]: The material for forming an organic film of the above [1], wherein the L₁ and L₂ constituting the substituents L on the aromatic ring of the organic film compound represented by the general formula (1) are represented by the following general formulae (6), 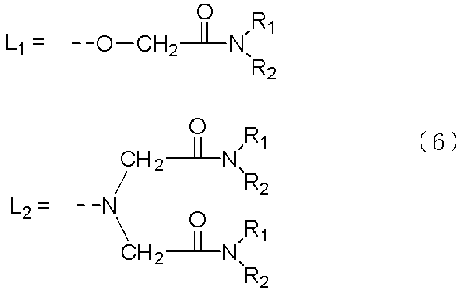 wherein R₁ and R₂ are as defined above.
[3]: The material for forming an organic film of the above [1] or [2], wherein the R₁ and R₂ in the L₁ and L₂ constituting the substituents L on the aromatic ring of the organic film compound represented by the general formula (1) are any of combinations shown by the following general formulae (7), 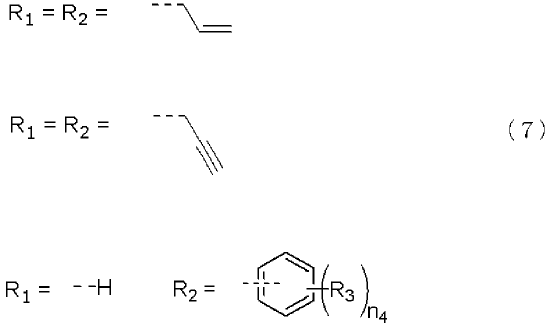 wherein R₃ and "n₄" are as defined above.
[4]: The material for forming an organic film of any one of the above [1] to [3], wherein the total number "n₁" of the substituents L on the aromatic ring of the organic film compound represented by the general formula (1) satisfies 3 ≤ n₁ ≤ 6.
[5]: The material for forming an organic film of any one of the above [1] to [4], wherein the organic film compound satisfies 1.00 ≤ Mw/Mn ≤ 1.15, where Mw is a weight-average molecular weight and Mn is a number-average molecular weight measured by gel permeation chromatography in terms of polystyrene.
[6]: The material for forming an organic film of any one of the above [1] to [5], wherein the organic solvent is a mixture of one or more kinds of organic solvent having a boiling point of lower than 180°C and one or more kinds of organic solvent having a boiling point of 180°C or higher.
[7]: The material for forming an organic film of any one of the above [1] to [6], further comprising at least one of a surfactant and a plasticizer.
[8]: A patterning process for forming a pattern in a body to be processed, comprising:
   forming an organic film by using the material for forming an organic film of any one of the above [1] to [7] on a body to be processed;
   forming a silicon-containing resist middle layer film by using a silicon-containing resist middle layer film material on the organic film;
   forming a resist upper layer film by using a photoresist composition on the silicon-containing resist middle layer film;
   forming a circuit pattern in the resist upper layer film;
   transferring the pattern to the silicon-containing resist middle layer film by etching while using the resist upper layer film having the formed pattern as a mask;
   transferring the pattern to the organic film by etching while using the silicon-containing resist middle layer film having the transferred pattern as a mask; and
   further forming the pattern in the body to be processed by etching the body to be processed while using the organic film having the transferred pattern as a mask.
[9]: A patterning process for forming a pattern in a body to be processed, comprising:
   forming an organic film by using the material for forming an organic film of any one of the above [1] to [7] on a body to be processed;
   forming a silicon-containing resist middle layer film by using a silicon-containing resist middle layer film material on the organic film;
   forming an organic antireflective coating on the silicon-containing resist middle layer film;
   forming a resist upper layer film by using a photoresist composition on the organic antireflective coating, so that a 4-layered film structure is constructed;
   forming a circuit pattern in the resist upper layer film;
   transferring the pattern to the organic antireflective coating and the silicon-containing resist middle layer film by etching while using the resist upper layer film having the formed pattern as a mask;
   transferring the pattern to the organic film by etching while using the silicon-containing resist middle layer film having the transferred pattern as a mask; and
   further forming the pattern in the body to be processed by etching the body to be processed while using the organic film having the transferred pattern as a mask.
[10]: A patterning process for forming a pattern in a body to be processed, comprising:
   forming an organic film by using the material for forming an organic film of any one of the above [1] to [7] on a body to be processed;
   forming an inorganic hard mask middle layer film selected from a silicon oxide film, a silicon nitride film, and a silicon oxynitride film on the organic film;
   forming a resist upper layer film by using a photoresist composition on the inorganic hard mask middle layer film;
   forming a circuit pattern in the resist upper layer film;
   transferring the pattern to the inorganic hard mask middle layer film by etching while using the resist upper layer film having the formed pattern as a mask;
   transferring the pattern to the organic film by etching while using the inorganic hard mask middle layer film having the transferred pattern as a mask; and
   further forming the pattern in the body to be processed by etching the body to be processed while using the organic film having the transferred pattern as a mask.
[11]: A patterning process for forming a pattern in a body to be processed, comprising:
   forming an organic film by using the material for forming an organic film of any one of the above [1] to [7] on a body to be processed;
   forming an inorganic hard mask middle layer film selected from a silicon oxide film, a silicon nitride film, and a silicon oxynitride film on the organic film;
   forming an organic antireflective coating on the inorganic hard mask middle layer film;
   forming a resist upper layer film by using a photoresist composition on the organic antireflective coating, so that a 4-layered film structure is constructed;
   forming a circuit pattern in the resist upper layer film;
   transferring the pattern to the organic antireflective coating and the inorganic hard mask middle layer film by etching while using the resist upper layer film having the formed pattern as a mask;
   transferring the pattern to the organic film by etching while using the inorganic hard mask middle layer film having the transferred pattern as a mask; and
   further forming the pattern in the body to be processed by etching the body to be processed while using the organic film having the transferred pattern as a mask.
[12]: The patterning process of the above [10] or [11], wherein the inorganic hard mask middle layer film is formed by a CVD method or an ALD method.
[13]: The patterning process of any one of the above [8] to [12], wherein the pattern in the resist upper layer film is formed by a lithography using light with a wavelength of 10 nm or more to 300 nm or less, a direct drawing with electron beam, nanoimprinting, or a combination thereof.
[14]: The patterning process of any one of the above [8] to [13], wherein alkali development or development with an organic solvent is performed as a development method in the patterning process.
[15]: The patterning process of any one of the above [8] to [14], wherein the body to be processed is a semiconductor device substrate or the semiconductor device substrate coated with any of a metal film, a metal carbide film, a metal oxide film, a metal nitride film, a metal oxycarbide film, and a metal oxynitride film.
[16]: The patterning process of the above [15], wherein the metal is silicon, titanium, tungsten, hafnium, zirconium, chromium, germanium, cobalt, copper, silver, gold, aluminum, indium, gallium, arsenic, palladium, iron, tantalum, iridium, manganese, molybdenum, ruthenium, or an alloy thereof.
[17]: An organic film compound represented by the following general formula (1), 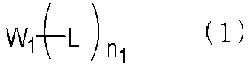 wherein W₁ represents an organic group having a valency of "n₁" and containing an aromatic ring, each L represents a substituent on the aromatic ring and is L₁, L₂, or both, represented by the following general formulae (2), and when a total number of the substituents L on the aromatic ring is "n₁", a number of L₁ is "x", and a number of L₂ is "y", "n₁", "x", and "y" satisfy relationships x + y = n₁, 2 ≤ n₁ ≤ 8, 0 ≤ y ≤ 8, and 0 ≤ x ≤ 8, 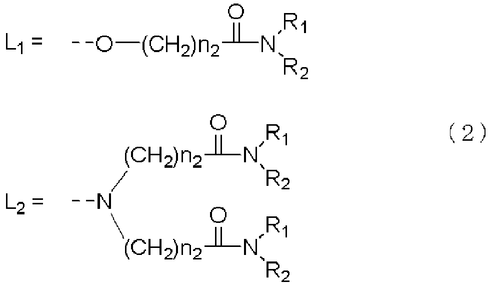 wherein "n₂" represents an integer of 1 to 3, R₁ represents one of the following formulae (3), and R₂ represents one of the following general formulae (4), 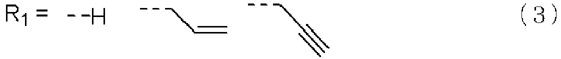 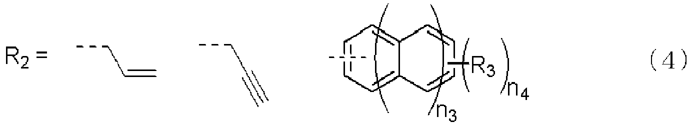 wherein R₃ represents one of the following formulae (5), "n₃" represents 0 or 1, and "n₄" represents 1 or 2.

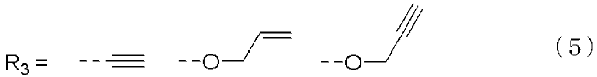

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention. The present invention also refers to the following numbered embodiments, wherein the term "claim" means "embodiment":
1. A material for forming an organic film, comprising: an organic film compound represented by the following general formula (1); and an organic solvent, 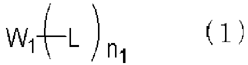 wherein W₁ represents an organic group having a valency of "n₁" and containing an aromatic ring, each L represents a substituent on the aromatic ring and is L₁, L₂, or both, represented by the following general formulae (2), and when a total number of the substituents L on the aromatic ring is "n₁", a number of L₁ is "x", and a number of L₂ is "y", "n₁", "x", and "y" satisfy relationships x + y = n₁, 2 ≤ n₁ ≤ 8, 0 ≤ y ≤ 8, and 0 ≤ x ≤ 8, 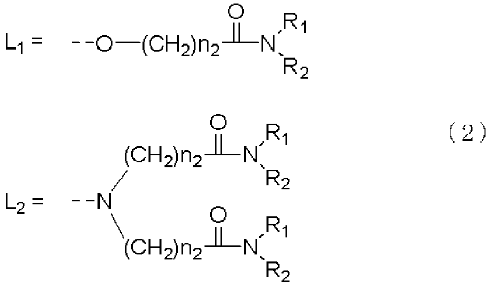 wherein "n₂" represents an integer of 1 to 3, R₁ represents one of the following formulae (3), and R₂ represents one of the following general formulae (4), 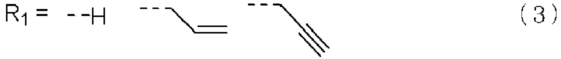 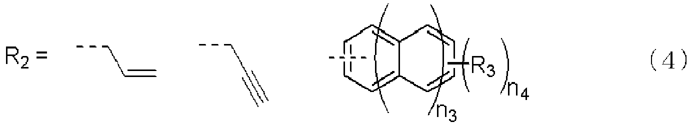 wherein R₃ represents one of the following formulae (5), "n₃" represents 0 or 1, and "n₄" represents 1 or 2. 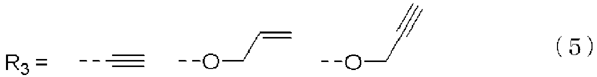
2. The material for forming an organic film according to claim 1, wherein the L₁ and L₂ constituting the substituents L on the aromatic ring of the organic film compound represented by the general formula (1) are represented by the following general formulae (6), 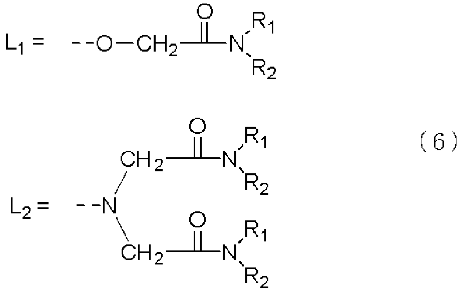 wherein R₁ and R₂ are as defined above.
3. The material for forming an organic film according to claim 1 or 2, wherein the R₁ and R₂ in the L₁ and L₂ constituting the substituents L on the aromatic ring of the organic film compound represented by the general formula (1) are any of combinations shown by the following general formulae (7), 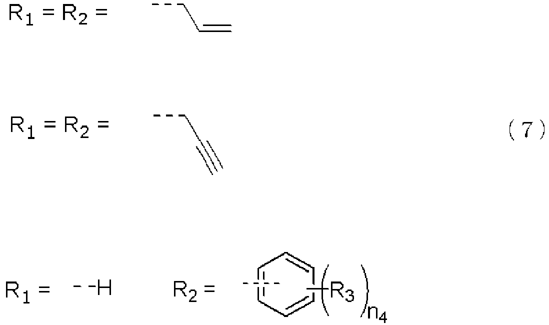 wherein R₃ and "n₄" are as defined above.
4. The material for forming an organic film according to any one of claims 1 to 3, wherein the total number "n₁" of the substituents L on the aromatic ring of the organic film compound represented by the general formula (1) satisfies 3 ≤ n₁ ≤ 6.
5. The material for forming an organic film according to any one of claims 1 to 4, wherein the organic film compound satisfies 1.00 ≤ Mw/Mn ≤ 1.15, where Mw is a weight-average molecular weight and Mn is a number-average molecular weight measured by gel permeation chromatography in terms of polystyrene.
6. The material for forming an organic film according to any one of claims 1 to 5, wherein the organic solvent is a mixture of one or more kinds of organic solvent having a boiling point of lower than 180°C and one or more kinds of organic solvent having a boiling point of 180°C or higher.
7. The material for forming an organic film according to any one of claims 1 to 6, further comprising at least one of a surfactant and a plasticizer.
8. A patterning process for forming a pattern in a body to be processed, comprising:
   forming an organic film by using the material for forming an organic film according to any one of claims 1 to 7 on a body to be processed;
   forming a silicon-containing resist middle layer film by using a silicon-containing resist middle layer film material on the organic film;
   forming a resist upper layer film by using a photoresist composition on the silicon-containing resist middle layer film;
   forming a circuit pattern in the resist upper layer film;
   transferring the pattern to the silicon-containing resist middle layer film by etching while using the resist upper layer film having the formed pattern as a mask;
   transferring the pattern to the organic film by etching while using the silicon-containing resist middle layer film having the transferred pattern as a mask; and
   further forming the pattern in the body to be processed by etching the body to be processed while using the organic film having the transferred pattern as a mask.
9. A patterning process for forming a pattern in a body to be processed, comprising:
   forming an organic film by using the material for forming an organic film according to any one of claims 1 to 7 on a body to be processed;
   forming a silicon-containing resist middle layer film by using a silicon-containing resist middle layer film material on the organic film;
   forming an organic antireflective coating on the silicon-containing resist middle layer film;
   forming a resist upper layer film by using a photoresist composition on the organic antireflective coating, so that a 4-layered film structure is constructed;
   forming a circuit pattern in the resist upper layer film;
   transferring the pattern to the organic antireflective coating and the silicon-containing resist middle layer film by etching while using the resist upper layer film having the formed pattern as a mask;
   transferring the pattern to the organic film by etching while using the silicon-containing resist middle layer film having the transferred pattern as a mask; and
   further forming the pattern in the body to be processed by etching the body to be processed while using the organic film having the transferred pattern as a mask.
10. A patterning process for forming a pattern in a body to be processed, comprising:
   forming an organic film by using the material for forming an organic film according to any one of claims 1 to 7 on a body to be processed;
   forming an inorganic hard mask middle layer film selected from a silicon oxide film, a silicon nitride film, and a silicon oxynitride film on the organic film;
   forming a resist upper layer film by using a photoresist composition on the inorganic hard mask middle layer film;
   forming a circuit pattern in the resist upper layer film;
   transferring the pattern to the inorganic hard mask middle layer film by etching while using the resist upper layer film having the formed pattern as a mask;
   transferring the pattern to the organic film by etching while using the inorganic hard mask middle layer film having the transferred pattern as a mask; and
   further forming the pattern in the body to be processed by etching the body to be processed while using the organic film having the transferred pattern as a mask.
11. A patterning process for forming a pattern in a body to be processed, comprising:
   forming an organic film by using the material for forming an organic film according to any one of claims 1 to 7 on a body to be processed;
   forming an inorganic hard mask middle layer film selected from a silicon oxide film, a silicon nitride film, and a silicon oxynitride film on the organic film;
   forming an organic antireflective coating on the inorganic hard mask middle layer film;
   forming a resist upper layer film by using a photoresist composition on the organic antireflective coating, so that a 4-layered film structure is constructed;
   forming a circuit pattern in the resist upper layer film;
   transferring the pattern to the organic antireflective coating and the inorganic hard mask middle layer film by etching while using the resist upper layer film having the formed pattern as a mask;
   transferring the pattern to the organic film by etching while using the inorganic hard mask middle layer film having the transferred pattern as a mask; and
   further forming the pattern in the body to be processed by etching the body to be processed while using the organic film having the transferred pattern as a mask.
12. The patterning process according to claim 10 or 11, wherein the inorganic hard mask middle layer film is formed by a CVD method or an ALD method.
13. The patterning process according to any one of claims 8 to 12, wherein the pattern in the resist upper layer film is formed by a lithography using light with a wavelength of 10 nm or more to 300 nm or less, a direct drawing with electron beam, nanoimprinting, or a combination thereof.
14. The patterning process according to any one of claims 8 to 13, wherein alkali development or development with an organic solvent is performed as a development method in the patterning process.
15. The patterning process according to any one of claims 8 to 14, wherein the body to be processed is a semiconductor device substrate or the semiconductor device substrate coated with any of a metal film, a metal carbide film, a metal oxide film, a metal nitride film, a metal oxycarbide film, and a metal oxynitride film.
16. The patterning process according to claim 15, wherein the metal is silicon, titanium, tungsten, hafnium, zirconium, chromium, germanium, cobalt, copper, silver, gold, aluminum, indium, gallium, arsenic, palladium, iron, tantalum, iridium, manganese, molybdenum, ruthenium, or an alloy thereof.
17. An organic film compound represented by the following general formula (1), 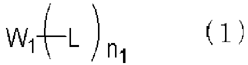 wherein W₁ represents an organic group having a valency of "n₁" and containing an aromatic ring, each L represents a substituent on the aromatic ring and is L₁, L₂, or both, represented by the following general formulae (2), and when a total number of the substituents L on the aromatic ring is "n₁", a number of L₁ is "x", and a number of L₂ is "y", "n₁", "x", and "y" satisfy relationships x + y = n₁, 2 ≤ n₁ ≤ 8, 0 ≤ y ≤ 8, and 0 ≤ x ≤ 8, 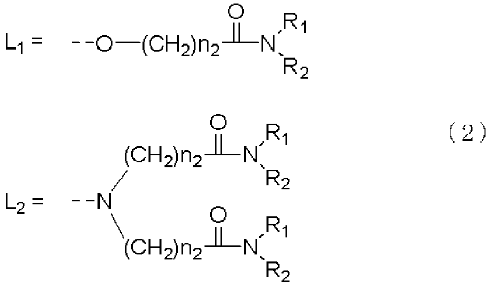 wherein "n₂" represents an integer of 1 to 3, R₁ represents one of the following formulae (3), and R₂ represents one of the following general formulae (4), 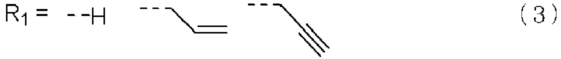 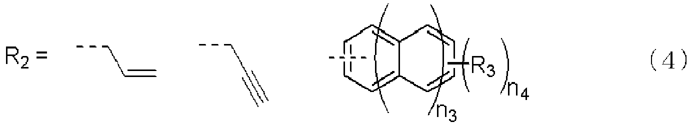 wherein R₃ represents one of the following formulae (5), "n₃" represents 0 or 1, and "n₄" represents 1 or 2.

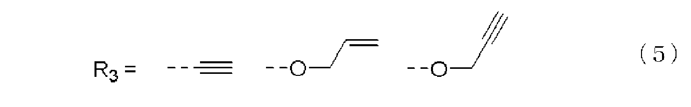

## Claims

1. A material for forming an organic film, comprising: an organic film compound represented by the following general formula (1); and an organic solvent, 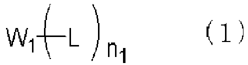 wherein W₁ represents an organic group having a valency of "n₁" and containing an aromatic ring, each L represents a substituent on the aromatic ring and is L₁, L₂, or both, represented by the following general formulae (2), and when a total number of the substituents L on the aromatic ring is "n₁", a number of L₁ is "x", and a number of L₂ is "y", "n₁", "x", and "y" satisfy relationships x + y = n₁, 2 ≤ n₁ ≤ 8, 0 ≤ y ≤ 8, and 0 ≤ x ≤ 8, 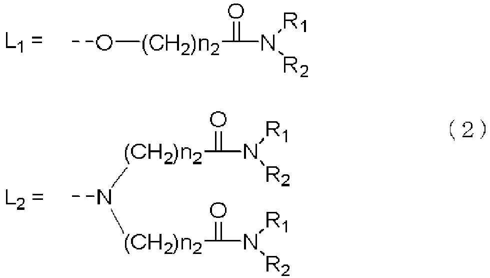 wherein "n₂" represents an integer of 1 to 3, R₁ represents one of the following formulae (3), and R₂ represents one of the following general formulae (4), 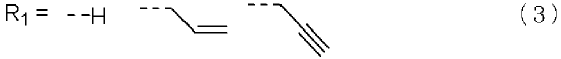 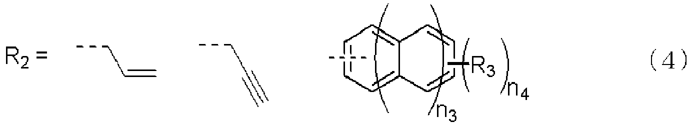 wherein R₃ represents one of the following formulae (5), "n₃" represents 0 or 1, and "n₄" represents 1 or 2.

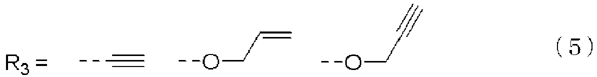

2. The material for forming an organic film according to claim 1, wherein the L₁ and L₂ constituting the substituents L on the aromatic ring of the organic film compound represented by the general formula (1) are represented by the following general formulae (6), 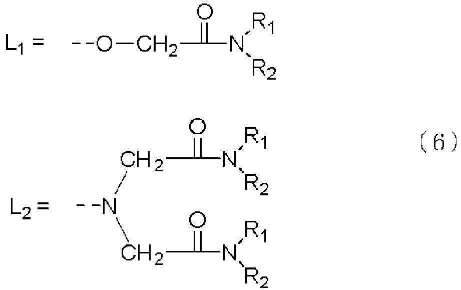 wherein R₁ and R₂ are as defined above.

3. The material for forming an organic film according to claim 1 or 2, wherein the R₁ and R₂ in the L₁ and L₂ constituting the substituents L on the aromatic ring of the organic film compound represented by the general formula (1) are any of combinations shown by the following general formulae (7), 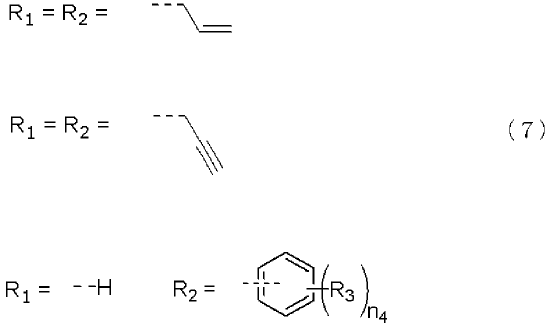 wherein R₃ and "n₄" are as defined above.

4. The material for forming an organic film according to any one of claims 1 to 3, wherein the total number "n₁" of the substituents L on the aromatic ring of the organic film compound represented by the general formula (1) satisfies 3 ≤ n₁ ≤ 6.

5. The material for forming an organic film according to any one of claims 1 to 4, wherein the organic film compound satisfies 1.00 ≤ Mw/Mn ≤ 1.15, where Mw is a weight-average molecular weight and Mn is a number-average molecular weight measured by gel permeation chromatography in terms of polystyrene.

6. The material for forming an organic film according to any one of claims 1 to 5, wherein the organic solvent is a mixture of one or more kinds of organic solvent having a boiling point of lower than 180°C and one or more kinds of organic solvent having a boiling point of 180°C or higher.

7. The material for forming an organic film according to any one of claims 1 to 6, further comprising at least one of a surfactant and a plasticizer.

8. A patterning process for forming a pattern in a body to be processed, comprising:
a)
forming an organic film by using the material for forming an organic film according to any one of claims 1 to 7 on a body to be processed;
forming a silicon-containing resist middle layer film by using a silicon-containing resist middle layer film material on the organic film;
forming a resist upper layer film by using a photoresist composition on the silicon-containing resist middle layer film;
forming a circuit pattern in the resist upper layer film;
transferring the pattern to the silicon-containing resist middle layer film by etching while using the resist upper layer film having the formed pattern as a mask;
transferring the pattern to the organic film by etching while using the silicon-containing resist middle layer film having the transferred pattern as a mask; and
further forming the pattern in the body to be processed by etching the body to be processed while using the organic film having the transferred pattern as a mask;
or
b)
forming an organic film by using the material for forming an organic film according to any one of claims 1 to 7 on a body to be processed;
forming a silicon-containing resist middle layer film by using a silicon-containing resist middle layer film material on the organic film;
forming an organic antireflective coating on the silicon-containing resist middle layer film;
forming a resist upper layer film by using a photoresist composition on the organic antireflective coating, so that a 4-layered film structure is constructed;
forming a circuit pattern in the resist upper layer film;
transferring the pattern to the organic antireflective coating and the silicon-containing resist middle layer film by etching while using the resist upper layer film having the formed pattern as a mask;
transferring the pattern to the organic film by etching while using the silicon-containing resist middle layer film having the transferred pattern as a mask; and
further forming the pattern in the body to be processed by etching the body to be processed while using the organic film having the transferred pattern as a mask;
or
c)
forming an organic film by using the material for forming an organic film according to any one of claims 1 to 7 on a body to be processed;
forming an inorganic hard mask middle layer film selected from a silicon oxide film, a silicon nitride film, and a silicon oxynitride film on the organic film;
forming a resist upper layer film by using a photoresist composition on the inorganic hard mask middle layer film;
forming a circuit pattern in the resist upper layer film;
transferring the pattern to the inorganic hard mask middle layer film by etching while using the resist upper layer film having the formed pattern as a mask;
transferring the pattern to the organic film by etching while using the inorganic hard mask middle layer film having the transferred pattern as a mask; and
further forming the pattern in the body to be processed by etching the body to be processed while using the organic film having the transferred pattern as a mask;
or
d)
forming an organic film by using the material for forming an organic film according to any one of claims 1 to 7 on a body to be processed;
forming an inorganic hard mask middle layer film selected from a silicon oxide film, a silicon nitride film, and a silicon oxynitride film on the organic film;
forming an organic antireflective coating on the inorganic hard mask middle layer film;
forming a resist upper layer film by using a photoresist composition on the organic antireflective coating, so that a 4-layered film structure is constructed;
forming a circuit pattern in the resist upper layer film;
transferring the pattern to the organic antireflective coating and the inorganic hard mask middle layer film by etching while using the resist upper layer film having the formed pattern as a mask;
transferring the pattern to the organic film by etching while using the inorganic hard mask middle layer film having the transferred pattern as a mask; and
further forming the pattern in the body to be processed by etching the body to be processed while using the organic film having the transferred pattern as a mask.

9. The patterning process according to embodiment (c) or embodiment (d) of claim 8, wherein the inorganic hard mask middle layer film is formed by a CVD method or an ALD method.

10. The patterning process according to claim 8 or 9, wherein the pattern in the resist upper layer film is formed by a lithography using light with a wavelength of 10 nm or more to 300 nm or less, a direct drawing with electron beam, nanoimprinting, or a combination thereof.

11. The patterning process according to any one of claims 8 to 10, wherein alkali development or development with an organic solvent is performed as a development method in the patterning process.

12. The patterning process according to any one of claims 8 to 11, wherein the body to be processed is a semiconductor device substrate or the semiconductor device substrate coated with any of a metal film, a metal carbide film, a metal oxide film, a metal nitride film, a metal oxycarbide film, and a metal oxynitride film.

13. The patterning process according to claim 12, wherein the metal is silicon, titanium, tungsten, hafnium, zirconium, chromium, germanium, cobalt, copper, silver, gold, aluminum, indium, gallium, arsenic, palladium, iron, tantalum, iridium, manganese, molybdenum, ruthenium, or an alloy thereof.

14. An organic film compound represented by the following general formula (1), 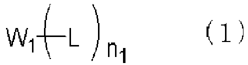 wherein W₁ represents an organic group having a valency of "n₁" and containing an aromatic ring, each L represents a substituent on the aromatic ring and is L₁, L₂, or both, represented by the following general formulae (2), and when a total number of the substituents L on the aromatic ring is "n₁", a number of L₁ is "x", and a number of L₂ is "y", "n₁", "x", and "y" satisfy relationships x + y = n₁, 2 ≤ n₁ ≤ 8, 0 ≤ y ≤ 8, and 0 ≤ x ≤ 8, 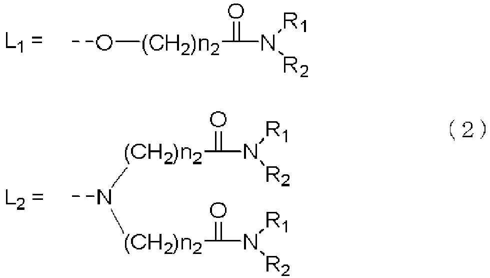 wherein "n₂" represents an integer of 1 to 3, R₁ represents one of the following formulae (3), and R₂ represents one of the following general formulae (4), 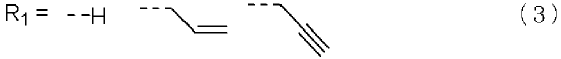 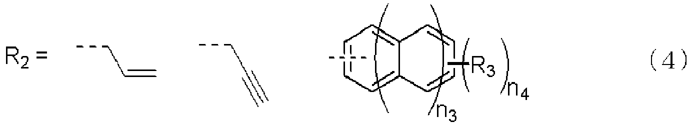 wherein R₃ represents one of the following formulae (5), "n₃" represents 0 or 1, and "n₄" represents 1 or 2.
